(19) Europäisches Patentamt
European Patent Office
Office européen des brevets

(11) EP 2 767 540 A1

(12) EUROPEAN PATENT APPLICATION
published in accordance with Art. 153(4) EPC

(43) Date of publication:
**20.08.2014 Bulletin 2014/34**

(21) Application number: **12840090.0**

(22) Date of filing: **04.10.2012**

(51) Int Cl.:
***C07D 495/22*** (2006.01) ***C07D 495/16*** (2006.01)
***H01L 29/786*** (2006.01) ***H01L 31/10*** (2006.01)
***H01L 51/05*** (2006.01) ***H01L 51/30*** (2006.01)
***H01L 51/42*** (2006.01)

(86) International application number:
**PCT/JP2012/075784**

(87) International publication number:
**WO 2013/054729 (18.04.2013 Gazette 2013/16)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.10.2011 JP 2011224664**
**17.10.2011 JP 2011227838**
**17.10.2011 JP 2011227839**
**19.03.2012 JP 2012061448**

(71) Applicant: **Sony Corporation**
**Tokyo 108-0075 (JP)**

(72) Inventors:
- **KOBAYASHI, Norihito**
**Tokyo 108-0075 (JP)**
- **IGARASHI, Eri**
**Tokyo 108-0075 (JP)**
- **KATSUHARA, Mao**
**Tokyo 108-0075 (JP)**

(74) Representative: **Horner, David Richard**
**D Young & Co LLP**
**120 Holborn**
**London EC1N 2DY (GB)**

(54) **DIOXAANTHANTHRENE-BASED COMPOUND, LAMINATED STRUCTURE AND MOLDING METHOD THEREOF, AND ELECTRONIC DEVICE AND PRODUCTION METHOD THEREOF**

(57) Provided is a dioxaanthanthrene compound represented by, for example, the following structural formula (1).

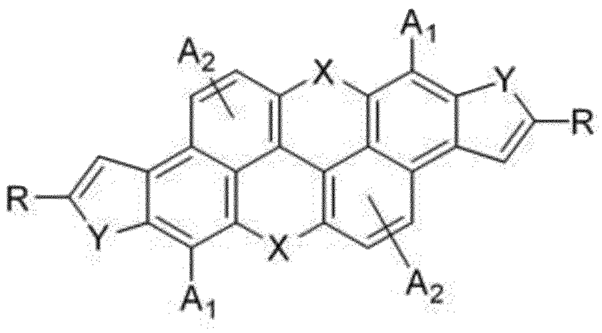

(1)

EP 2 767 540 A1

# FIG. 1

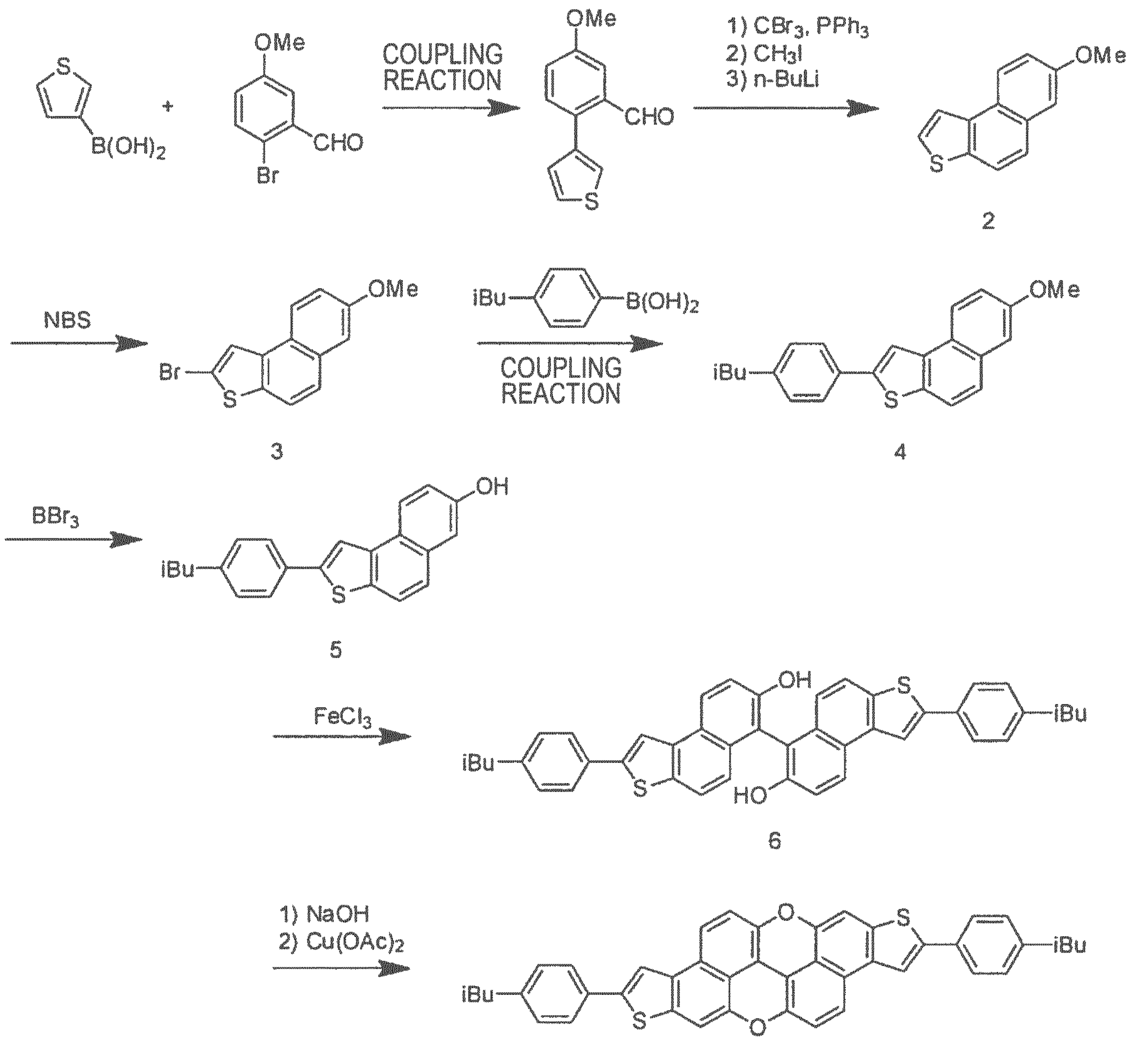
COUPLING REACTION
1) $CBr_3$, $PPh_3$
2) $CH_3I$
3) n-BuLi
NBS
COUPLING REACTION
$BBr_3$
$FeCl_3$
1) NaOH
2) $Cu(OAc)_2$

2

3

4

5

6

1

## Description

Technical Field

The present disclosure relates to a dioxaanthanthrene compound, and an electronic device that has a semiconductor layer including this dioxaanthanthrene compound. Alternatively, the present disclosure relates to a laminated structure and a formation method thereof, and an electronic device and a manufacturing method thereof.

Background Art

Currently, a field effect transistor (FET) including a thin film transistor (TFT) used in a variety of electronic equipment is configured of, for example, a channel formation region and source/drain electrodes formed in a substrate such as a silicon semiconductor substrate or a silicon semiconductor material layer, a gate insulating layer including $SiO_2$ formed on a surface of the substrate, and a gate electrode disposed to face the channel formation region with the gate insulating layer. In addition, such an FET is simply referred to as a top-gate type FET. Alternatively, the FET is configured by a gate electrode disposed on a base, a gate insulating layer disposed on the base including the gate electrode and including $SiO_2$, and a channel formation region and source/drain electrodes formed on the gate insulating layer. In addition, such an FET is simply referred to as a bottom-gate type FET. A very expensive device for manufacturing a semiconductor device is used to manufacture the FET having the structure described above, and it is thus necessary to reduce the manufacturing cost.

Among these, recently, electronic devices having an active layer formed of an organic semiconductor material have been actively developed, and in particular, organic electronic devices (which may be simply referred to hereinafter as organic devices) such as organic transistors, organic light emitting elements, or organic solar cells are attracting attention. The ultimate goal of these organic devices may be to have a low cost, a light weight, flexibility, and high performance. When compared with inorganic materials of which silicon is a prime example, the organic semiconductor material (1) allows a large-sized organic device to be manufactured at a low cost at a low temperature in a simple process, (2) allows the organic device having the flexibility to be manufactured, and (3) allows performance or a physical property of the organic device to be controlled by modifying molecules constituting the organic semiconductor material to a desired form. The organic semiconductor material thus has such various advantages.

An active layer formed of an organic semiconductor material is frequently formed on an insulating material layer. Further, in this case, usually, the active layer is obtained first by forming the insulating material layer, then coating an organic semiconductor material solution on the insulating material layer, and drying. A spin coating method is often used for the coating of the organic semiconductor material solution.

As the organic semiconductor material constituting the semiconductor layer, for example, polyacene compounds such as anthanthrene, tetracene (naphthacene), and pentacene represented by the following structural formulae are being widely researched. Further, in JP 2010-006794A, the present applicant has also proposed various dioxaanthanthrene compounds and a semiconductor device that uses such dioxaanthanthrene compounds.

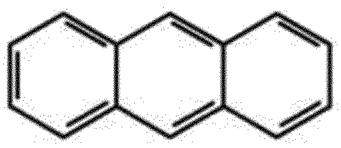

Anthanthrene

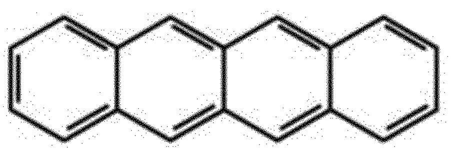

Tetracene (naphthacene)

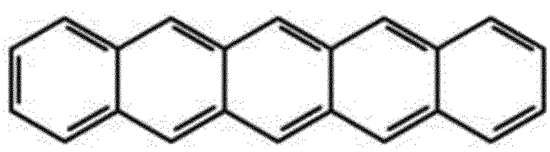

Pentacene

Since these acene compounds have a strong cohesive force due to molecular interaction that utilizes the "C-H ... π" interaction between adjacent molecules, the acene compound have a high crystallinity. Here, "C-H ... π" interaction

is one of the interactions acting between adjacent molecules, in which the C-H groups (edges) in the periphery of a molecule are weakly attracted toward the π orbital (faces) protruding above and below the molecular plane, generally resulting in an edge-to-face arrangement. Further, in a solid, the molecules thus pack in a herringbone arrangement in which the molecules are in contact with each other at their planes and sides. In addition, it has been reported that such a structure exhibits high carrier mobility and excellent semiconductor device properties (refer to Wei-Qiao Deng and William A. Goddard III, J. Phys. Chem. B, 2004 American Chemical Society, Vol. 108, No. 25, 2004, p. 8614-8621).

Citation List

Patent Literature

Patent Literature 1: JP 2010-006794A
Patent Literature 2: JP 2009-177136A

Non-Patent Literature

Non-Patent Literature 1: Wei-Qiao Deng and William A. Goddard III, J. Phys. Chem. B, 2004 American Chemical Society, Vol. 108, No. 25, 2004, p.8614-8621
Non-Patent Literature 2: T. Ohe et.al., App. Phys. Lett. 93, 053303, 2008
Non-Patent Literature 3: Journal of Organic Chemistry, 2010, 75, 8241-8251

Summary of Invention

Technical Problem

Thus, polyacene compounds are expected to improve carrier mobility due to the π-system widening as the ring length grows, so that a large orbital overlap is formed between adjacent molecules. However, pentacene is the polyacene compound that has the longest ring length that can stably exist. Polyacene compounds that have a ring length longer than pentacene (e.g., hexacene etc.) lack stability in air, and are difficult to isolate. This is thought to be because polyacene compounds are easily decomposed by oxygen, light, water, high temperatures and the like due to having a reactive site in the molecule. Thus, as long as the compound has only a polyacene skeleton, it is difficult to secure molecular stability. Therefore, the production of a channel formation region in a thin-film transistor (TFT) for driving an organic EL display, for example, that is manufactured by carrying out a complex integration process, is difficult with such a polyacene compound.

Further, in the various dioxaanthanthrene compounds disclosed in JP 2010-006794, a thin film including the dioxaanthanthrene compound represented by the following structural formula can exhibit crystallinity and undergo changes in its molecular arrangement under a high-temperature atmosphere due to the straight-chain alkyl group that is used to improve solubility and molecular orientation. Namely, a mesophase (liquid crystalline transition) can occur. Further, these changes in the molecular arrangement can, for example, affect the properties of a semiconductor device, and cause mobility, on-current and the like to deteriorate. Moreover, there is a strong demand for an organic semiconductor material having a melting point that is high enough to avoid being affected by high-temperature processes during the manufacturing steps of the electronic device and the usage environment, usage conditions and the like of the electronic device.

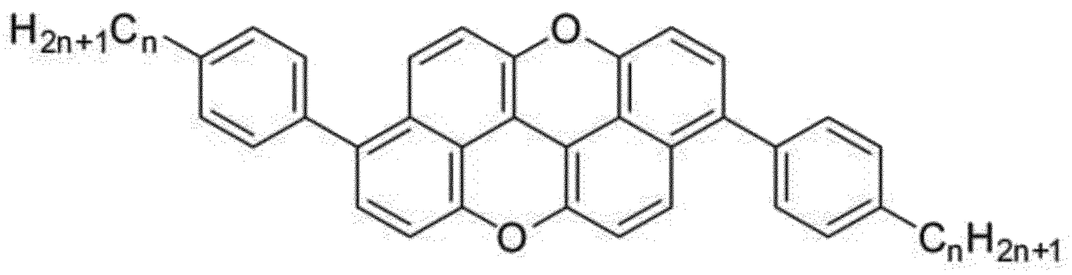

In the above-described method for forming an active layer by forming the insulating material layer, then coating an organic semiconductor material solution on the insulating material layer based on a spin coating method and drying, there is a risk that the insulating material layer can become contaminated before the active layer is formed. A technique for forming a bilayer structure of an insulating material layer and an active layer by simultaneously dissolving the material constituting the insulating material layer and the organic semiconductor material in a solvent, and coating the resultant mixture, whereby spontaneous phase separation occurs during drying, is known from JP 2009-177136 and T. Ohe et. al., App. Phys. Lett. 93, 053303, 2008, for example. However, it is difficult to uniformly control such spontaneous and natural phase separation over a large surface area, so that the thickness of the active layer tends to become uneven. Moreover, it is very difficult to obtain a state in which the interface between the insulating material layer and the active layer is flat, and yet the insulating material layer and the active layer are reliably in separate phases. Consequently, it is difficult to suppress the occurrence of unevenness in the properties of the electronic device.

According to a first aspect of the present disclosure, provided are an organic semiconductor material (specifically, a dioxaanthanthrene compound) having a high level of stability and a high level of process adaptability, and an electronic device that has a semiconductor layer formed of that organic semiconductor material.

Further, according to a second aspect of the present disclosure, provided are an organic semiconductor material (specifically, a dioxaanthanthrene compound) that is not susceptible to changes in its properties even under a high-temperature atmosphere, and an electronic device that includes a semiconductor layer formed of that organic semiconductor material.

In addition, according to a third aspect of the present disclosure, provided are a laminated structure and an electronic device, and a manufacturing method thereof, that have a layered structure of a first organic material layer (first layer) and a second organic material layer (second layer), in which the interface between the first organic material layer (first layer) and the second organic material layer (second layer) has a high level of smoothness, and these layers have a high film thickness precision, and yet are reliably in separate phases.

Solution to Problem

According to a first embodiment of the present disclosure to realize the first purpose described above, there is provided a dioxaanthanthrene compound represented by any one of structural formulae selected from the group consisting of the following structural formula (1) to structural formula (9).

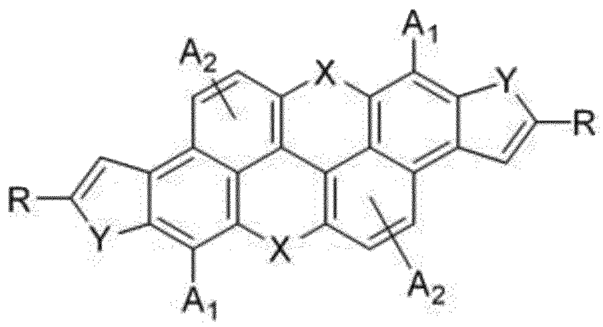

(1)

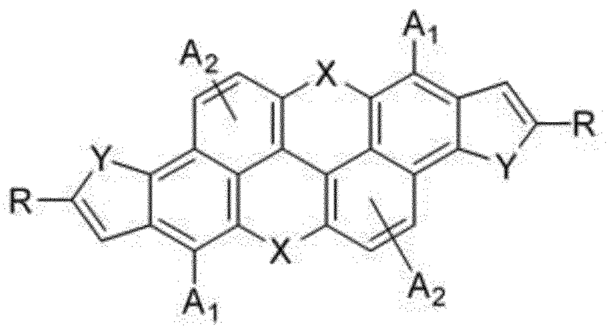

(2)

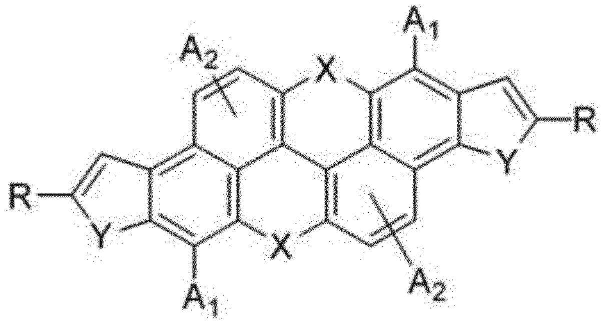

(3)

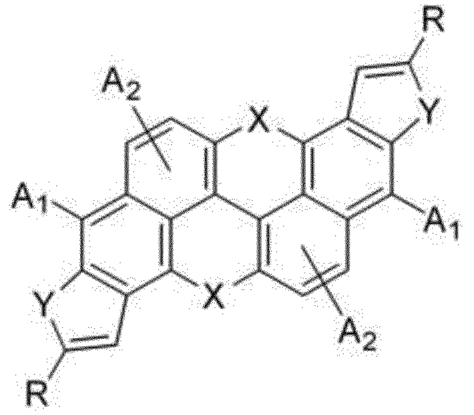

(4)

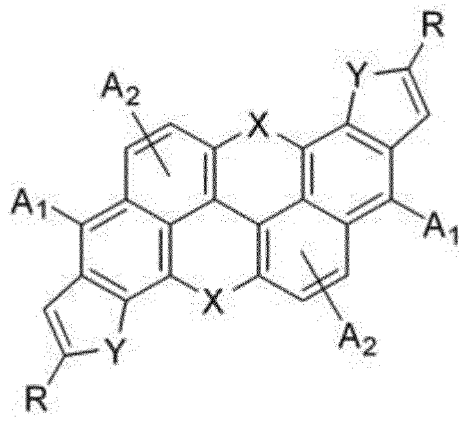

(5)

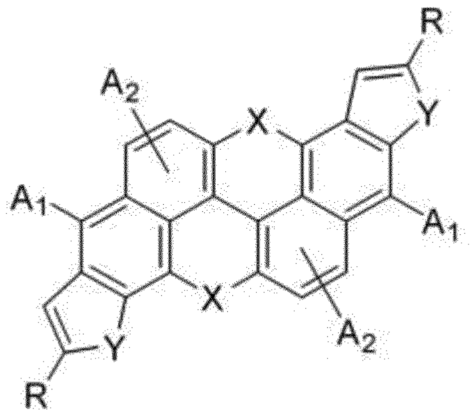

(6)

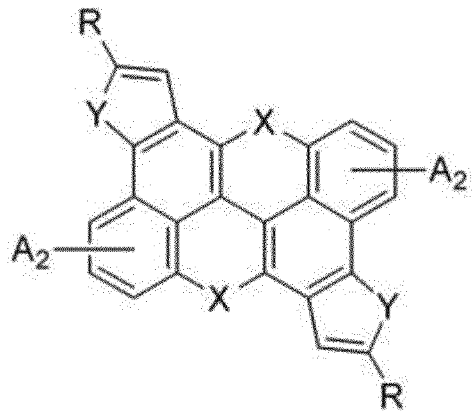

(7)

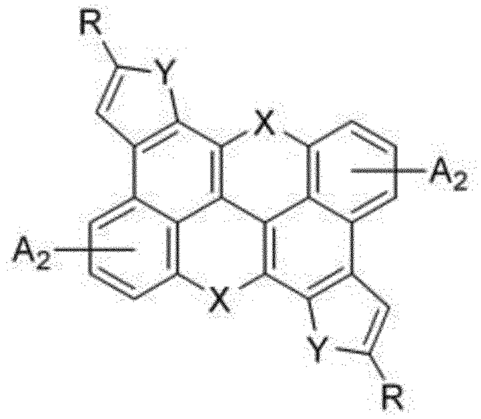

(8)

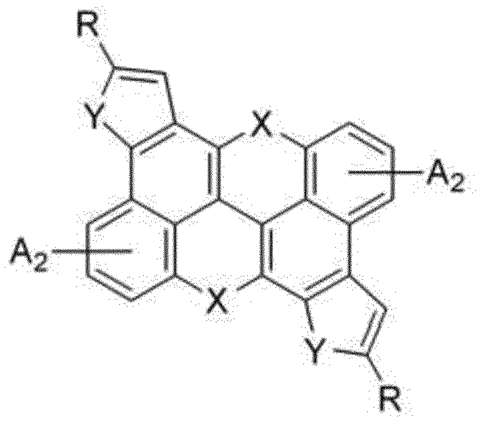

(9)

Here, X represents one atom selected from the group consisting of oxygen, sulfur, selenium and tellurium. Y represents one atom selected from the group consisting of oxygen, sulfur, selenium and tellurium. R, $A_1$, and $A_2$ each represent a hydrogen atom or a substituent selected from the group consisting of an alkyl group, a cycloalkyl group, an alkenyl group, an alkynyl group, an aryl group, an arylalkyl group, an aromatic heterocycle, a heterocyclic group, an alkoxy group, a cycloalkoxy group, an aryloxy group, an alkylthio group, a cycloalkylthio group, an arylthio group, an alkoxycarbonyl group, an aryloxycarbonyl group, a sulfamoyl group, an acyl group, an acyloxy group, an amide group, a carbamoyl group, a ureido group, a sulfinyl group, an alkylsulfonyl group, an arylsulfonyl group, an amino group, a halogen atom, a fluorinated hydrocarbon group, a cyano group, a nitro group, a hydroxy group, a mercapto group, and a silyl group.

According to the first embodiment of the present disclosure, R, $A_1$, and $A_2$ each represent a hydrogen atom or a substituent selected from the group consisting of an alkyl group, an alkenyl group, an aryl group, an arylalkyl group, an aromatic heterocycle, and a halogen atom.

Further, in the dioxaanthanthrene compound according to a first embodiment of the present disclosure that

includes such a preferred mode, X is preferably oxygen. In addition, in the dioxaanthanthrene compound according to a first embodiment of the present disclosure that includes such a preferred mode, Y is preferably sulfur. Still further, in the dioxaanthanthrene compound according to a first embodiment of the present disclosure that includes such a preferred mode, $A_1$ and $A_2$ are preferably a hydrogen atom.

Alternatively, the electronic device according to the first embodiment of the present disclosure for achieving the above-described first aspect, includes at least
a first electrode;
a second electrode disposed separated from the first electrode; and
an active layer formed of an organic semiconductor material provided from the first electrode to the second electrode, wherein the organic semiconductor material is formed of the dioxaanthanthrene compound according to the first embodiment of the present disclosure that includes the above-described various preferred modes.

According to a second embodiment of the present disclosure to realize the second purpose described above, there is provided a dioxaanthanthrene compound represented by the following structural formula (11),

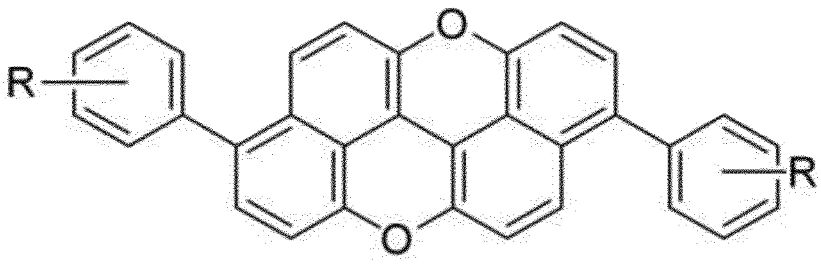

(11)

R represents an alkyl group having a branch with four or more carbon atoms.

Further, the electronic device according to the second embodiment of the present disclosure for achieving the above-described second aspect, includes at least
a first electrode;
a second electrode disposed separated from the first electrode; and
an active layer formed of an organic semiconductor material provided from the first electrode to the second electrode, wherein the organic semiconductor material includes the dioxaanthanthrene compound according to the above-described second embodiment of the present disclosure.

According to a third embodiment of the present disclosure to realize the second purpose described above, there is provided a dioxaanthanthrene compound represented by the following structural formula (21-1), or structural formula (21-2), or structural formula (21-3).

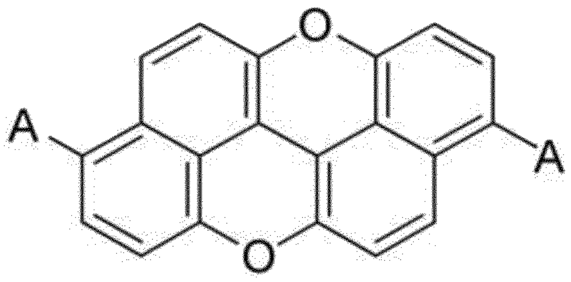

(21−1)

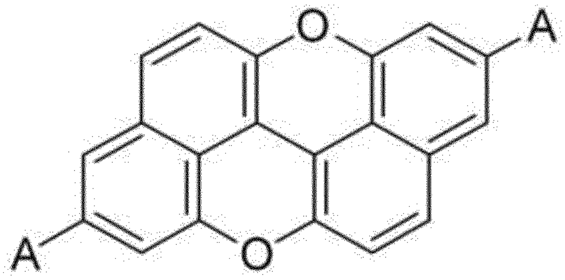

(21−2)

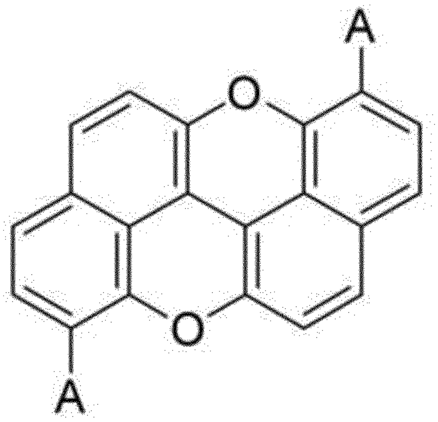

(21-3)

Here, substituent A is represented by the following structural formula (22-1) or (22-2). $X_1$, $X_2$, $X_3$, $Y_1$, $Y_2$, $Y_3$, $Y_4$, $Y_5$, $Y_6$, $Y_7$, and $Y_8$ each represent a hydrogen atom or a substituent selected from the group consisting of an alkyl group, a cycloalkyl group, an alkenyl group, an alkynyl group, an aryl group, an arylalkyl group, an aromatic heterocycle, a heterocyclic group, an alkoxy group, a cycloalkoxy group, an aryloxy group, an alkylthio group, a cycloalkylthio group, an arylthio group, an alkoxycarbonyl group, an aryloxycarbonyl group, a sulfamoyl group, an acyl group, an acyloxy group, an amide group, a carbamoyl group, a ureido group, a sulfinyl group, an alkylsulfonyl group, an arylsulfonyl group, an amino group, a halogen atom, a fluorinated hydrocarbon group, a cyano group, a nitro group, a hydroxy group, a mercapto group, and a silyl group.

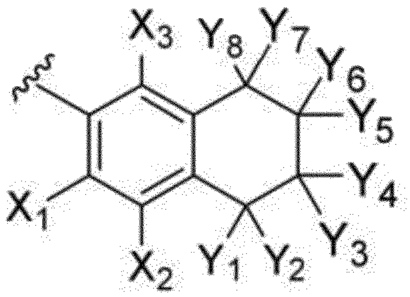

(22-1)

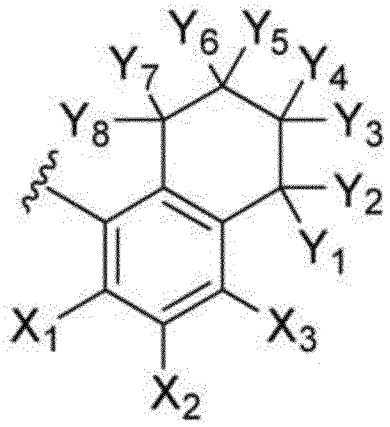

(22-2)

Alternatively, the dioxaanthanthrene compound according to the fourth embodiment of the present disclosure for achieving the above-described second aspect, is represented by the following structural formula (23-1) or structural formula (23-2).

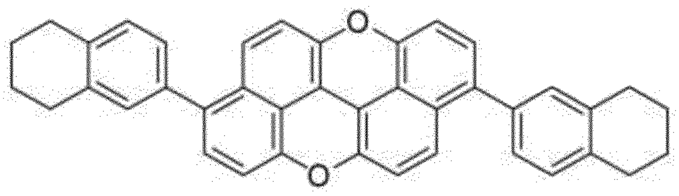

(23−1)

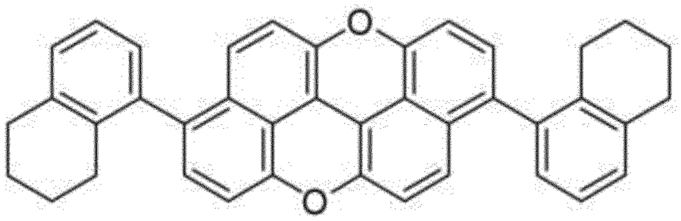

(23−2)

According to the third embodiment of the present disclosure to realize the second purpose described above, there is provided an electronic device including at least: a first electrode, a second electrode disposed separated from the first electrode, and an active layer formed of an organic semiconductor material provided from the first electrode to the second electrode. The organic semiconductor material includes the dioxaanthanthrene compound according to the third embodiment or the fourth embodiment of the present disclosure.

According to the first embodiment of the present disclosure to realize the third purpose described above, there is provided a method for forming a laminated structure, the method including the steps, in the sequence set forth, of forming a first layer formed of a first organic material on a support, and forming a second layer formed of a second organic material that is different from the first organic material by forming on the first layer a second organic material solution layer in which the second organic material is dissolved in a solvent, and then drying the second organic material solution layer. When the second organic material solution layer has been formed on the first layer, the first organic material and the second organic material mix at an interface between the first layer and the second organic material solution layer due to a surface of the first layer being dissolved by the solvent included in the second organic material solution layer. When the second organic material solution layer has dried, the first layer and the second layer separate.

According to the second embodiment of the present disclosure to realize the third purpose described above, there is provided a method for forming a laminated structure, for obtaining a laminated structure of a first layer formed of a first organic material and a second layer formed of a second organic material that is different from the first organic material, the method including forming on a support a first organic material solution layer in which the first organic material is dissolved in a first solvent and a second organic material solution layer in which the second organic material is dissolved in a second solvent, and then drying the first organic material solution layer and the second organic material solution layer. When the first organic material solution layer and the second organic material solution layer have been formed on the support, the first organic material and the second organic material mix at an interface between the first organic material solution layer and the second organic material solution layer. When first organic material solution layer and the second organic material solution layer have dried, the first layer and the second layer separate.

According to the first embodiment of the present disclosure to realize the third purpose described above, there is provided a method for manufacturing an electronic device, the method including at least the steps, in the sequence set forth, of:

(A) forming on a base a control electrode and a first insulating layer covering the control electrode;
(B) forming on the first insulating layer a first layer formed of a first organic material; and
(C) forming a second layer formed of a second organic material that is different from the first organic material by forming on the first layer a second organic material solution layer in which the second organic material is dissolved in a solvent, and then drying the second organic material solution layer.

The first organic material is formed of an insulating material and the second organic material is formed of an organic semiconductor material.

A second insulating layer is configured by the first layer.

An active layer is configured by the second layer.

When the second organic material solution layer has been formed on the first layer, the first organic material and the second organic material mix at an interface between the first layer and the second organic material solution layer due to a surface of the first layer being dissolved by the solvent included in the second organic material solution layer.

When the second organic material solution layer has dried, the first layer and the second layer separate.

According to the second embodiment of the present disclosure to realize the third purpose described above, there is provided a method for manufacturing an electronic device, the method including at least the steps, in the sequence set forth, of:

(A) forming a control electrode in a groove portion formed in a base;
(B) forming on the base and the control electrode a first layer formed of a first organic material; and
(C) forming a second layer formed of a second organic material that is different from the first organic material by forming on the first layer a second organic material solution layer in which the second organic material is dissolved in a solvent, and then drying the second organic material solution layer,

The first organic material is formed of an insulating material and the second organic material is formed of an organic semiconductor material.

An insulating layer is configured by the first layer.

An active layer is configured by the second layer.

When the second organic material solution layer has been formed on the first layer, the first organic material and the second organic material mix at an interface between the first layer and the second organic material solution layer due to a surface of the first layer being dissolved by the solvent included in the second organic material solution layer.

When the second organic material solution layer has dried, the first layer and the second layer separate.

According to the third embodiment of the present disclosure to realize the third purpose described above, there is provided a method for manufacturing an electronic device, the method including at least the steps, in the sequence set forth, of:

(A) forming on a base a control electrode and a first insulating layer covering the control electrode; and
(B) obtaining a laminated structure of a first layer formed of a first organic material and a second layer formed of a second organic material that is different from the first organic material by forming on the first insulating layer a first organic material solution layer in which the first organic material is dissolved in a first solvent and a second organic material solution layer in which the second organic material is dissolved in a second solvent, and then drying the first organic material solution layer and the second organic material solution layer.

The first organic material is formed of an insulating material and the second organic material is formed of an organic semiconductor material.

A second insulating layer is configured by the first layer.

An active layer is configured by the second layer.

When the first organic material solution layer and the second organic material solution layer have been formed, the first organic material and the second organic material mix at an interface between the first organic material solution layer and the second organic material solution layer.

When the first organic material solution layer and the second organic material solution layer have dried, the first layer and the second layer separate.

According to a fourth embodiment of the present disclosure to realize the third purpose described above, there is provided a method for manufacturing an electronic device, the method including at least the steps, in the sequence set forth, of:

(A) forming a control electrode in a groove portion formed in a base; and
(B) obtaining a laminated structure of a first layer formed of a first organic material and a second layer formed of a second organic material that is different from the first organic material by forming on the base and the control electrode a first organic material solution layer in which the first organic material is dissolved in a first solvent and a second organic material solution layer in which the second organic material is dissolved in a second solvent, and then drying the first organic material solution layer and the second organic material solution layer.

The first organic material is formed of an insulating material and the second organic material is formed of an organic semiconductor material.

An insulating layer is configured by the first layer.

An active layer is configured by the second layer.

When the first organic material solution layer and the second organic material solution layer have been formed, the first organic material and the second organic material mix at an interface between the first organic material solution layer and the second organic material solution layer.

When the first organic material solution layer and the second organic material solution layer have dried, the

first layer and the second layer separate.

According to a fifth embodiment of the present disclosure to realize the third purpose described above, there is provided a method for manufacturing an electronic device, the method including at least the steps, in the sequence set forth, of:

(A) forming a first electrode and a second electrode in a groove portion formed in a base;
(B) forming on the base, the first electrode, and the second electrode a first layer formed of a first organic material; and
(C) forming a second layer formed of a second organic material that is different from the first organic material by forming on the first layer a second organic material solution layer in which the second organic material is dissolved in a solvent, and then drying the second organic material solution layer.

The first organic material is formed of an organic semiconductor material and the second organic material is formed of an insulating material.

An active layer is configured by the first layer.

An insulating layer is configured by the second layer.

When the second organic material solution layer has been formed on the first layer, the first organic material and the second organic material mix at an interface between the first layer and the second organic material solution layer due to a surface of the first layer being dissolved by the solvent included in the second organic material solution layer.

When the second organic material solution layer has dried, the first layer and the second layer separate.

According to a sixth embodiment of the present disclosure to realize the third purpose described above, there is provided a method for manufacturing an electronic device, the method including at least the steps, in the sequence set forth, of:

(A) forming a first electrode and a second electrode in a groove portion formed in a base; and
(B) obtaining a laminated structure of a first layer formed of a first organic material and a second layer formed of a second organic material that is different from the first organic material by forming on the base, the first electrode, and the second electrode a first organic material solution layer in which the first organic material is dissolved in a first solvent and a second organic material solution layer in which the second organic material is dissolved in a second solvent, and then drying the first organic material solution layer and the second organic material solution layer.

The first organic material is formed of an organic semiconductor material and the second organic material is formed of an insulating material.

An active layer is configured by the first layer.

An insulating layer is configured by the second layer.

When the first organic material solution layer and the second organic material solution layer have been formed, the first organic material and the second organic material mix at an interface between the first organic material solution layer and the second organic material solution layer.

When the first organic material solution layer and the second organic material solution layer have dried, the first layer and the second layer separate.

According to an embodiment of the present disclosure to realize the third purpose described above, there is provided a laminated structure including a first layer formed of a first organic material and a second layer formed of a second organic material that is different from the first organic material. A combination of the first organic material and the second organic material is configured by a combination of materials so that a value obtained by subtracting a Gibbs free energy $G_1$ of the first organic material and a Gibbs free energy $G_2$ of the second organic material from a Gibbs free energy $G_0$ of a mixed system of the first organic material and the second organic material is positive. Here, the first organic material is formed of an insulating material or an organic semiconductor material, and the second organic material is formed of an organic semiconductor material or an insulating material. Further, the second layer is formed on the first layer, or alternatively, the first layer is formed on the second layer.

According to a fourth embodiment of the present disclosure to realize the third purpose described above, there is provided an electronic device including an electrode structure, an insulating layer, and an active layer. The insulating layer is formed of a first organic material configured from an insulating material. The active layer is formed of a second organic material configured from an organic semiconductor material. A combination of the first organic material and the second organic material is configured by a combination of materials so that a value obtained by subtracting a Gibbs free energy $G_1$ of the first organic material and a Gibbs free energy $G_2$ of the second organic material from a Gibbs free energy $G_0$ of a mixed system of the first organic material and the second organic material is positive. Here, the active layer is formed on the insulating layer, or alternatively, the insulating layer is formed on the active layer.

Advantageous Effects of Invention

For the dioxaanthanthrene compound (a so-called peri-xanthenoxanthene compound, 6,12-dioxaanthanthrene compound, hereinafter sometimes abbreviated to "PXX compound") according to the first embodiment of the present disclosure, or alternatively, the electronic device using this PXX compound in a channel formation region, the π-conjugated system can be widened by fusing a thiophene ring to a PXX skeleton, which has a proven record relating to stability and high mobility. Namely, a large intermolecular interaction can be obtained due to the formation of a much wider (larger) π-orbital overlap between adjacent PXX compound molecules. Consequently, a much greater improvement in carrier mobility can be achieved. Further, since a large amount of intermolecular contact can be expected based on the "Y" atoms (e.g., sulfur atoms) that project out at the periphery of the PXX compound molecules, orbital overlap between the PXX compound molecules is greatly increased, so that much higher conductivity can be exhibited. In addition, introducing a substituent, such as R, $A_1$, and $A_2$, to a predetermined position of the PXX compound molecules allows the solubility, the molecular arrangement and the like to be controlled, and also allows a high-mobility organic semiconductor that can be coated or printed to be easily obtained. Still further, by fusing a ring (e.g., a heterocyclic compound such as a thiophene ring) containing a "Y" atom (e.g., sulfur atom) to a site of the PXX compound having a high electron density (specifically, the second and third sites, and the eighth and ninth sites), reactivity with oxygen in the air can be suppressed, and the molecular HOMO level can be deepened. As a result, an organic semiconductor material that is stable in air can be provided.

Further, for the dioxaanthanthrene compound according to the second embodiment of the present disclosure, or alternatively, the electronic device according to the second embodiment of the present disclosure, because R is an alkyl group having a branch with four or more carbon atoms, liquid crystallinity is not exhibited even under a high-temperature atmosphere, namely, there is no change in the molecular arrangement, so that the properties of the electronic device are less susceptible to changing even under a high-temperature atmosphere. Further, the melting point is high enough to avoid being affected by high-temperature processes during the manufacturing steps of the electronic device and the usage environment, usage conditions and the like of the electronic device.

For the dioxaanthanthrene compound according to the third or fourth embodiment of the present disclosure, or alternatively, the dioxaanthanthrene compound (PXX compound) used in the electronic device according to the third embodiment of the present disclosure, the third and ninth sites, or, the second and eighth sites, or, the first and seventh sites of the 6,12-dioxaanthanthrene are substituted with a phenyl group, and, a cyclic alkyl group (cyclic alkane) is introduced on the phenyl group. Consequently, liquid crystallinity is not exhibited under a high-temperature atmosphere, namely, changes do not occur in the molecular arrangement, so the properties of the electronic device are not susceptible to changing even under a high-temperature atmosphere. Further, the melting point is high enough to avoid being affected by high-temperature processes during the manufacturing steps of the electronic device and the usage environment, usage conditions and the like of the electronic device.

In the method for forming a laminated structure according to the first embodiment of the present disclosure, and in the method for manufacturing an electronic device according to the first, second, and fifth embodiments of the present disclosure, when the second organic material solution layer has been formed on the first layer, the first organic material and the second organic material mix at the interface between the first layer and the second organic material solution layer due to the surface of the first layer being dissolved by the solvent included in the second organic material solution layer. However, at regions away from the interface, there is no mixing of the first organic material and the second organic material, so that when the second organic material solution layer has dried, the first layer and the second layer separate. Further, in the method for forming a laminated structure according to the second embodiment of the present disclosure, and in the method for manufacturing an electronic device according to the third, fourth, and sixth embodiments of the present disclosure, when the first organic material solution layer and the second organic material solution layer are formed, the first organic material and the second organic material mix at the interface between the first organic material solution layer and the second organic material solution layer, but at regions away from the interface, there is no mixing of the first organic material and the second organic material, so that when the first organic material solution layer and the second organic material solution layer have dried, the first layer and the second layer separate. Therefore, the interface between the first organic material solution layer (first layer) and the second organic material solution layer (second layer) has high level of smoothness, and these layers have a high film thickness precision, and yet are reliably in separate phases, so that there is no contamination of the first organic material solution layer (first layer) before the second organic material solution layer (second layer) is formed. Consequently, an electronic device can be obtained that has little unevenness in its properties and has excellent performance. For the laminated structure according to the embodiments of the present disclosure or the electronic device according to the fourth embodiment of the present disclosure, since the relationship among the value for the Gibbs free energy of the mixed system of the first organic material layer and the second organic material layer, the value for the Gibbs free energy of the first organic material, and the value for the Gibbs free energy of the second organic material is defined, a separated state can be obtained reliably, spontaneously, and naturally when forming these layers. It is noted that such a phenomenon is based on the

Flory-Huggins theory. Regarding the Flory-Huggins theory, refer to, for example, J. L. Barrat and J. P. Hansen, "Basic Concept for Simple and Complex Liquids", Cambridge University Press, Cambridge UK, 2003, and P.M. Chaikin, T.C. Lubensky, "Principles of Condensed Matter Physics" (first volume), Yoshioka Shoten (2000). Further, a state can be obtained in which the interface between these layers has a high level of smoothness, and these layers have a high film thickness precision.

Brief Description of Drawings

[FIG. 1] FIG. 1 is a diagram illustrating a synthesis pathway of the dioxaanthanthrene compound of Working Example 1.
[FIG. 2] FIGS. 2A and 2B are schematic partial end diagrams of a base and the like for illustrating an outline of the method for manufacturing the electronic device of Working Example 4.
[FIG. 3] FIGS. 3A and 3B are schematic partial end diagrams of a base and the like for illustrating an outline of the method for manufacturing the electronic device of Working Example 5.
[FIG. 4] FIGS.4A and 4B are schematic partial end diagrams of a base and the like for illustrating an outline of the method for manufacturing the electronic device of Working Example 6.
[FIG. 5] FIGS. 5A, 5B and 5C are schematic partial end diagrams of a base and the like for illustrating an outline of the method for manufacturing the electronic device of Working Example 7.
[FIG. 6] FIGS. 6A and 6B are schematic partial end diagrams of the electronic device of Working Example 8.
[FIG. 7] FIGS. 7A, 7B, 7C, 7D, and 7E are schematic partial end diagrams of a base and the like for illustrating the laminated structure, the three-terminal type electronic device, the method for forming the laminated structure, and the method for manufacturing the electronic device (the method for manufacturing an electronic device according to the first embodiment of the present disclosure) of Working Example 9.
[FIG. 8] FIGS. 8A, 8B, 8C, 8D, and 8E are schematic partial end diagrams of a base and the like for illustrating the laminated structure, the three-terminal type electronic device, the method for forming the laminated structure, and the method for manufacturing the electronic device (the method for manufacturing an electronic device according to the second embodiment of the present disclosure) of Working Example 10.
[FIG. 9] FIGS. 9A, 9B, 9C, and 9D are schematic partial end diagrams of a base and the like for illustrating the laminated structure, the three-terminal type electronic device, the method for forming the laminated structure, and the method for manufacturing the electronic device (the method for manufacturing an electronic device according to the third embodiment of the present disclosure) of Working Example 11.
[FIG. 10] FIGS. 10A, 10B, 10C, and 10D are schematic partial end diagrams of a base and the like for illustrating the laminated structure, the three-terminal type electronic device, the method for forming the laminated structure, and the method for manufacturing the electronic device (the method for manufacturing an electronic device according to the fourth embodiment of the present disclosure) of Working Example 12.
[FIG. 11] FIGS. 11A, 11B, 11C, 11D, and 11E are schematic partial end diagrams of a base and the like for illustrating the laminated structure, the three-terminal type electronic device, the method for forming the laminated structure, and the method for manufacturing the electronic device (the method for manufacturing an electronic device according to the first embodiment of the present disclosure) of Working Example 13.
[FIG. 12] FIGS. 12A, 12B, 12C, and 12D are schematic partial end diagrams of a base and the like for illustrating the laminated structure, the three-terminal type electronic device, the method for forming the laminated structure, and the method for manufacturing the electronic device (the method for manufacturing an electronic device according to the sixth embodiment of the present disclosure) of Working Example 14.
[FIG. 13] FIGS. 13A and 13B are schematic partial end diagrams of the two-terminal type electronic device of Working Example 15.

Description of Embodiments

Hereinafter, preferred embodiments of the present disclosure will be described in detail with reference to the appended drawings. Note that, in this specification and the drawings, elements that have substantially the same function and structure are denoted with the same reference signs, and repeated explanation is omitted. It is noted that the embodiments of the present disclosure are not limited to the Working Examples, and the respective numerical values and materials in the Working Examples are examples. The description will now be made in the following order.

1. Dioxaanthanthrene compound according to the first to fourth embodiments of the present disclosure, laminated structure and formation method thereof according to an embodiment of the present disclosure, electronic device

according to the first to fourth embodiments of the present disclosure, method for manufacturing an electronic device according to the first to sixth embodiments of the present disclosure, and overall description

2. Working Example 1 (dioxaanthanthrene compound according to the first embodiment of the present disclosure)
3. Working Example 2 (dioxaanthanthrene compound according to the second embodiment of the present disclosure)
4. Working Example 3 (dioxaanthanthrene compound according to the third or fourth embodiment of the present disclosure)
5. Working Example 4 (electronic device according to the first to third embodiments of the present disclosure, three-terminal type electronic device)
6. Working Example 5 (modification of Working Example 4)
7. Working Example 6 (another modification of Working Example 4)
8. Working Example 7 (yet another modification of Working Example 4)
9. Working Example 8 (yet another modification of Working Example 4, two-terminal type electronic device)
10. Working Example 9 (laminated structure according to the embodiments of the present disclosure, electronic device according to the fourth embodiment of the present disclosure, method for forming a laminated structure according to the first embodiment of the present disclosure, and method for manufacturing an electronic device according to the first embodiment of the present disclosure)
11. Working Example 10 (laminated structure according to the embodiments of the present disclosure, electronic device according to the fourth embodiment of the present disclosure, method for forming a laminated structure according to the second embodiment of the present disclosure, and method for manufacturing an electronic device according to the second embodiment of the present disclosure)
12. Working Example 11 (laminated structure according to the embodiments of the present disclosure, electronic device according to the fourth embodiment of the present disclosure, method for forming a laminated structure according to the first embodiment of the present disclosure, and method for manufacturing an electronic device according to the third embodiment of the present disclosure)
13. Working Example 12 (laminated structure according to the embodiments of the present disclosure, electronic device according to the fourth embodiment of the present disclosure, method for forming a laminated structure according to the second embodiment of the present disclosure, and method for manufacturing an electronic device according to the fourth embodiment of the present disclosure)
14. Working Example 13 (laminated structure according to the embodiments of the present disclosure, electronic device according to the fourth embodiment of the present disclosure, method for forming a laminated structure according to the first embodiment of the present disclosure, and method for manufacturing an electronic device according to the fifth embodiment of the present disclosure)
15. Working Example 15 (laminated structure according to the embodiments of the present disclosure, electronic device according to the fourth embodiment of the present disclosure, method for forming a laminated structure according to the second embodiment of the present disclosure, and method for manufacturing an electronic device according to the sixth embodiment of the present disclosure)
16. Working Example 15 (laminated structure according to the embodiments of the present disclosure, electronic device according to the fourth embodiment of the present disclosure), other matters

Dioxaanthanthrene compound according to the first to fourth embodiments of the present disclosure, laminated structure and formation method thereof according to an embodiment of the present disclosure, electronic device according to the first to fourth embodiments of the present disclosure, method for manufacturing an electronic device according to the first to sixth embodiments of the present disclosure, and overall description

In the dioxaanthanthrene compound according to the first embodiment of the present disclosure, or, the dioxaanthanthrene compound which is an organic semiconductor material constituting an active layer in the electronic device according to the first embodiment of the present disclosure, examples of the alkyl group constituting R, $A_1$, and $A_2$, respectively, include a methyl group, an ethyl group, a propyl group, an isopropyl group, an isobutyl group, an isopentyl group, an isohexyl group, a tertiary butyl group, a pentyl group, a hexyl group, an octyl group, a dodecyl group and the like. It is noted that the alkyl group may be linear or branched. Further, examples of the cycloalkyl group may include a cyclopentyl group, a cyclohexyl group and the like; examples of the alkenyl may include a vinyl group and the like; examples of the alkynyl group may include an ethynyl group; examples of the aryl group may include a phenyl group, a naphthyl group, a biphenyl group and the like; examples of the arylalkyl group may include a methyl aryl group, an ethyl aryl group, an isopropyl aryl group, normal butyl aryl group, a p-tolyl group, a p-ethylphenyl group, a p-isopropylphenyl group, a p-iso-butylphenyl group, a 4-propylphenyl group, a 4-butylphenyl group, a 4-nonylphenyl group, an o-methylphenyl group, an o-isobutyl group, an o,p-dimethylphenyl group, a p-ethyl-o-methyl-phenyl group, a p-isobutyl-o-methylphenyl group and the like; examples of the aromatic heterocycle may include a pyridyl group, a thienyl group, a furyl group, a pyridazinyl group, a pyrimidinyl group, a pyrazinyl group, a triazinyl group, an imidazolyl group, a pyrazolyl group, a thiazolyl group, a quinazolinyl group, a phthalazinyl group and the like; examples of the heterocyclic group may

include a pyrrolidyl group, an imidazolidyl group, a morpholinyl group, an oxazolidyl group and the like; examples of the alkoxy group may include a methoxy group, an ethoxy group, a propyloxy group, a pentyloxy group, a hexyloxy group and the like; examples of the cycloalkoxy group may include a cyclopentyl group, a cyclohexyl group and the like; examples of the aryloxy group may include a phenoxy group, a naphthyloxy group and the like; examples of the alkylthio group may include a methylthio group, an ethylthio group, a propylthio group, a pentylthio group, a hexylthio group and the like; examples of the cycloalkylthio group may include a cyclopentylthio group, a cyclohexylthio group and the like; examples of the arylthio group may include a phenylthio group, a naphthylthio group and the like; examples of the alkoxycarbonyl group may include a methyloxycarbonyl group, an ethyloxycarbonyl group, a butyloxycarbonyl group, an octyloxycarbonyl group and the like; examples of the aryloxycarbonyl group may include a phenyloxycarbonyl group, a naphthyloxycarbonyl group and the like; examples of the sulfamoyl group may include an aminosulfonyl group, a methylaminosulfonyl group, a dimethylaminosulfonyl group, a cyclohexylaminosulfonyl group, a phenylaminosulfonyl group, a naphthylaminosulfonyl group, a 2-pyridylaminosulfonyl group and the like; examples of the acyl group may include an acetyl group, an ethylcarbonyl group, a propylcarbonyl group, a cyclohexylcarbonyl group, an octylcarbonyl group, a 2-ethylhexylcarbonyl group, a dodecylcarbonyl group, a phenylcarbonyl group, a naphthylcarbonyl group, a pyridylcarbonyl group and the like; examples of the acyloxy group may include an acetyloxy group, an ethylcarbonyloxy group, an octylcarbonyloxy group, a phenylcarbonyloxy group and the like; examples of the amide may include a methylcarbonylamino group, an ethylcarbonylamino group, a dimethylaminocarbonylamino group, a pentylcarbonylamino group, a cyclohexylcarbonylamino group, 2-ethylhexylcarbonylamino group, a phenylcarbonylamino group, a naphthylcarbonylamino group and the like; examples of the carbamoyl group may include an aminocarbamoyl group, a methylaminocarbamoyl group, a dimethylaminocarbamoyl group, a cyclohexylaminocarbamoyl group, a 2-ethylhexylaminocarbamoyl group, a phenylaminocarbamoyl group, a naphthylaminocarbamoyl group, a 2-pyridylaminocarbonyl group and the like; examples of the ureido group may include a methylureido group, an ethylureido group, a cyclohexylureido group, a dodecylureido group, a phenylureido group, a naphthylureido group, a 2-pyridylaminoureido group and the like; examples of the sulfinyl may include a methylsulfinyl group, an ethylsulfinyl group, a butylsulfinyl group, a cyclohexyl sulfinyl group, a 2-ethylhexylsulfinyl group, a phenylsulfinyl group, a naphthylsulfinyl group, a 2-pyridylsulfinyl group and the like; examples of the alkylsulfonyl group may include a methylsulfonyl group, an ethylsulfonyl group, a butylsulfonyl group, a cyclohexylsulfonyl group, a 2-ethylhexylsulfonyl group, a dodecylsulfonyl group and the like; examples of the arylsulfonyl group may include a phenylsulfonyl group, a naphthylsulfonyl group, a 2-pyridylsulfonyl group and the like; examples of the amino group may include an amino group, an ethylamino group, a dimethylamino group, a butylamino group, a 2-ethylhexylamino group, an anilino group, a naphthylamino group, a 2-pyridylamino group and the like; examples of the halogen atom may include a fluorine atom, a chlorine atom, a bromine atom, an iodine atom and the like; and examples of the fluorinated hydrocarbon group may include a fluoromethyl group, a trifluoromethyl group, a pentafluoroethyl group, a pentafluorophenyl group and the like. Further examples may also include a cyano group, a nitro group, a hydroxy group, a mercapto group, a silyl group and the like. Examples of the silyl group a trimethylsilyl group, a triisopropylsilyl group, a triphenylsilyl group, a phenyl group diethylsilyl and the like. Here, the above-described substituents may be further substituted with an above-described substituent. Further, a plurality of these substituents may be joined to each other to form a ring.

In the electronic device according to the second embodiment of the present disclosure, it is preferred that the active layer is formed by coating the dioxaanthanthrene compound on a base, and then drying. Further, for the dioxaanthanthrene compound according to the second embodiment of the present disclosure, or alternatively, the dioxaanthanthrene compound that is used in the electronic device according to the second embodiment of the present disclosure, it is preferred that solubility in an organic solvent (specifically, toluene) is 0.5 grams or more based on 100 grams of organic solvent, as this allows a layer including the dioxaanthanthrene compound (e.g., the active layer) to be formed based on a wet method, such as an application method a printing method, and a coating method.

In the dioxaanthanthrene compound according to the third embodiment of the present disclosure, $X_1$, $X_2$, $X_3$, $Y_1$, $Y_2$, $Y_3$, $Y_4$, $Y_5$, $Y_6$, $Y_7$, and $Y_8$ each represent a hydrogen atom or a substituent selected from the group consisting of an alkyl group, an alkenyl group, an aryl group, an arylalkyl group, an aromatic heterocycle, and a halogen atom.

In the dioxaanthanthrene compound according to the third embodiment of the present disclosure, or, the dioxaanthanthrene compound according to the third embodiment of the present disclosure which is an organic semiconductor material constituting an active layer in the electronic according to the third embodiment of the present disclosure, examples of the alkyl group constituting X, $X_1$, $X_2$, $X_3$, $Y_1$, $Y_2$, $Y_3$, $Y_4$, $Y_5$, $Y_6$, $Y_7$ and $A_8$, respectively, include a methyl group, an ethyl group, a propyl group, an isopropyl group, an isobutyl group, an isopentyl group, an isohexyl group, a tertiary butyl group, a pentyl group, a hexyl group, an octyl group, a dodecyl group and the like. It is noted that the alkyl group may be linear or branched. Further, examples of the cycloalkyl group may include a cyclopentyl group, a cyclohexyl group and the like; examples of the alkenyl may include a vinyl group and the like; examples of the alkynyl group may include an ethynyl group; examples of the aryl group may include a phenyl group, a naphthyl group, a biphenyl group and the like; examples of the arylalkyl group may include a methyl aryl group, an ethyl aryl group, an isopropyl aryl group, normal butyl aryl group, a p-tolyl group, a p-ethylphenyl group, a p-isopropylphenyl group, a p-iso-butylphenyl group, a 4-propylphenyl group, a 4-butylphenyl group, a 4-nonylphenyl group, and the like; examples of the aromatic heterocycle may include a pyridyl group, a thienyl group, a furyl group, a pyridazinyl group, a pyrimidinyl group, a pyrazinyl group, a triazinyl group, an imidazolyl group, a pyrazolyl group, a thiazolyl group, a quinazolinyl group, a phthalazinyl group and the like; examples of the heterocyclic group may include a pyrrolidyl group, an imidazolidyl group, a morpholinyl group, an oxazolidyl group and the like; examples of the alkoxy group may include a methoxy group, an ethoxy group, a propyloxy group, a pentyloxy group, a hexyloxy group and the like; examples of the cycloalkoxy group may include a cyclopentyl group, a cyclohexyl group and the like; examples of the aryloxy group may include a phenoxy group, a naphthyloxy group and the like; examples of the alkylthio group may include a methylthio group, an ethylthio group, a propylthio group, a pentylthio group, a hexylthio group and the like; examples of the cycloalkylthio group may include a cyclopentylthio group, a cyclohexylthio group and the like; examples of the arylthio group may include a phenylthio group, a naphthylthio group and the like; examples of the alkoxycarbonyl group may include a methyloxycarbonyl group, an ethyloxycarbonyl group, a butyloxycarbonyl group, an octyloxycarbonyl group and the like; examples of the aryloxycarbonyl group may include a phenyloxycarbonyl group, a naphthyloxycarbonyl group and the like; examples of the sulfamoyl group may include an aminosulfonyl group, a methylaminosulfonyl group, a dimethylaminosulfonyl group, a cyclohexylaminosulfonyl group, a phenylaminosulfonyl group, a naphthylaminosulfonyl group, a 2-pyridylaminosulfonyl group and the like; examples of the acyl group may include an acetyl group, an ethylcarbonyl group, a propylcarbonyl group, a cyclohexylcarbonyl group, an octylcarbonyl group, a 2-ethylhexylcarbonyl group, a dodecylcarbonyl group, a phenylcarbonyl group, a naphthylcarbonyl group, a pyridylcarbonyl group and the like; examples of the acyloxy group may include an acetyloxy group, an ethylcarbonyloxy group, an octylcarbonyloxy group, a phenylcarbonyloxy group and the like; examples of the amide may include a methylcarbonylamino group, an ethylcarbonylamino group, a dimethylaminocarbonylamino group, a pentylcarbonylamino group, a cyclohexylcarbonylamino group, 2-ethylhexylcarbonylamino group, a phenylcarbonylamino group, a naphthylcarbonylamino group and the like; examples of the carbamoyl group may include an aminocarbamoyl group, a methylaminocarbamoyl group, a dimethylaminocarbamoyl group, a cyclohexylaminocarbamoyl group, a 2-ethylhexylaminocarbamoyl group, a phenylaminocarbamoyl group, a naphthylaminocarbamoyl group, a 2-pyridylaminocarbonyl group and the like; examples of the ureido group may include a methylureido group, an ethylureido group, a cyclohexylureido group, a dodecylureido group, a phenylureido group, a naphthylureido group, a 2-pyridylaminoureido group and the like; examples of the sulfinyl may include a methylsulfinyl group, an ethylsulfinyl group, a butylsulfinyl group, a cyclohexyl sulfinyl group, a 2-ethylhexylsulfinyl group, a phenylsulfinyl group, a naphthylsulfinyl group, a 2-pyridylsulfinyl group and the like; examples of the alkylsulfonyl group may include a methylsulfonyl group, an ethylsulfonyl group, a butylsulfonyl group, a cyclohexylsulfonyl group, a 2-ethylhexylsulfonyl group, a dodecylsulfonyl group and the like; examples of the arylsulfonyl group may include a phenylsulfonyl group, a naphthylsulfonyl group, a 2-pyridylsulfonyl group and the like; examples of the amino group may include an amino group, an ethylamino group, a dimethylamino group, a butylamino group, a 2-ethylhexylamino group, an anilino group, a naphthylamino group, a 2-pyridylamino group and the like; examples of the halogen atom may include a fluorine atom, a chlorine atom, a bromine atom, an iodine atom and the like; and examples of the fluorinated hydrocarbon group may include a fluoromethyl group, a trifluoromethyl group, a pentafluoroethyl group, a pentafluorophenyl group and the like. Further examples may also include a cyano group, a nitro group, a hydroxy group, a mercapto group, a silyl group and the like. Examples of the silyl group a trimethylsilyl group, a triisopropylsilyl group, a triphenylsilyl group, a phenyl group diethylsilyl and the like. Here, the above-described substituents may be further substituted with an above-described substituent. Further, a plurality of these substituents may be joined to each other to form a ring.

The method for manufacturing an electronic device according to the first to fourth embodiments of the present disclosure may further include a step of forming a first electrode and a second electrode on a second layer after the second layer has been formed. Further, the method for manufacturing an electronic device according to the fifth and sixth embodiments of the present disclosure may further include a step of forming a control electrode on a second layer after the second layer has been formed.

In the method for forming a laminated structure according to the first embodiment of the present disclosure, the method for manufacturing an electronic device according to the first, second or fifth embodiment of the present disclosure that include the above-described preferred modes, or alternatively, the preferred modes of the below-described laminated structure according to the embodiments of the present disclosure and the electronic device according to the fourth embodiment of the present disclosure, it is desirable that the rate at which the first layer is dissolved in the solvent when the second organic material solution layer has been formed on the first layer is more than 0 nm/min to 50 nm/min or less.

In the method for forming a laminated structure according to the first and second embodiments of the present disclosure that include the above-described preferred structures, and the method for manufacturing an electronic device according to the first to sixth embodiments of the present disclosure that include the above-described preferred modes and structures, it is preferred that the combination of the first organic material and the second organic material is configured by a combination of materials so that a change in Gibbs free energy before and after mixing the first organic material and the second organic material is positive. Namely, it is preferred that the value obtained by subtracting a

Gibbs free energy $G_1$ of the first organic material and a Gibbs free energy $G_2$ of the second organic material from a Gibbs free energy $G_0$ of a mixed system of the first organic material and the second organic material is positive.

In the laminated structure according to the embodiments of the present disclosure that includes the above-described preferred structures and modes, it is desirable that at the interface between the first layer and the second layer, the first organic material and the second organic material do not mix, and that the first layer and the second layer are separated. In this case, it is preferred that,
by forming on the first layer a second organic material solution layer in which the second organic material is dissolved in a solvent, the first organic material and the second organic material mix at an interface between the first layer and the second organic material solution layer due to a surface of the first layer being dissolved by the solvent included in the second organic material solution layer, and
when the second organic material solution layer has dried, the first layer and the second layer separate. Alternatively, in this case, it is preferred that,
by forming a first organic material solution layer in which a first organic material is dissolved in a first solvent and a second organic material solution layer in which a second organic material different from the first organic material is dissolved in a second solvent, and then drying the first organic material solution layer and the second organic material solution layer, the first organic material and the second organic material mix at an interface between the first organic material solution layer and the second organic material solution layer, and when first organic material solution layer and the second organic material solution layer have dried, the first layer and the second layer separate.

In the electronic device according to the fourth embodiment of the present disclosure, it is desirable that at the interface between the insulating layer and the active layer, the first organic material and the second organic material do not mix, and that the insulating layer and the active layer are separated. In this case, it is preferred that,
by forming on the first layer (the layer constituting the insulating layer or the active layer) a second organic material solution layer in which the second organic material (the material constituting the active layer or the insulating layer) is dissolved in a solvent, the first organic material and the second organic material mix at an interface between the first layer and a second organic material solution layer due to a surface of the first layer being dissolved by the solvent included in the second organic material solution layer, and
when the second organic material solution layer has dried, the first layer (the layer constituting the insulating layer or the active layer) and the second layer (the layer constituting the active layer or the insulating layer) separate; or alternatively, in this case, it is preferred that,
by forming a first organic material solution layer in which a first organic material is dissolved in a first solvent and a second organic material solution layer in which a second organic material different from the first organic material is dissolved in a second solvent, and then drying the first organic material solution layer and the second organic material solution layer, the first organic material and the second organic material mix at an interface between the first organic material solution layer and the second organic material solution layer, and when first organic material solution layer and the second organic material solution layer have dried, the first layer (the layer constituting the insulating layer or the active layer) and the second layer (the layer constituting the active layer or the insulating layer) separate.

In addition, in the laminated structure according to the embodiments of the present disclosure that includes the various above-described preferred modes and structures, the electronic device according to the fourth embodiment of the present disclosure that includes the various above-described preferred modes and structures, the method for forming a laminated structure according to the first and second embodiments of the present disclosure that includes the various above-described preferred modes and structures, and the method for manufacturing an electronic device according to the first to sixth embodiments of the present disclosure that include the various above-described preferred modes and structures, it is preferred that the first organic material and the second organic material are formed of a non-curable material. Here, non-curable material refers to a material in which crosslinking does not occur due to heat or ultraviolet light. Examples of the non-curable material may include amorphous polymer materials, such as poly-$\alpha$-methyl styrene and a cycloolefin copolymer.

In the laminated structure according to the embodiments of the present disclosure that includes the various above-described preferred modes and structures, the electronic device according to the fourth embodiment of the present disclosure that includes the various above-described preferred modes and structures, the method for forming a laminated structure according to the first and second embodiments of the present disclosure that includes the various above-described preferred modes and structures, and the method for manufacturing an electronic device according to the first to sixth embodiments of the present disclosure that include the various above-described preferred modes and structures (hereinafter, these are sometimes collectively referred to simply as "the laminated structure etc. according to the embodiments of the present disclosure"), when the surface of the first layer is dissolved by the solvent included in the second organic material solution layer, the depth to which the first layer is dissolved is preferably, although not limited to, $1\times10^{9}$ m to $1\times10^{-8}$ m from the surface of the first layer. In the case of forming a first organic material solution layer and a second organic material solution layer, the first organic material solution layer and the second organic material solution layer may be simultaneously formed, or the formation of the first organic material solution layer and the formation

of the second organic material solution layer may be consecutive. Namely, a method can be employed in which the first organic material solution layer and the second organic material solution layer are successively formed by simultaneously depositing using a laminar flow so that the first organic material solution and the second organic material solution do not mix to form the first organic material solution layer, and then immediately forming the second organic material solution layer.

In the laminated structure etc. according to the embodiments of the present disclosure, examples of the organic semiconductor material that includes a non-curable material and constitutes the first layer or the second layer include polymers and polycyclic condensation products, such as polypyrrole and its derivatives; polythiophene and its derivatives; an isothianaphthene, such as polyisothianaphthene; a thienylenevinylene, such as polythienylenevinylene; a poly(p-phenylenevinylene), such as poly(p-phenylenevinylene); polyaniline and its derivatives; polyacetylene; a polydiacetylene; a polyazulene; a polypyrene; a polycarbazole; a polyselenophene; a polyfuran; a poly(p-phenylene); a polyindole; a polypyridazine; polyvinylcarbazole, polyphenylenesulfide, and polyvinylene sulfide. Alternatively, examples may include an oligomer having the same repeating unit as these polymers. Alternatively, further example include an acene, such as naphthacene, pentacene[2,3,6,7-dibenzoanthracene] and its derivatives, anthracene derivatives, oligothiophene derivatives, hexacene, heptacene, dibenzopentacene, tetrabenzopentacene, pyrene, benzopyrene, dibenzopyrene, chrysene, perylene, coronene, terylene, ovalene, quaterrylene, and circumanthracene, and derivatives in which a part of the carbon atoms of the acene are substituted with a functional group such as an N atom, an S atom, and an O atom, or a carbonyl group (dioxaanthanthrene compounds including peri-xanthenoxanthene and its derivatives, triphenodioxazine, triphenodithiazine, hexacene-6,15-quinone, peri-xanthenoxanthene (PXX, 6,12-dioxaanthanthrene) etc.), and derivatives in which a hydrogen atom of these is substituted with another functional group. Alternatively, examples may further include metal phthalocyanines represented by copper phthalocyanine; tetrathiafulvalene and tetrathiafulvalene derivatives; tetrathiapentalene and its derivaties; tetracarboxylic acid diimides, such as naphthalene 1,4,5,8-tetracarboxylic acid diimide, N,N'-bis(4-trifluoromethylbenzyl)naphthalene 1,4,5,8-tetracarboxylic acid diimide, N,N'-bis(1H,1H-perfluorooctyl), N,N'-bis(1H,1H-perfluorobutyl), and N,N'-dioctylnaphthalene 1,4,5,8-tetracarboxylic acid diimide; naphthalene tetracarboxylic acid diimides, such as naphthalene 2,3,6,7-tetracarboxylic acid diimide; condensed ring tetracarboxylic acid diimides such as anthracene tetracarboxylic acid diimides, such as anthracene, 2,3,6,7-tetracarboxylic acid diimide; C60, C70, C76, C78, C84, etc. fullerenes and derivatives thereof; carbon nanotubes such as SWNT; and a pigment and its derivatives, such as a merocyanine pigment, a hemicyanine pigment and the like. Alternatively, further examples may include poly-3-hexylthiophene (P3HT) in which a hexyl group is introduced into polythiophene, polyanthracene, triphenylene, polyellurophene, polynaphthalene, polyethylenedioxythiophene, poly(3,4-ethylendioxythiophene)/polystyrenesulfonic acid (PEDOT/PSS), and quinacridone. Alternatively, further examples may include a compound selected from the group consisting of condensed polycyclic aromatic compounds, porphyrin derivatives, phenyl vinylidene-based conjugated oligomers, and thiophene-based conjugated oligomers. Specific examples thereof include condensed polycyclic aromatic compound such as acene-based molecules (pentacene, tetracene etc.), porphyrin molecules, and conjugated oligomers (phenyl vinylidene-based or thiophene-based). Alternatively, further examples may include porphyrin, 4,4'-biphenyldithiole (BPDT), 4,4'-diisocyanobiphenyl, 4,4'-diisocyano-p-terphenyl, 2,5-bis(5'-thioacetyl-2'-thiophenyl)thiophene, 2,5-bis(5'-thioacetyl-2'-thiophenyl)thiophene, 4,4'-diisocyanophenyl, benzidine (biphenyl-4-4'-diamine), TCNQ (tetracyanoquinodimethane), tetrathiafulvalene and its derivatives, charge-transfer complexes represented by a tetrathiafulvalene (TTF)-TCNQ complex, a bisethylenetetrathiafulvalene (BEDTTTF)-perchloric acid complex, a BEDTTTF-iodine complex, and a TCNQ-iodine complex, biphenyl-4,4'-dicarboxylic acid, 1,4-di(4-thiophenylacetylinyl)-2-ethylbenzene, 1,4-di(4-isocyanophenylacetylinyl)-2-ethylbenzene, dendrimer, 1,4-di(4-thiophenylethyl)-2-ethylbenzene, 2,2"-dihydroxy-1,1':4',1"-terphenyl, 4,4'-biphenyldiethanal, 4,4'-biphenyldiol, 4,4'-biphenylisocyanate, 1,4-diacetylbenzene, diethylbiphenyl-4,4'-dicarboxylate, benzo[1,2-c;3,4-c';5,6-c"]tris[1,2]dithiol-1,4,7-trithion, α-sexithiophene, tetrathiotetracene, tetraselenotetracene, tetratelluric tetracene, poly(3-alkylthiophene), poly(3-thiophene-[β]-ethane sulfonic acid), poly(N-alkylpyrrole)poly(3-alkylpyrrole), poly(3,4-dialkylpyrrole), poly(2,2'-thienylpyrrole), and poly(dibenzothiophene sulfide).

Further, in the laminated structure etc. according to the embodiments of the present disclosure, for example, a polymer material, such as a styrene resin, an olefin resin, a fluorene resin, a phenolic resin, and a novolac resin, can be used as the insulating material that is formed of a non-curable material and constitutes the first layer or the second layer.

The first solvent dissolving the first organic material and the solvent or second solvent dissolving the second organic material may be appropriately selected from among solvents capable of properly dissolving the first organic material or the second organic material to a desired concentration.

It is noted that in the laminated structure etc. according to the embodiments of the present disclosure, examples of a more preferred organic semiconductor material constituting the first layer or the second layer include peri-xanthenoxanthene compounds (PXX compounds), pentacene derivatives (TIPS-pentacene etc.), anthradithiophene derivatives (TES-ADT etc.), oligothiophene derivatives and the like, and examples of a more preferred insulating material constituting the first layer or the second layer include polyethylene, polypropylene, polyisoprene, polybutylene, polystyrene, polyvinyl xylene, cycloolefin polymer, polymethyl methacrylate, polycarbonate, polyvinyl cinnamate, fibroin and the like. Further,

examples of a more preferred solvent include aromatic solvents, such as o-xylene, m-xylene, p-xylene, toluene, chlorobenzene, chloronaphthalene, and Solvesso, ketone solvents, such as acetone and MEK, aliphatic solvents, such as hexane, ether solvents, such as PGMEA and PGME, ester solvents, such as ethyl acetate and butyl acetate, acetate solvents, such as cellosolve acetate, and chloroform. In addition, as the combination of the (more preferred organic semiconductor material constituting the first layer or the second layer) × (more preferred insulating material constituting the first layer or the second layer) × (more preferred solvent), preferred examples include, but are not especially limited to, (a PXX derivate) × (polystyrene, or, polyxylene, or, a cycloolefin polymer) × (various xylenes, or, toluene), (TIPS-pentacene) × (polymethyl methacrylate, or, polystyrene, or, polyxylene, or, a cycloolefin polymer) × (various xylenes, or, toluene, or chloroform, or. chlorobenzene).

The first insulating layer in the laminated structure etc. according to the embodiments of the present disclosure may be a monolayer, or may be multilayer. Examples of the material constituting the first insulating layer not only include an inorganic insulating material, such as a silicon oxide-based material, silicon nitride (SiNy), and a metal oxide high-dielectric insulating film, such as aluminum oxide ($Al_2O_3$) and $HfO_2$, but also a thermosetting resin, such as a phenol resin, a polyimide resin, a novolac resin, a cinnamate resin, an acrylic resin, an epoxy resin, and a poly-para-xylylene resin. These may also be used in combination. Here, examples of the silicon oxide-based material include oxidized silicon ($SiO_X$), BPSG, PSG, BSG, AsSG, PbSG, silicon oxynitride (SiON), SOG (spin-on glass), or a low-permittivity $SiO_2$-based material (e.g., polyarylether, cycloperfluorocarbon polymer and benzocyclobutene, a cyclic fluororesin, polytetrafluoroethylene, fluorinated aryl ether, fluorinated polyimide, amorphous carbon, and organic SOG). It is noted that examples of the method for forming the first insulating layer include, in addition to the below-described coating methods, below-described physical vapor deposition methods (PVD methods), and various chemical vapor deposition methods (CVD methods), optionally combining any of a lift-off method, a sol-gel method, an electrodeposition method, and a shadow mask method with a patterning technique. When forming the first layer formed of a first organic material on the first insulating layer, or alternatively, the first organic material solution layer in which a first organic material is dissolved in a first solvent and the second organic material solution layer in which a second organic material different from the first organic material is dissolved in a second solvent on the first insulating layer, it is preferred that the first insulating layer is constituted from a material in which the surface of the first insulating layer does not dissolve.

A coating method, for example, can be used as the method for forming the second organic material solution layer in the method for forming a laminated structure according to the first embodiment of the present disclosure, the method for forming the first organic material solution layer and the second organic material solution layer in the method for forming a laminated structure according to the second embodiment of the present disclosure, the method for forming the second organic material solution layer in the method for manufacturing an electronic device according to the first embodiment or the second embodiment of the present disclosure, the method for forming the first organic material solution layer and the second organic material solution layer in the method for manufacturing an electronic device according to the third embodiment or fourth embodiment of the present disclosure, the method for forming the second organic material solution layer in the method for manufacturing an electronic device according to the fifth embodiment of the present disclosure, or the method for forming the first organic material solution layer and the second organic material solution layer in the method for manufacturing an electronic device according to the sixth embodiment of the present disclosure. Here, examples of the coating method may include various printing methods, such as a screen printing method, an ink jet printing method, an offset printing method, a reverse offset printing method, a gravure printing method, a gravure offset printing method, relief printing, flexo printing, and a micro contact method; a spin coating method; various coating methods, such as an air doctor coater method, a blade coater method, a rod coater method, a knife coater method, a squeeze coater method, a reverse roll coater method, a transfer roll coater method, a gravure coater method, a kiss coater method, a cast coater method, a spray coater method, a slit coater method, a slit orifice coater method, a calender coater method, a casting method, a capillary coater method, a bar coater method, and a dipping method; a spray method; a method using a dispenser; and a method that coats a wet mat such as a stamp method. The first layer and the second layer may optionally be patterned based on a known method, such as a wet-etching method, a dry-etching method, or a laser ablation method. Further, in this case, it is preferred to coat the patterned first layer and second layer with a passivation film.

In addition to the above-described coating methods, various below-described PVD methods, including a resistance heating evaporation method, a sputtering method, and a vacuum deposition method, and various CVD methods, may also be used as the method for forming the first layer in the method for forming a laminated structure according to the first embodiment of the present disclosure, and the method for forming the first layer in the method for manufacturing an electronic device according to the first, second, and fifth embodiments of the present disclosure. It is noted that if a spin coating method or a spray method is employed as the method for forming the second organic material solution layer, since an in-plane distribution of the thickness of the second organic material solution layer can occur, sufficient care needs to be given to the employment of these methods.

The electronic device according to the first to third embodiments of the present disclosure may have a so-called three-terminal structure, or a two-terminal structure. In the former case, the electronic device further includes an insulating

layer and a control electrode that is disposed facing a portion of the active layer positioned between the first electrode and the second electrode via the insulating layer. Further, for example, a field-effect transistor (FET), specifically, a thin-film transistor (TFT), is configured by an electronic device having such a three-terminal structure, or alternatively, a light emitting element is configured by an electronic device having such a three-terminal structure. Namely, a light emitting element (an organic light emitting element, an organic light emitting transistor) in which an active layer emits light based on the application of a voltage to the control electrode and the first electrode and second electrode can be configured. In these electronic devices, the current flowing in the active layer from the first electrode to the second electrode is controlled based on the voltage applied to the control electrode. Here, in the light emitting element, the organic semiconductor material constituting the active layer has a light-emitting function based on charge storage due to modulation based on the voltage applied to the control electrode and recombination of the injected electrons and holes. Emission intensity, which is proportional to the absolute value of the current flowing from the first electrode to the second electrode, can be modulated the voltage applied to the control electrode and the voltage applied between the first electrode and the second electrode. Whether the electronic device exhibits a function as a field-effect transistor or as a light emitting element depends on the state (bias) of voltage application to the first and second electrodes. First, when the control electrode is modulated under a condition in which a bias is applied in a range where electrons are not injected from the second electrode, a current flows from the first electrode to the second electrode. This is a transistor operation. On the other hand, when the bias to the first electrode and the second electrode is increased under a condition in which holes have been sufficiently stored, electron injection starts, and light is emitted based on the recombination with holes. Further, an example of an electronic device having a two-terminal structure includes a photoelectric conversion element in which current flows between the first electrode and the second electrode by irradiation of light on the active layer. If a photoelectric conversion element is configured by the electronic device, specifically, a solar cell or various sensors, such as an image sensor or a light sensor, can be configured by the photoelectric conversion element. Alternatively, the electronic device can configure an organic electroluminescence element (organic EL element) or an organic EL display device, and can function as a chemical substance sensor. Namely, the electronic device can be used in a mode as a display element, a display device, a solar cell, or a sensor. It is noted that the photoelectric conversion element can also be configured from an electronic device having a three-terminal structure. In this case, a voltage may or may not be applied to the control electrode. If a voltage is applied, the current that is flowing can be modulated based on the application of the voltage to the control electrode. Further, the light emitting part of the organic EL element can also be configured by the dioxaanthanthrene compound according to the first to third embodiments of the present disclosure.

The first electrode and second electrode, and the active layer are formed on the base, or alternatively, above the base.

In the case of configuring a semiconductor device from the electronic device according to the first to third embodiments of the present disclosure, specific examples of the semiconductor device include a bottom-gate/bottom-contact type field-effect transistor (FET), a bottom-gate/top-contact type FET, a top-gate/bottom-contact type FET, and a top-gate/top-contact type FET.

If the semiconductor device is configured by a bottom-gate/bottom-contact type field-effect transistor (FET), this bottom-gate/bottom-contact type FET includes

(A) a gate electrode (control electrode) formed on a base,
(B) a gate insulating layer (insulating layer) formed on the gate electrode and the base,
(C) source/drain electrodes (first electrode and second electrode) formed on the gate insulating layer, and
(D) a channel formation region configured by an active layer, which is formed on the gate insulating layer between the source/drain electrodes.

Alternatively, if the semiconductor device is configured by a bottom-gate/top-contact type FET, this bottom-gate/top-contact type FET includes

(A) a gate electrode (control electrode) formed on a base,
(B) a gate insulating layer (insulating layer) formed on the gate electrode and the base,
(C) a channel formation region and a channel formation region extension portion which are formed on the gate insulating layer and are configured by an active layer, and
(D) source/drain electrodes (first electrode and second electrode) formed on the channel formation region extension portion.

Alternatively, if the semiconductor device is configured by a top-gate/bottom-contact type FET, this top-gate/bottom-contact type FET includes

(A) source/drain electrodes (first electrode and second electrode) formed on a base,

(B) a channel formation region which is formed on the base between the source/drain electrodes and is configured by an active layer,
(C) a gate insulating layer (insulating layer) formed on the source/drain electrodes and the channel formation region, and
(D) a gate electrode (control electrode) formed on the gate insulating layer.

Alternatively, if the semiconductor device is configured by a top-gate/top-contact type FET, this top-gate/top-contact type FET includes

(A) a channel formation region and a channel formation region extension portion which are formed on a base and are configured by an active layer,
(B) source/drain electrodes (first electrode and second electrode) formed on the channel formation region extension portion,
(C) a gate insulating layer (insulating layer) formed on the source/drain electrodes and the channel formation region, and
(D) a gate electrode (control electrode) formed on the gate insulating layer.

Further, based on the method for manufacturing an electronic device according to the first to fourth embodiments of the present disclosure that include the above-described predetermined modes and structures, a bottom-gate/top-contact type semiconductor device (specifically, a TFT) can be manufactured. Based on the method for manufacturing an electronic device according to the fifth and sixth embodiments of the present disclosure that include the above-described predetermined modes and structures, a top-gate/bottom-contact type semiconductor device (specifically, a TFT), which is a semiconductor device having a three-terminal structure, can be manufactured. In addition, examples of the electronic device according to the fourth embodiment of the present disclosure that has a three-terminal structure include a bottom-gate/top-contact type semiconductor device (specifically, a TFT), and a top-gate/bottom-contact type semiconductor device (specifically, a TFT).

Specifically, if the electronic device is a bottom-gate/top-contact type semiconductor device having a three-terminal structure,
a gate electrode formed on a base or in a base is configured by a control electrode,
a gate insulating layer formed on the gate electrode and the base is configured by an insulating layer,
a channel formation region and a channel formation region extension portion formed on the gate insulating layer are configured by an active layer, and
and a pair of source/drain electrodes formed on the channel formation region extension portion is configured by a first electrode and a second electrode. Here, the electrode structure is configured by a control electrode (gate electrode), and the first electrode and second electrode (pair of source/drain electrodes).

Further, if the electronic device is a top-gate/bottom-contact type semiconductor device having a three-terminal structure,
a pair of source/drain electrodes formed on a base is configured by a first electrode and a second electrode,
a channel formation region formed on the pair of source/drain electrodes, and, a channel formation region extension portion formed on the source/drain electrodes, are configured by an active layer,
a gate insulating layer formed on the channel formation region and the channel formation region extension portion is configured by an insulating layer, and
a gate electrode formed on the gate insulating layer facing the channel formation region is configured by a control electrode. Here, the electrode structure is configured by a control electrode (gate electrode), and the first electrode and second electrode (pair of source/drain electrodes).

Here, the base can be configured by a silicon oxide-based material (e.g., $SiO_x$, spin-on glass (SOG), silicon oxynitride (SiON)): silicon nitride ($SiN_Y$); a metal oxide high-dielectric insulating film, such as aluminum oxide ($Al_2O_3$) and $HfO_2$; metal oxides; and metal salts. If the base is configured by these materials, the base may be formed on a support (or above a support) appropriately selected from among the materials listed below. Namely, examples of the support, or alternatively, a base other than the above-described base, include organic polymers (in the form of a polymer material of a flexible plastic film, a plastic sheet, or a plastic substrate configured by a polymer material), such as polymethylmethacrylate (PMMA), polyvinyl alcohol (PVA), polyvinyl phenol (PVP), polyether sulfone (PES), polyimide, polyamide, polyacetal, polycarbonate (PC), polyethylene terephthalate (PET), polyethylene naphthalate (PEN), polyethyl ether ketone, polyolefins and the like. Alternatively, examples may include natural mineral-based insulating materials, such as mica, metal-based semiconductor materials, molecular semiconductor materials and the like. If a base configured by such a flexible polymer material is used, for example, the electronic device can be mounted on or integrated with an image display device (display device) or electronic equipment having a curved surface shape. Alternatively, further examples of the base include various glass substrates, various glass substrates in which an insulating film is formed on

the surface, a quartz substrate, a quartz substrate in which an insulating film is formed on the surface, a silicon substrate in which an insulating film is formed on the surface, a sapphire substrate, a conductive substrate (various metals such as gold, aluminum, stainless steel, and nickel, a substrate or a foil configured by various alloys, a substrate including highly-oriented graphite) in which an insulating film is formed on the surface, and paper. As the support having an electrical insulating property, a suitable material may be selected from among the above-described materials. Further examples of the support include a conductive substrate (a substrate including a metal such as gold and aluminum, a substrate including highly-oriented graphite, a stainless steel substrate etc.). In addition, depending on the mode and structure of the electronic device, the electronic device may be disposed on a support member, and this support member may be configured by the above-described materials. A buffer layer for improving adhesive properties and flatness, a barrier film for improving gas barrier properties and the like may also be formed on the above-described base.

Examples of the material constituting the control electrode, first electrode, second electrode, gate electrode, source/drain electrodes, and wiring (hereafter, these are collectively referred to as "control electrode etc.") include metals, such as platinum (Pt), gold (Au), palladium (Pd), chromium (Cr), nickel (Ni), aluminum (Al), silver (Ag), tantalum (Ta), tungsten (W), copper (Cu), titanium (Ti), indium (In), tin (Sn), iron (Fe), cobalt (Co), zinc (Zn), magnesium (Mg), manganese (Mn), ruthenium (Rh), a rubidium (Rb), and molybdenum (Mo), or, conductive substances, such as an alloy including these metals elements, conductive particles including these metals, conductive particles including an alloy of these metals, polysilicon containing impurities, a carbon material and the like. A laminated structure layers including these elements can also be used. Further examples of the material constituting the control electrode etc. include an organic material (conductive polymer), such as poly (3,4-ethylenedioxythiophene)/polystyrene sulfonate [PEDOT/PSS], TTF-TCNQ, and poly aniline. The materials which constitute the control electrode etc. may be the same material or a different material.

Although the method for forming the control electrode etc. depends on the materials constituting these parts, examples may include a physical vapor deposition method (PVD method); pulsed laser deposition (PLD), an arc discharge method; various chemical vapor deposition methods including an MOCVD method; a spin coating method; various printing methods, such as a screen printing method, an ink jet printing method, an offset printing method, a reverse offset printing method, a gravure printing method, a gravure offset printing method, relief printing, flexo printing, and a micro contact method; various coating methods, such as an air doctor coater method, a blade coater method, a rod coater method, a knife coater method, a squeeze coater method, a reverse roll coater method, a transfer roll coater method, a gravure coater method, a kiss coater method, a cast coater method, a spray coater method, a slit coater method, a slit orifice coater method, a calender coater method, a casting method, a capillary coater method, a bar coater method, and a dipping method; a stamp method; a casting method; a method using a dispenser; a spray method; a lift-off method; a shadow mask method; as well as a combination of any plating method, such as an electrolytic plating method, an electroless plating method, or a combination thereof, with optionally a patterning technique. Examples of the PVD method include (a) an electron beam heating method, a resistance heating evaporation method, various vacuum deposition methods, such as flash evaporation, a method of heating a crucible and the like (b) a plasma evaporation method, (c) various sputtering methods, such as a diode sputtering method, a direct-current sputtering method, a direct-current magnetron sputtering method, a high-frequency sputtering method, a magnetron sputtering method, an ion beam sputtering method, a bias sputtering method and the like, and (d) various ion ion plating methods, such as a DC (direct current) method, a RF method, a multi-cathode method, an activation reaction method, a field evaporation method, a high-frequency ion plating method, a reactive ion plating method and the like. When the control electrode etc. are formed based on an etching method, a dry-etching method or a wet-etching method may be employed. Examples of dry-etching methods include ion milling and reactive ion etching (RIE). Further, the control electrode etc. may also be formed based on a laser ablation method, a mask evaporation method, a laser transfer method and the like.

Examples of the material constituting the insulating layer (the gate insulating layer) not only include an inorganic insulating material, such as a silicon oxide-based material; silicon nitride ($SiN_Y$); and a metal oxide high-dielectric insulating film, such as aluminum oxide ($Al_2O_3$) and $HfO_2$, but also an organic insulating material (organic polymer), such as a straight-chain hydrocarbon having on one end a functional group that can be bonded to the control electrode etc. (the gate electrode), such as polymethylmethacrylate (PMMA); polyvinyl phenol (PVP); polyvinyl alcohol (PVA); polyimide; polycarbonate (PC); polyethylene terephthalate (PET); polystyrene; a silanol derivative (silane coupling agent) such as N-2(aminoethyl)-3-aminopropyltrimethoxysilane (AEAPTMS), 3-mercaptopropyltrimethoxysilane (MPTMS), or octadecyltrichlorosilane (OTS); octadecanethiol; and dodecyl isocyanate). A combination of these may also be used. Here, examples of the silicon oxide-based material include oxidized silicon ($SiO_X$), BPSG, PSG, BSG, AsSG, PbSG, silicon oxynitride (SiON), SOG (spin-on glass), or a low-permittivity material (e.g., polyarylether, cycloperfluorocarbon polymer and benzocyclobutene, a cyclic fluororesin, an amorphous resin (e.g., CYTOP manufactured by Asahi Glass Co., Ltd.), polytetrafluoroethylene, fluorinated aryl ether, fluorinated polyimide, amorphous carbon, and organic SOG).

The insulating layer (gate insulating layer) can be formed by the above-described various PVD methods; various CVD methods, a spin coating method; the above-described various printing methods; the above-described various coating methods; a dipping method; a casting method; a sol-gel method; an electrodeposition method; a shadow mask

method; as well as any spray method. Alternatively, the insulating layer can also be formed by oxidizing or nitriding the surface of the control electrode (gate electrode), or obtained by forming an oxide film or a nitride film on the surface of the control electrode. Although the method of oxidizing the surface of the control electrode depends on the material constituting the control electrode, examples may include an oxidizing method using $O_2$ plasma or an anodization method. Further, although the method of nitriding the surface of the control electrode depends on the materials constituting the control electrode, examples may include a nitriding method using $N_2$ plasma. Alternatively, for an Au electrode, the insulating layer (gate insulating layer) can also be formed on the surface of the control electrode (the gate electrode) by, for example, covering the control electrode surface in a self-organizing manner by a method such as a dipping method with insulating molecules having a functional group capable of forming a chemical bond with the control electrode, like a linear hydrocarbon in which one end is modified by a mercapto group. Alternatively, the insulating layer (the gate insulating layer) may be formed by modifying the surface of the control electrode (the gate electrode) with a silanol derivative (silane coupling agent).

Examples of the method used for forming the active layer, or the channel formation region and the channel formation region extension portion, may include, but are not limited to, the above-described various printing methods; various coating methods; a method using a dispenser; a pin-coating method; and any wet spray method. In some cases, the above-described various PVD methods; CVD methods; a lift-off method; or a shadow mask method can also be employed. An additive (e.g., so-called doping material, such as an n-type impurity or a p-type impurity) can also be added to the dioxaanthanthrene compound.

Examples of devices in which the electronic device according to the first to fourth embodiments of the present disclosure is mounted may include, but are not limited to, an image display device. Here, examples of an image display device may include a so-called desktop type personal computer, a notebook type personal computer, a mobile type personal computer, a PDA (personal digital assistant), a mobile phone, a game machine, electronic paper such as an electronic book and an electronic newspaper, a message board such as a signboard, a poster, and a blackboard, a copy machine, rewritable paper to substitute for printer paper, a calculator, a display unit in household appliances, a card display unit such as a point card, and various image display devices in electronic advertizing and electronic POP (e.g., an organic electroluminescence display device, a liquid crystal display device, a plasma display device, an electrophoretic display device, a cold cathode field emission display device etc.). Further examples include various lighting apparatuses.

If the electronic device is applied or used in various image display devices or various electronic machines, the used electronic device may be used as a monolithic integrated circuit in which multiple electronic devices have been integrated on a support member, or each electronic device may be individually separated and used as a discrete component. Further, the electronic device may be sealed with a resin.

Working Example 1

Working Example 1 relates to the dioxaanthanthrene compound (hereinafter abbreviated to "PXX compound") according to the first embodiment of the present disclosure. The PXX compound of Working Example 1 is represented by the following structural formula (1), more specifically, the following structural formula (1A). Namely, in the PXX compound of Working Example 1, "X" is oxygen (O), Y is sulfur (S), and $A_1$ and $A_2$ are a hydrogen (H) atom. Further, "R" is a para-isobutylphenyl group.

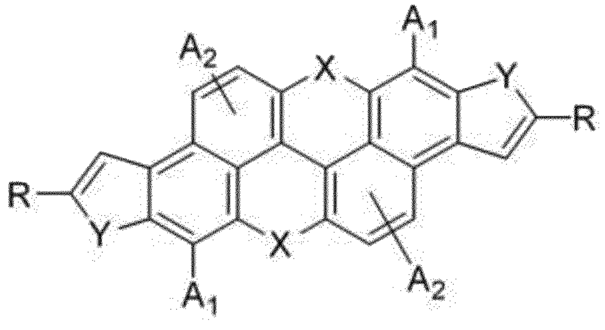

(1)

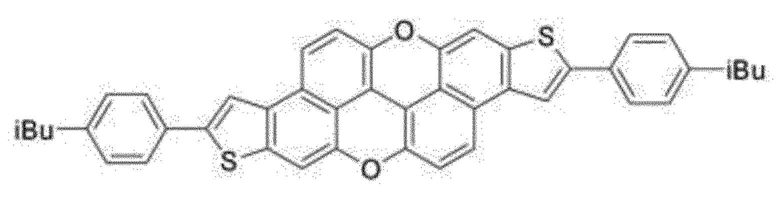

(1A)

literature "Journal of Organic Chemistry, 2010, 75, 8241-8251". Then, compound 3, which is obtained by reacting NBS or bromine with compound 2, and (para-isobutylphenyl)boronic acid are cross-coupled in the presence of a palladium catalyst, to obtain compound 4. Further, compound 4 is reacted with boron tribromide to obtain demethylated compound 5. This compound 5 is cross-coupled in the presence of iron(III) chloride to obtain compound 6. Next, the target PXX compound of Working Example 1 is obtained by carrying out a cyclization reaction by reacting compound 6 with copper acetate under basic conditions.

The PXX compound of Working Example 1 is stable in air, and can be easily isolated.

Working Example 2

Working Example 2 relates to the dioxaanthanthrene compound (hereinafter abbreviated to "PXX compound,") according to the second embodiment of the present disclosure. The PXX compound of Working Example 2 is represented by the following structural formula (11), more specifically, the following structural formula (11A). It is noted that, for convenience, the PXX compound having this structure is referred to as "i-$C_4$Ph-PXX" of Working Example 2A. In the i-$C_4$Ph-PXX of Working Example 2A, the third and ninth sites of 6,12-dioxaanthanthrene are substituted with a phenyl group, and, the fourth site (para) of each phenyl group is substituted with an isobutyl group.

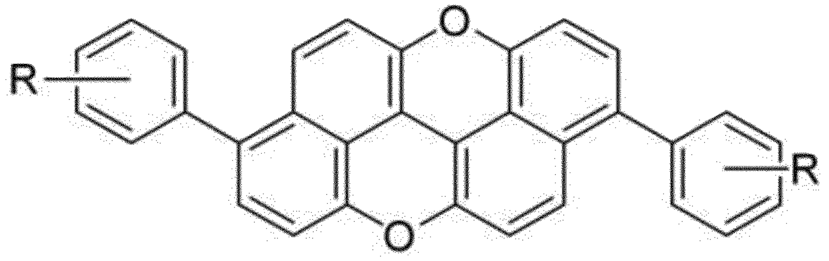

(11)

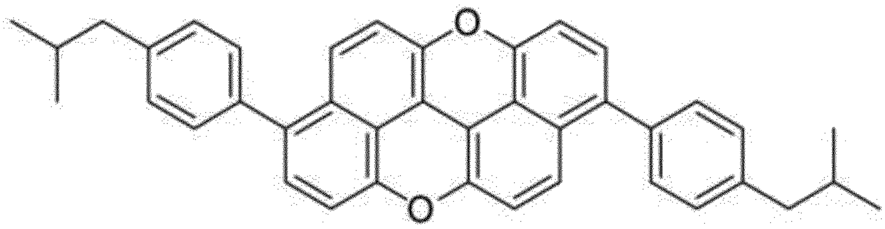

(11A)

This PXX compound (i-$C_4$Ph-PXX) of Working Example 2 can be obtained by reacting peri-xanthenoxanthene and bromine to obtain 3,9-dibromo-perixanthenoxanthene, and then performing a cross-coupling reaction with 3.5 equivalents of (para-isobutylphenyl)boronic acid in the presence of a palladium catalyst.

Alternatively, the PXX compound of Working Example 2 is represented by the following structural formula (11B). It is noted that, for convenience, the PXX compound having this structure is referred to as "i-$C_5$Ph-PXX" of Working Example 2B. In the i-$C_5$Ph-PXX of Working Example 2B, the third and ninth sites of 6,12-dioxaanthanthrene are substituted with a phenyl group, and, the fourth site (para) of each phenyl group is substituted with an isopentyl group.

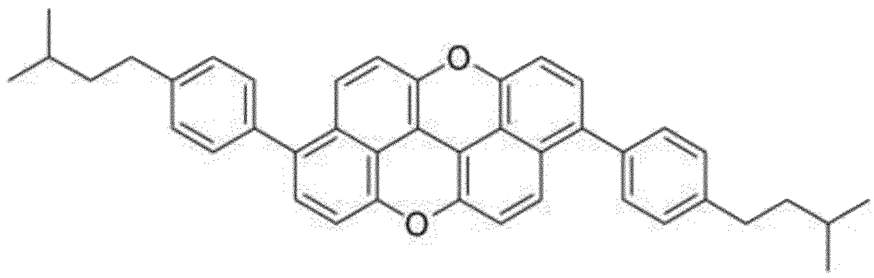

(11B)

This PXX compound (i-$C_5$Ph-PXX) of Working Example 2 can be obtained by reacting peri-xanthenoxanthene and bromine to obtain 3,9-dibromo-perixanthenoxanthene, and then performing a cross-coupling reaction with 3.5 equivalents of 1-pinacolboryl-4-isopentylbenzene in the presence of a palladium catalyst. It is noted that 1-pinacolboryl-4-isopentylbenzene can be obtained by subjecting 1-bromo-4-isopentylbenzene, which is obtained from bromobenzene

via a Friedel-Crafts acylation reaction and a Wolff-Kishner reduction reaction, to a boration reaction.

The melting point and the mesophase transition temperature of the PXX compound can be determined by TG-DTA (thermogravimetry/differential thermal analysis) and DSC (differential scanning calorimetry). Further, solubility in an organic solvent was measured. Specifically, how many grams of PXX compound would dissolve in 10 grams of toluene was measured. The measurement results are shown in Table 1. In Table 1, the mesophase transition temperature is denoted as "transition temperature". The results of how many grams of PXX compound would dissolve in 10 grams of toluene is denoted as "solubility" (units: grams).

The melting point and the mesophase transition temperature of a compound ($C_3$Ph-PXX) having the following structural formula (A) as Comparative Example 2A, a compound ($C_4$Ph-PXX) having the following structural formula (B) as Comparative Example 2B, a compound ($C_5$Ph-PXX) having the following structural formula (C) as Comparative Example 2C, and a compound (i-$C_3$Ph-PXX) having the following structural formula (D) as Comparative Example 2D were also measured. The measurement results are shown in the following Table 1.

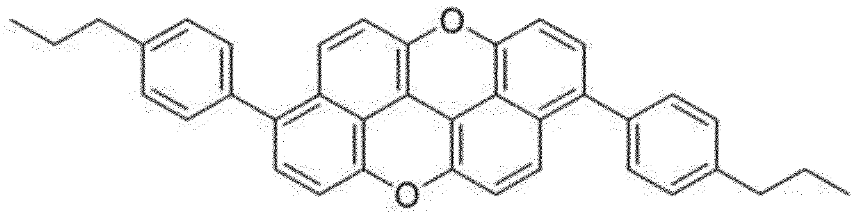

(A)

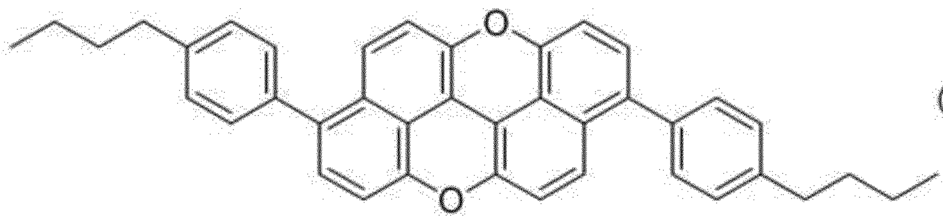

(B)

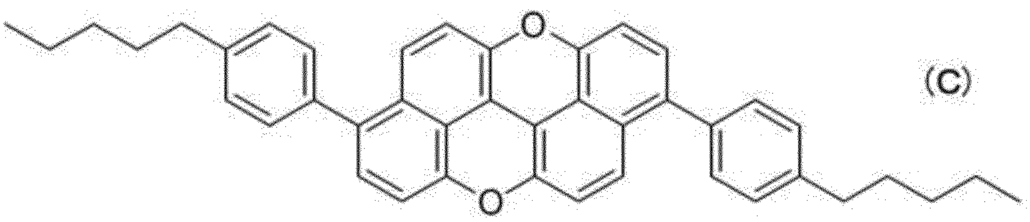

(C)

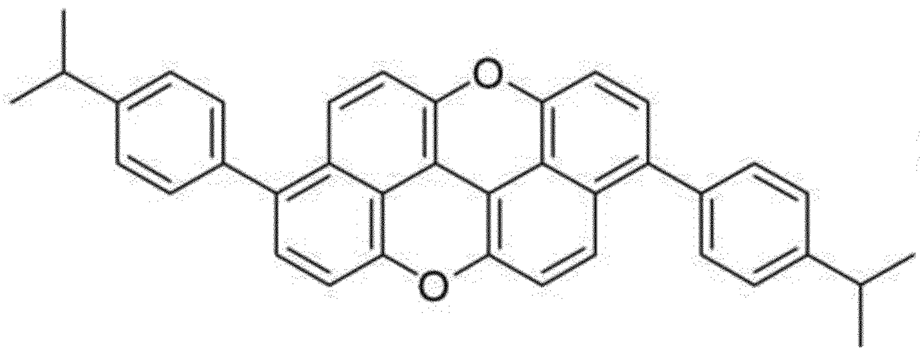

(D)

The PXX compounds (i-$C_4$Ph-PXX, i-$C_5$Ph-PXX) of Working Example 2 exhibited a higher melting point than the compounds of Comparative Examples 2A, 2B, and 2C. Further, for the PXX compounds of Working Example 2, a mesophase transition temperature could not be measured. Namely, mesophase transition did not occur. On the other hand, although Comparative Example 2D exhibited a high melting point, and mesophase transition did not occur, only 0.37 grams dissolved in 100 grams of toluene, so that solubility in an organic solvent was poorer than the PXX compounds of Working Example 2. In other words, the PXX compounds of Working Example 2 have a high solubility.

Table 1

| | | Melting Point | Transition Temperature | Solubility |
|---|---|---|---|---|
| Working Example 2A | i-C4Ph-PXX | 251 °C | Not found | 1.5g |
| Working Example 2B | i-C5Ph-PXX | 245°C | Not found | 1.3g |
| Comparative Example 2A | C3Ph-PXX | 242°C | Around 188°C | --- |
| Comparative Example 2B | C4Ph-PXX | 231°C | Around 220°C | --- |
| Comparative Example 2C | C5Ph-PXX | 217°C | Around 77°C | --- |
| Comparative Example 2D | i-C3Ph-PXX | >280°C | Not found | 0.37g |

Thus, for the dioxaanthanthrene compound of Working Example 2, because R is an alkyl group having a branch with four or more carbon atoms, liquid crystallinity is not exhibited even under a high-temperature atmosphere, namely, there are no changes in the molecular arrangement. Further, this dioxaanthanthrene compound has a high melting point.

Working Example 3

Working Example 3 relates to the dioxaanthanthrene compound (PXX compound) according to the third and fourth embodiments of the present disclosure. The PXX compound of Working Example 3 is represented by the following structural formula (21-1), or alternatively, the following structural formula (23-1). It is noted that this PXX compound is referred to as the PXX compound of Working Example 3A. Here, in structural formula (21-1), substituent A is represented by the above-described structural formula (22-1). Further, the $X_1$, $X_2$, $X_3$, $Y_1$, $Y_2$, $Y_3$, $Y_4$, $Y_5$, $Y_6$, $Y_7$, and $Y_8$ in structural formula (22-1) each represent a hydrogen atom, the third and ninth sites of 6,12-dioxaanthanthrene are substituted with a phenyl group, and, a cyclic alkyl group (cyclic alkane) is introduced on the third and fourth sites of the phenyl group.

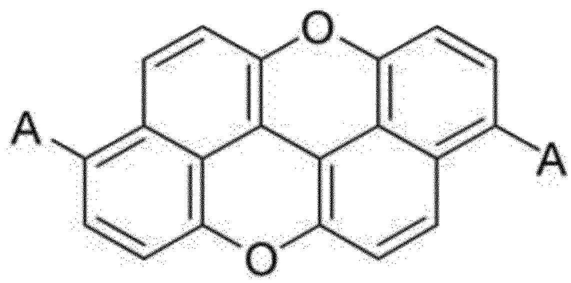

(21-1)

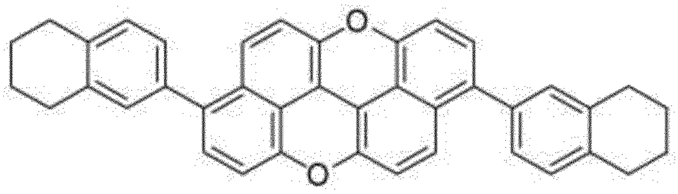

(23-1)

This PXX compound of Working Example 3A can be obtained by reacting peri-xanthenoxanthene and bromine to obtain 3,9-dibromo-peri-xanthenoxanthene, and then performing a cross-coupling reaction with 3.5 equivalents of (5,6,7,8-tetrahydro-2-naphthalenyl)boronic acid in the presence of a palladium catalyst.

Alternatively, the PXX compound of Working Example 3 is represented by the above structural formula (21-1), or alternatively, the following structural formula (23-2). It is noted that this PXX compound is referred to as the PXX compound of Working Example 3B. Here, in structural formula (21-1), substituent A is represented by the above-described structural formula (22-2). Further, the $X_1$, $X_2$, $X_3$, $Y_1$, $Y_2$, $Y_3$, $Y_4$, $Y_5$, $Y_6$, $Y_7$, and $Y_8$ in structural formula (22-2) each represent a hydrogen atom, the third and ninth sites of 6,12-dioxaanthanthrene are substituted with a phenyl group, and, a cyclic alkyl group (cyclic alkane) is introduced on the second and third sites of the phenyl group.

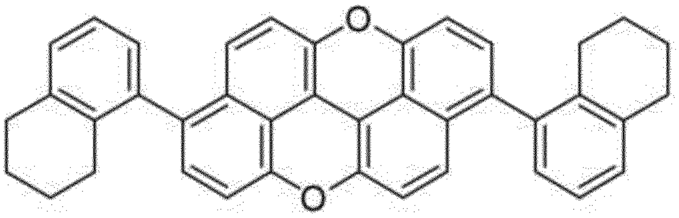

(23−2)

This PXX compound of Working Example 3B can be obtained by reacting peri-xanthenoxanthene and bromine to obtain 3,9-dibromo-peri-xanthenoxanthene, and then performing a cross-coupling reaction with 3.5 equivalents of (5,6,7,8-tetrahydro-1-naphthalenyl)boronic acid in the presence of a palladium catalyst.

The melting point and the mesophase transition temperature of the PXX compound can be determined by TG-DTA (thermogravimetry/differential thermal analysis) and DSC (differential scanning calorimetry).

The melting point and the mesophase transition temperature of a compound ($C_4$Ph-PXX) having the following structural formula (E) as Comparative Example 3A were also measured. The measurement results are shown in the following Table 2.

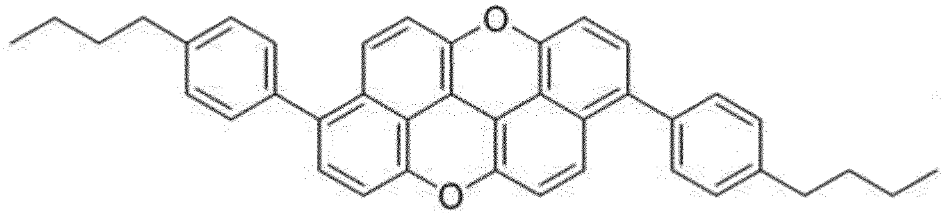

(E)

The PXX compound of Working Example 3A exhibited a higher melting point than the compound of Comparative Examples 3A. Further, for the PXX compounds of Working Examples 3A and 3B, a mesophase transition temperature could not be measured. Namely, mesophase transition did not occur.

Table 2

| | Melting Point | Transition Temperature |
|---|---|---|
| Working Example 3A | 349°C | Not found |
| Working Example 3B | | Not found |
| Comparative Example 3A | 231°C | Around 220°C |

Thus, for the dioxaanthanthrene compound of Working Example 3, because the third and ninth sites of the 6,12-dioxaanthanthrene are substituted with a phenyl group, and, a cyclic alkyl group (cyclic alkane) is introduced on the phenyl group, liquid crystallinity is not exhibited even under a high-temperature atmosphere. Namely, there are no changes in the molecular arrangement. Further, the dioxaanthanthrene compound has a high melting point.

Working Example 4

Working Example 4 relates to the electronic device according to the first to third embodiments of the present disclosure that have a semiconductor layer including the dioxaanthanthrene compound according to the first embodiment of the present disclosure to Working Example 3. The electronic devices of Working Example 4 or the below Working Examples 5 to 8 include
a first electrode;
a second electrode disposed separated from the first electrode; and
an active layer formed of an organic semiconductor material provided from the first electrode to the second electrode, in which the organic semiconductor material includes the dioxaanthanthrene compound according to the first to third embodiments of the present disclosure that include the above-described various preferred modes. Specifically, the electronic devices of Working Example 4 and the below Working Examples 5 to 7 are three-terminal type electronic devices that include

(A) a control electrode,
(B) a first electrode and a second electrode, and

(C) an active layer including a metal oxide semiconductor that is provided between the first electrode and the second electrode and is disposed facing the control electrode via an insulating layer.

More specifically, the three-terminal type electronic devices of Working Example 4 and the below Working Examples 5 to 7 are field-effect transistors (FETs) in which the current flowing in an active layer from a first electrode to a second electrode is controlled based on the voltage applied to a control electrode, in which the control electrode corresponds to a gate electrode, the first electrode and the second electrode correspond to source/drain electrodes, an insulating layer corresponds to a gate insulating layer film, and the active layer corresponds to a channel formation region.
Namely, as illustrated in the schematic partial end view of FIG. 2B, the electronic device of Working Example 4 is a semiconductor device, specifically, a bottom-gate/bottom-contact type field-effect transistor (more specifically, a thin-film transistor (TFT)), which includes

(A) a gate electrode 14 (corresponding to the control electrode) formed on a base 10,
(B) a gate insulating layer 15 (corresponding to an insulating layer) formed on the gate electrode 14 and the base 10,
(C) source/drain electrodes 16 (corresponding to the first electrode and the second electrode) formed on the gate insulating layer 15, and
(D) a channel formation region 17 configured by an active layer 20, which is formed on the gate insulating layer 15 between the source/drain electrodes 16.

An outline of the method for manufacturing the electronic device (field-effect transistor) of Working Example 4 will now be described with reference to FIGS. 2A and 2B, which are schematic partial end views of the base and the like.

Step-400

First, the gate electrode 14 is formed on the base 10. Specifically, based on a photolithography technique, a resist layer (not illustrated), from which the portion where the gate electrode 14 is to be formed has been removed, is formed on the insulating film 12 including $SiO_2$ that is formed on the surface of the glass substrate 11. Then, a titanium (Ti) layer (not illustrated) as an adhesion layer and a gold (Au) layer as the gate electrode 14 are successively deposited on the whole face by a vacuum deposition method, after which the resist layer is removed. In this way, based on a so-called lift-off method, the gate electrode 14 can be obtained (refer to FIG. 8A). It is noted that the gate electrode 14 can also be formed on the insulating film 12 including $SiO_2$ that is formed on the surface of the glass substrate 11 based on a printing method.

Step-410

Next, the gate insulating layer 15 corresponding to the insulating layer is formed on the base 10 (more specifically, the insulating film 12 formed on the surface of the glass substrate 11) including the gate electrode 14. Specifically, the gate insulating layer 15 that includes $SiO_2$ is formed on the gate electrode 14 and the insulating film 12 based on a sputtering method. When depositing the gate insulating layer 15, an extraction portion (not illustrated) of the gate electrode 14 can be formed without using a photolithography process by covering a part of the gate electrode 14 with a hard mask.

Step-420

Then, source/drain electrodes 16 including a 1 nm-thick chromium (Cr) layer (not illustrated) as an adhesion layer and a 25 nm-thick gold (Au) layer are successively deposited on the gate insulating layer 15 by a vacuum deposition method (refer to FIG. 2A). When depositing these layers, the source/drain electrodes 16 can be formed without using a photolithography process by covering a part of the gate insulating layer 15 with a hard mask. It is noted that the source/drain electrodes 16 can also be formed on the gate insulating layer 15 based on a printing method.

Step-430

Next, if the dioxaanthanthrene compound described in Working Example 1 or 2 is used, the channel formation region 17 (the active layer 20) is formed on the gate insulating layer 15 and the source/drain electrodes 16 by a wet method (coating method). Specifically, the channel formation region 17 can be formed on the gate insulating layer 15 and the source/drain electrodes 16 by coating a PXX compound solution, in which the PXX compound obtained in Working Example 1 is dissolved in a solvent, such as an aromatic compound such as toluene or xylene, a long-chain hydrocarbon alcohol such as octyl alcohol or nonyl alcohol and the like, on the whole face by a spin coating method, and drying (refer to FIG. 2B). Alternatively, the channel formation region 17 can be formed on the gate insulating layer

15 and the source/drain electrodes 16 by coating a PXX compound solution, in which the PXX compound of Working Example 2 is dissolved in toluene, on the whole face by a spin coating method, and drying. It is noted that the channel formation region 17 can optionally be patterned.

Alternatively, if the dioxaanthanthrene compound described in Working Example 3 is used, the channel formation region 17 (the active layer 20) is formed on the gate insulating layer 15 and the source/drain electrodes 16 based on a PVD method. Specifically, the channel formation region 17 can be formed on the gate insulating layer 15 and the source/drain electrodes 16 by depositing the PXX compound of Working Example 3A or 3B on the whole face by a vacuum deposition method.

For example, in the manufacture of an image display device, following on from this step, an image display unit (specifically, an image display unit including an organic electroluminescence element, an electrophoretic display element, a semiconductor light emitting element or the like) can also be formed based on a known method on or above the thus-obtained TFT. In each of the below-described working examples too, an image display unit can be obtained by carrying out a similar step after manufacture of the electronic device (TFT) is completed.

Step-440

Alternatively, a bottom-gate/bottom-contact type FET (specifically, a TFT) can be obtained by forming a passivation film (not illustrated) on the whole face.

For the electronic device of Working Example 4 in which the dioxaanthanthrene compound described in Working Example 1 is used in the channel formation region (the electronic device according to the first embodiment of the present disclosure), the $\pi$-conjugated system can be widened by fusing a thiophene ring to a PXX skeleton, which has a proven record relating to stability and high mobility. Namely, a large intermolecular interaction can be obtained due to the formation of a much wider (larger) $\pi$-orbital overlap between adjacent PXX compound molecules. Consequently, a much greater improvement in carrier mobility can be achieved. Further, since a large amount of intermolecular contact can be expected based on the sulfur atoms that project out at the periphery of the PXX compound molecules, intermolecular orbital overlap is greatly increased, so that much higher conductivity can be exhibited. In addition, introducing a substituent to a predetermined position of the PXX compound molecules allows the solubility, the molecular arrangement and the like to be controlled, and also allows a high-mobility organic semiconductor that can be coated or printed to be easily obtained. Still further, by fusing a sulfur-containing ring (thiophene ring) to a site on the PXX skeleton having a high electron density, reactivity with oxygen in the air can be suppressed, and the molecular HOMO level can be deepened. As a result, an organic semiconductor material that is stable in air can be provided.

Further, for the electronic device of Working Example 4 in which the dioxaanthanthrene compound described in Working Example 2 is used in the channel formation region (the electronic device according to the second embodiment of the present disclosure), liquid crystallinity is not exhibited even under a high-temperature atmosphere, namely, there is no change in the molecular arrangement, so that the properties of the electronic device are less susceptible to changing even under a high-temperature atmosphere. Further, the melting point is high enough to avoid being affected by high-temperature processes during the manufacturing steps of the electronic device and the usage environment, usage conditions and the like of the electronic device. For example, if the TFT is mounted in a liquid crystal display device, and used to drive a liquid crystal display element, although the temperature of the TFT can increase due to the heat emitted by a so-called backlight arranged at the rear face of the liquid crystal display device, for the TFT of Working Example 4, the properties of the TFT are less susceptible to change even under high-temperature atmosphere.

Further, for the electronic device of Working Example 4 in which the dioxaanthanthrene compound described in Working Example 3 is used in the channel formation region (the electronic device according to the third embodiment of the present disclosure), liquid crystallinity is not exhibited even under a high-temperature atmosphere, namely, there is no change in the molecular arrangement, so that the properties of the electronic device are less susceptible to changing even under a high-temperature atmosphere. Further, the melting point is high enough to avoid being affected by high-temperature processes during the manufacturing steps of the electronic device and the usage environment, usage conditions and the like of the electronic device. For example, if the TFT is mounted in a liquid crystal display device, and used to drive a liquid crystal display element, although the temperature of the TFT can increase due to the heat emitted by a so-called backlight arranged at the rear face of the liquid crystal display device, for the TFT of Working Example 4, the properties of the TFT are less susceptible to change even under high-temperature atmosphere.

Working Example 5

Working Example 5 is a modification of Working Example 4. In Working Example 5, the three-terminal type electronic device is a bottom-gate/top-contact type FET (specifically, a TFT). As illustrated in the schematic partial end view of FIG. 3B, the field-effect transistor of Working Example 5 includes

(A) the gate electrode 14 (corresponding to the control electrode) formed on the base 10,
(B) the gate insulating layer 15 (corresponding to an insulating layer) formed on the gate insulating layer 15 and the base 10,
(C) the channel formation region 17 and the channel formation region extension portion 18 which are formed on the gate insulating layer 15 and are configured by the active layer 20, and
(D) source/drain electrodes 16 (corresponding to the first electrode and the second electrode) formed on the channel formation region extension portion 18.

An outline of the method for manufacturing the electronic device (field-effect transistor) of Working Example 5 will now be described with reference to FIGS. 3A and 3B, which are schematic partial end views of the base and the like.

Step-500

First, the gate electrode 14 is formed on the base 10 in the same manner as in "Step-400" of Working Example 4, and then the gate insulating layer 15 is formed on the base (more specifically, the insulating film 12) including the gate electrode 14 in the same manner as in "step-410" of Working Example 4.

Step-510

Next, the active layer 20 is formed on the gate insulating layer 15 in the same manner as in "Step-430" of Working Example 4 (refer to FIG. 3A). In this way, the channel formation region 17 and the channel formation region extension portion 18 can be obtained. Depending on the situation, instead of the method for forming the gate insulating layer 15 and the active layer 20 according to "Step-500" and "Step-510", a laminated structure of the gate insulating layer 15 and the active layer 20 can also be obtained by dissolving the insulator material constituting the gate insulating layer 15 and the PXX compound obtained in Working Example 1 constituting the active layer 20 in the above-described solvent, coating the resultant solution on the base 10 and the gate electrode 14, and drying, whereby the gate insulating layer 15 and the active layer 20 are separated utilizing a phase separation phenomenon.

Step-520

Then, the source/drain electrodes 16 are formed on the channel formation region extension portion 18 so as to sandwich the channel formation region 17 (refer to FIG. 3B). Specifically, a gold (Au) layer as the source/drain electrodes 16 is formed based on a vacuum deposition method in the same manner as in"Step-420" of Working Example 4. When performing the deposition, the source/drain electrodes 16 can be formed without using a photolithography process by covering a part of the channel formation region extension portion 18 with a hard mask. It is noted that the source/drain electrodes 16 can also be formed based on a printing method.

Step-530

Next, the electronic device of Working Example 5 is completed by forming a passivation film (not illustrated) on the whole face.

Working Example 6

Working Example 6 is a modification of Working Example 4. In Working Example 6, the three-terminal type electronic device is a top-gate/bottom-contact type FET (specifically, a TFT). As illustrated in the schematic partial end view of FIG. 4B, the field-effect transistor of Working Example 6 includes

(A) source/drain electrodes 16 (corresponding to the first electrode and the second electrode) formed on the base 10,
(B) the channel formation region 17 which is formed on the base 10 between the source/drain electrodes 16 and is configured by the active layer 20,
(C) the gate insulating layer 15 (corresponding to the insulating layer) formed on the source/drain electrodes 16 and the channel formation region 17, and
(D) the gate electrode 14 (corresponding to the control electrode) formed on the gate insulating layer 15.

An outline of the method for manufacturing the electronic device (field-effect transistor) of Working Example 6 will now be described with reference to FIGS. 4A and 4B, which are schematic partial end views of the base and the like.

Step-600

First, the source/drain electrodes 16 are formed on the insulating film 12 corresponding to the base in the same manner as in "Step-420" of Working Example 4, and then the channel formation region 17 (the active layer 20) is formed on the insulating film 12 including the source/drain electrodes 16 in the same manner as in "Step-430" of Working Example 4 (refer to FIG. 4A).

Step-610

Next, the gate insulating layer 15 is formed in the same manner as in "Step-410" of Working Example 4. Then, the gate electrode 14 is formed on the portion of the gate insulating layer 15 on the channel formation region 17 in the same manner as in "Step-400" of Working Example 4 (refer to FIG. 4B).

Step-620

Then, the electronic device of Working Example 6 is completed by forming a passivation film (not illustrated) on the whole face.

Working Example 7

Working Example 7 is a modification of Working Example 4. In Working Example 7, the three-terminal type electronic device is a top-gate/top-contact type FET (specifically, a TFT). As illustrated in the schematic partial end view of FIG. 5C, the field-effect transistor of Working Example 7 includes

(A) the channel formation region 17 and the channel formation region extension portion 18 formed on the base 10 and configured by the active layer 20,
(B) source/drain electrodes 16 (corresponding to the first electrode and the second electrode) formed on the channel formation region extension portion 18,
(C) the gate insulating layer 15 (corresponding to the insulating layer) formed on the source/drain electrodes 16 and the channel formation region 17, and
(D) the gate electrode 14 (corresponding to the control electrode) formed on the gate insulating layer 15.

An outline of the method for manufacturing the electronic device (field-effect transistor) of Working Example 7 will now be described with reference to FIGS. 5A, 5B, and 5C, which are schematic partial end views of the base and the like.

Step-700

First, the channel formation region 17 and the channel formation region extension portion 18 can be obtained by forming the active layer 20 on the base 10 (more specifically, the insulating film 12) in the same manner as in "Step-430" of Working Example 4 (refer to FIG. 5A).

Step-710

Next, the source/drain electrodes 16 are formed on the channel formation region extension portion 18 in the same manner as in "Step-420" of Working Example 4 (refer to FIG. 5B).

Step-720

Then, the gate insulating layer 15 is formed in the same manner as in "Step-410" of Working Example 4. Next, the gate electrode 14 is formed on the portion of the gate insulating layer 15 on the channel formation region 17 in the same manner as in "Step-400" of Working Example 4 (refer to FIG. 5C).

Step-730

Next, the electronic device of Working Example 7 is completed by forming a passivation film (not illustrated) on the whole face.

Working Example 8

Although Working Example 8 is also a modification of Working Example 4, in Working Example 8 the electronic device is specifically a two-terminal type electronic device, and more specifically, as illustrated in the schematic partial end views of FIGS. 6A or 6B, includes
a first electrode 31 and a second electrode 32, and
an active layer 33 formed between the first electrode 31 and the second electrode 32.

It is noted that the active layer 33 includes the PXX compound described in Working Examples 1 to 3. Further, power is generated by the irradiation of light on the active layer 33. Namely, the electronic device of Working Example 8 functions as a photoelectric conversion element or a solar cell. Alternatively, the electronic device of Working Example 8 functions as a light emitting element in which the active layer 33 emits light due to the application of a voltage to the first electrode 31 and the second electrode 32.

Alternatively, the electronic device of Working Example 8 can also function as a chemical substance sensor including a two-terminal type electronic device. Specifically, when a chemical substance to be detected is adsorbed on the active layer 33, the electric resistance value between the first electrode 31 and the second electrode 32 changes. Therefore, the amount (concentration) of the chemical substance adsorbed on the active layer 33 can be measured by flowing a current between the first electrode 31 and the second electrode 32, or alternatively, applying an appropriate voltage between the first electrode 31 and the second electrode 32, and measuring the electric resistance value of the active layer 33. It is noted that since the chemical substance is in an adsorption equilibrium state at the active layer 33, if the amount (concentration) of the chemical substance in the atmosphere in which the active layer 33 is placed changes, the equilibrium state also changes.

Excluding the above points, basically, the composition and structure of the electronic device of Working Example 8 may be essentially the same as the composition and structure of the electronic device described in Working Example 4 or 5, apart from the point that a control electrode and an insulating layer are not provided. Accordingly, a detailed description thereof will be omitted. The electronic device of Working Example 8 can be obtained by executing the same steps as "Step-420" to "Step-430" of Working Example 4, or alternatively, by executing the same steps as "Step-510" to "Step-520" of Working Example 5.

If the electronic device of Working Example 8 that includes the PXX compound described in Working Example 2 or 3 is made to function as a solar cell, although the temperature of the active layer may increase due to the collection of sunlight, the PXX compound according to an embodiment of the present disclosure constituting the active layer does not exhibit crystallinity under a high-temperature atmosphere, namely, changes do not occur in the molecular arrangement, Further, this PXX compound has a high melting point, so that the properties of the solar cell are not susceptible to change even under a high-temperature atmosphere. In addition, even when made to function as a chemical substance sensor, the sensor is hardly affected by the atmosphere temperature.

Working Example 9

Working Example 9 relates to the laminated structure according to the embodiments of the present disclosure, the method for forming a laminated structure according to the first embodiment of the present disclosure, the electronic device according to the fourth embodiment of the present disclosure, and the method for manufacturing an electronic device according to the first embodiment of the present disclosure. FIG. 7E illustrates a schematic partial end view of the laminated structure and the electronic device according to Working Example 9.

The laminated structure of Working Example 9, or, the laminated structure of the below-described Working Examples 10 to 14, is a laminated structure in which are laminated a first layer 101, 201, 301, 401, 501, and 601 formed of a first organic material and a second layer 102, 202, 302, 402, 502, and 602 formed of a second organic material, which is different from the first organic material. Further, the electronic device of Working Example 9, or, the electronic device of the below-described Working Examples 10 to 14, includes an electrode structure, an insulating layer 45, and an active layer 50, in which the electrode structure is configured by a control electrode 44, and first and second electrodes 46. It is noted that the electronic devices of Working Examples 9 to 12 are, specifically, a bottom-gate/top-contact type TFT, and the electronic devices of Working Examples 13 and 14 are a top-gate/bottom-contact type TFT.

Further, in the electronic device of Working Example 9, the insulating layer 45 is configured by a first insulating layer 45A and a second insulating layer 45B from the control electrode side, in which the second insulating layer 45B is formed of the first organic material. In addition, the active layer 50 is formed of the second organic material, which is constituted from an organic semiconductor material. Still further, the combination of the first organic material and the second organic material is configured from a combination of materials so that a value obtained by subtracting a Gibbs free energy $G_1$ of the first organic material and a Gibbs free energy $G_2$ of the second organic material from a Gibbs free energy $G_0$ of a mixed system of the first organic material and the second organic material is positive. Namely, this combination satisfies the following.

$$G_0 > G_1 + G_2$$

It is noted that if,

$$G_0 < G_1 + G_2$$

the mixed system of the first organic material and the second organic material is a more stable energy state than when the first layer and the second layer are separate, so that it is impossible to obtain a state in which the first layer and the second layer are separate. Specifically, in Working Example 9, the first organic material is formed of an insulating material that is a non-curable material (non-crosslinking polymer), specifically, a polyolefine resin, and more specifically, TOPAS®, and the second organic material is formed of an organic semiconductor material that is a non-curable material (non-crosslinking polymer), specifically, a peri-xanthenoxanthene (6,12-dioxaanthanthrene) derivative, and more specifically, ethylphenyl-PXX. Further, xylene is used as the solvent dissolving the first organic material (first solvent), and toluene is used as the solvent dissolving the second organic material (second solvent).

Here, the electronic devices of Working Examples 9 to 12 are, specifically, a bottom-gate/top-contact type semiconductor device having a three-terminal structure, in which
the gate electrode formed on the base 10 is configured by the control electrode 44,
the gate insulating layer (in Working Examples 9 and 11, a second gate insulating layer, and in Working Examples 10 and 12, a gate insulating layer) formed on the gate electrode and the base 10 is configured by the insulating layer 45,
the channel formation region 47 and the channel formation region extension portion 48 formed on the gate electrode layer are configured by the active layer 50, and
the pair of source/drain electrodes formed on the channel formation region extension portion 48 is configured by the first and second electrodes 46.

It is noted that, in the following description, the term "gate electrode 44" may be used instead of "control electrode 44", the terms "channel formation region 47 and/or channel formation region extension portion 48" instead of "active layer 50", the term "source/drain electrodes 46" instead of "first and second electrodes 46", the term "second gate insulating layer 45B" or "gate insulating layer 45" instead of "insulating layer 45", and the term "first gate insulating layer 45A" instead of "first insulating layer 45".

The method for forming the laminated structure and the method for manufacturing the electronic device of Working Example 9 will be described below with reference to FIGS. 7A, 7B, 7C, 7D, and 7E, which are schematic partial end views of the base and the like.

First, the gate electrode 44, and the first gate insulating layer 45A covering the gate electrode 44, are formed on the base 10, which is formed of a glass substrate 11 on which an insulating film 12 including $SiO_2$ is formed on the surface.

Step-900

Specifically, based on a photolithography technique, a resist layer (not illustrated), from which the portion where the gate electrode 44 is to be formed has been removed, is formed on the insulating film 12 including $SiO_2$ that is formed on the surface of the glass substrate 11. Then, a titanium (Ti) layer (not illustrated) as an adhesion layer and a gold (Au) layer as the gate electrode 44 are successively deposited on the whole face by a vacuum deposition method, after which the resist layer is removed. In this way, based on a so-called lift-off method, the gate electrode 44 can be obtained (refer to FIG. 8A). It is noted that the gate electrode 44 can also be formed on the insulating film 12 including $SiO_2$ that is formed on the surface of the glass substrate 11 based on a printing method.

Step-910

Next, a polyvinylphenol (PVP) solution that includes a crosslinking agent is coated on the base 10 and the gate electrode 44 by a slit coater method. Then, the coated PVP solution is heated to 150°C to obtain the first gate insulating layer 45A formed of polyvinylphenol. In this way, the structure illustrated in FIG. 7A can be obtained.

Step-920

Then, a first layer 101 (second gate insulating layer 45B) formed of the first organic material is formed on a support (specifically, the first gate insulating layer 45A). Specifically, the first layer 101 (second gate insulating layer

45B) formed of a polyolefin resin and having a thickness of 20 nm can be formed by depositing a solution of a polyolefin resin in xylene on the first gate insulating layer 45 by a slit coater method, and then drying at 140°C. In this way, the structure illustrated in FIG. 7B can be obtained.

Step-930

Next, a second organic material solution layer 102', in which a second organic material different from the first organic material is dissolved in a solvent, is formed on the first layer 101 (the second gate insulating layer 45B), and then dried to form a second layer 102 (the channel formation region 47 and channel formation region extension portion 48) formed of the second organic material. Specifically, a second organic material solution layer 102' formed of a peri-xanthenoxanthene derivative in toluene is deposited on the first layer 101 (the second gate insulating layer 45B) by a slit coater method, and then dried at 150°C.

Here, when the second organic material solution layer 102' is formed on the first layer 101, the surface of the first layer 101 (the second gate insulating layer 45B) is dissolved by the solvent (specifically, toluene) included in the second organic material solution layer 102'. This causes mixing (refer to FIG. 7C) of the first organic material and the second organic material at the interface between the first layer 101 and the second organic material solution layer 102'. However, at regions away from the interface, there is no mixing of the first organic material and the second organic material, so that when the second organic material solution layer 102' is dried, the first layer 101 (the second gate insulating layer 45B) and the second layer 102 (the channel formation region 47 and channel formation region extension portion 48) separate (refer to FIG. 7D). Namely, when the second organic material solution layer 102' is dried, the first layer 101 (the second gate insulating layer 45B) and the second layer 102 (the channel formation region 47 and channel formation region extension portion 48) spontaneously and naturally separate from each other. Consequently, at the interface between the first layer 101 and the second layer 102, the first organic material and the second organic material do not mix, and the first layer 101 and the second layer 102 are separated. It is noted that the region where the first organic material and the second organic material mix is indicated by reference numeral 103. The rate at which the first layer 101 is dissolved in the solvent when the second organic material solution layer 102' is formed on the first layer 101 is, specifically, about 10 nm/min.

Step-940

Then, source/drain electrodes 46 are formed on the second layer 102 (specifically, the channel formation region extension portion 48). Specifically, a source/drain electrode 46 configured by a 25 nm-thick gold (Au) layer is deposited by a vacuum deposition method (refer to FIG. 7E). When depositing these layers, the source/drain electrodes 46 can be formed without using a photolithography process by covering a part of the second layer 102 with a hard mask. It is noted that the source/drain electrodes 46 can also be formed based on a printing method.

For example, in the manufacture of an image display device, following on from this step, an image display unit (specifically, an image display unit including an organic electroluminescence element, an electrophoretic display element, a semiconductor light emitting element or the like) can also be formed based on a known method on or above the thus-obtained TFT. In each of the below-described working examples too, an image display unit can be obtained by carrying out a similar step after manufacture of the electronic device is completed.

Alternatively, a passivation film (not illustrated) is formed on the whole face. By doing so, a bottom-gate/top-contact type semiconductor device (a FET, specifically, a TFT) can be obtained. Alternatively, a passivation film (not illustrated) may be formed on the whole face after patterning the channel formation region extension portion 48 and the second gate insulating layer 45B. This enables the adhesive properties of the active layer 50 and the second gate insulating layer 45B to be improved.

In Working Example 9, when the second organic material solution layer 102' is formed on the first layer 101, the surface of the first layer 101 is dissolved by the solvent included in the second organic material solution layer 102'. This causes mixing of the first organic material and the second organic material at the interface between the first layer 101 and the second organic material solution layer 102'. However, at regions away from the interface, there is no mixing of the first organic material and the second organic material, so that when the second organic material solution layer 102' is dried, the first layer 101 and the second layer 102 separate. Consequently, a high level of smoothness could be obtained at the interface between the first organic material layer (the first layer 101) and the second organic material layer (the second layer 102), and a high level of precision in film thickness and reliable separation could be obtained for these layers. Further, the first organic material layer (the first layer 101) did not become contaminated before the second organic material layer (the second layer 102) was formed. In addition, as a result, an electronic device having little unevenness in its properties and excellent performance could be manufactured. Still further, for the laminated structure or the electronic device of Working Example 9, since a relationship is defined among the value for the Gibbs free energy of the mixed system of the first organic material layer and the second organic material layer, the value for the Gibbs free

energy of the first organic material, and the value for the Gibbs free energy of the second organic material, a separated state can be obtained reliably, spontaneously, and naturally when forming these layers. Moreover, the interface between these layers has a high level of smoothness, and a high level of precision in film thickness could be obtained for these layers.

Working Example 10

Working Example 10 relates to the laminated structure according to the embodiments of the present disclosure, the method for forming a laminated structure according to the first embodiment of the present disclosure, the electronic device according to the fourth embodiment of the present disclosure, and the method for manufacturing an electronic device according to the second embodiment of the present disclosure. FIG. 8E illustrates a schematic partial end view of the laminated structure and the electronic device according to Working Example 10. In Working Example 10, unlike Working Example 9, the gate insulating layer is a monolayer structure. Further, the gate electrode is formed in a groove portion provided in the base. Apart from these points, since the composition and structure of the electronic device of Working Example 10 are the same as the composition and structure of the electronic device of Working Example 9, a detailed description thereof will be omitted. It is noted that the first organic material and the second organic material in Working Example 10 are the same as in Working Example 9.

The method for forming the laminated structure and the method for manufacturing the electronic device of Working Example 10 will be described below with reference to FIGS. 8A, 8B, 8C, 8D, and 8E, which are schematic partial end views of the base and the like.

Step-1000

First, the gate electrode 44 is formed in a groove portion 43 formed in the base 10. Specifically, based on a known photolithography technique and etching technique, the groove portion 43 is formed on a region of the base 10, which is formed of the glass substrate 11 on which the insulating film 12 including $SiO_2$ is formed on the surface, where the gate electrode is to be formed. Next, based on a photolithography technique, a resist layer (not illustrated) from which the portion where the gate electrode 44 is to be formed has been removed is formed. Then, a titanium (Ti) layer (not illustrated) as an adhesion layer and a gold (Au) layer as the gate electrode 44 are successively deposited on the whole face by a vacuum deposition method, after which the resist layer is removed. In this way, based on a so-called lift-off method, the gate electrode 44 can be obtained (refer to FIG. 8A). It is noted that the gate electrode 44 can also be formed in the groove portion 43 based on a printing method.

Step-1010

Then, a first layer 201 (the gate insulating layer 45) formed of a first organic material is formed on a support (specifically, the base 10 and the gate electrode 44) in the same manner as in "Step-920" of Working Example 9. In this way, the structure illustrated in FIG. 8B can be obtained.

Step-1020

Next, a second organic material solution layer 202’, in which a second organic material different from the first organic material is dissolved in a solvent, is formed on the first layer 201 (the gate insulating layer 45) in the same manner as in "Step-930" of Working Example 9, and then dried to form a second layer 202 (the channel formation region 47 and channel formation region extension portion 48) formed of the second organic material.

Here, similar to Working Example 9, when the second organic material solution layer 202’ is formed on the first layer 201, the surface of the first layer 201 (the gate insulating layer 45) is dissolved by the solvent (specifically, toluene) included in the second organic material solution layer 202’. This causes mixing (refer to FIG. 8C) of the first organic material and the second organic material at the interface between the first layer 201 and the second organic material solution layer 202’. However, at regions away from the interface, there is no mixing of the first organic material and the second organic material, so that when the second organic material solution layer 202’ is dried, the first layer 201 (the gate insulating layer 45) and the second layer 202 (the channel formation region 47 and channel formation region extension portion 48) separate (refer to FIG. 8D). Namely, when the second organic material solution layer 202’ is dried, the first layer 201 (the gate insulating layer 45) and the second layer 202 (the channel formation region 47 and channel formation region extension portion 48) spontaneously and naturally separate from each other. It is noted that the region where the first organic material and the second organic material mix is indicated by reference numeral 203.

Step-1030

Then, a bottom-gate/top-contact type semiconductor device (a FET, specifically, a TFT) can be obtained (refer to FIG. 8E) by performing the same step as in "Step-940" of Working Example 9.

In Working Example 10, when the second organic material solution layer 202' is formed on the first layer 201, the surface of the first layer 201 is dissolved by the solvent included in the second organic material solution layer 202'. This causes mixing of the first organic material and the second organic material at the interface between the first layer 201 and the second organic material solution layer 202'. However, at regions away from the interface, there is no mixing of the first organic material and the second organic material, so that when the second organic material solution layer 202' is dried, the first layer 201 and the second layer 202 separate. Consequently, a high level of smoothness could be obtained at the interface between the first organic material layer (the first layer 201) and the second organic material layer (the second layer 202), and a high level of precision in film thickness and reliable separation could be obtained for these layers. Further, the first organic material layer (the first layer 201) did not become contaminated before the second organic material layer (the second layer 202) was formed. In addition, as a result, an electronic device having little unevenness in its properties and excellent performance could be manufactured. Still further, for the laminated structure or the electronic device of Working Example 10, since a relationship is defined among the value for the Gibbs free energy of the mixed system of the first organic material layer and the second organic material layer, the value for the Gibbs free energy of the first organic material, and the value for the Gibbs free energy of the second organic material, a separated state can be obtained reliably, spontaneously, and naturally when forming these layers. Moreover, the interface between these layers has a high level of smoothness, and a high level of precision in film thickness could be obtained for these layers.

Working Example 11

Working Example 11 relates to the laminated structure according to the embodiments of the present disclosure, the method for forming a laminated structure according to the second embodiment of the present disclosure, the electronic device according to the fourth embodiment of the present disclosure, and the method for manufacturing an electronic device according to the third embodiment of the present disclosure. FIG. 9D illustrates a schematic partial end view of the laminated structure and the electronic device according to Working Example 11. In Working Example 11, unlike Working Example 9, the gate insulating layer is a monolayer structure. Apart from this point, since the structure of the electronic device of Working Example 11 is the same as the structure of the electronic device of Working Example 9, a detailed description thereof will be omitted.

Here, specifically, in Working Example 11, the first organic material is formed of an insulating material, specifically, $\alpha$-methylstyrene, which is a non-curable material (non-crosslinking polymer). The second organic material is formed of an organic semiconductor material that is a non-curable material (non-crosslinking polymer), specifically, TIPS-pentacene. Xylene is used as the first solvent for dissolving the first organic material, and toluene is used as the second solvent for dissolving the second organic material.

The method for forming the laminated structure and the method for manufacturing the electronic device of Working Example 11 will be described below with reference to FIGS. 9A, 9B, 9C, and 9D, which are schematic partial end views of the base and the like.

Step-1100

First, the gate electrode 44 and the first gate insulating layer 45A covering the gate electrode 44 are formed on the base 10 in the same manner as in "Step-900" and "Step-910" of Working Example 9 (refer to FIG. 9A).

Step-1110

Next, using a double-nozzle slit coater, a first organic material solution layer 301', in which a first organic material formed of an insulating material is dissolved in a first solvent, and a second organic material solution layer 302', in which a second organic material that is different from the first organic material and that is formed of an organic semiconductor material is dissolved in a second solvent, are formed on a support (specifically, the first gate insulating layer 45A), and then the first organic material solution layer 301' and the second organic material solution layer 302' are dried to obtain a laminated structure of a first layer 301 formed of the first organic material and a second layer 302 formed of the second organic material. It is noted that the second gate insulating layer 45B is configured by the first layer 301, and the channel formation region 47 and the channel formation region extension portion 48 are configured by the second layer 302.

In Working Example 11, when the first organic material solution layer 301' and the second organic material solution layer 302' are formed, the first organic material and the second organic material mix at the interface between

the first organic material solution layer 301' and the second organic material solution layer 302' (refer to FIG. 9B). However, at regions away from the interface, there is no mixing of the first organic material and the second organic material, so that when the first organic material solution layer 301' and the second organic material solution layer 302' are dried, the first layer 301 (the second gate insulating layer 45B) and the second layer 302 (the channel formation region 47 and channel formation region extension portion 48) separate (refer to FIG. 9C). Namely, when the first organic material solution layer 301' and the second organic material solution layer 302' are dried, the first layer 301 (the second gate insulating layer 45B) and the second layer 302 (the channel formation region 47 and channel formation region extension portion 48) spontaneously and naturally separate from each other. It is noted that the region where the first organic material and the second organic material mix is indicated by reference numeral 303.

Step-1120

Then, a bottom-gate/top-contact type semiconductor device (a FET, specifically, a TFT) can be obtained (refer to FIG. 9D) by performing the same step as in "Step-940" of Working Example 9.

In Working Example 11, when the first organic material solution layer 301' and the second organic material solution layer 302' are formed, it causes mixing of the first organic material and the second organic material solution layer 302' at the interface between the first organic material solution layer 301' and the second organic material solution layer 302'. However, at regions away from the interface, there is no mixing of the first organic material and the second organic material, so that when the first organic material solution layer 301' and the second organic material solution layer 302' are dried, the first layer 301 and the second layer 302 separate. Consequently, a high level of smoothness could be obtained at the interface between the first organic material layer (the first layer 301) and the second organic material layer (the second layer 302), and a high level of precision in film thickness and reliable separation could be obtained for these layers. Further, the first organic material layer (the first layer 301) did not become contaminated before the second organic material layer (the second layer 302) was formed. In addition, as a result, an electronic device having little unevenness in its properties and excellent performance could be manufactured. Still further, for the laminated structure or the electronic device of Working Example 11, since a relationship is defined among the value for the Gibbs free energy of the mixed system of the first organic material layer and the second organic material layer, the value for the Gibbs free energy of the first organic material, and the value for the Gibbs free energy of the second organic material, a separated state can be obtained reliably, spontaneously, and naturally when forming these layers. Moreover, the interface between these layers has a high level of smoothness, and a high level of precision in film thickness could be obtained for these layers.

Working Example 12

Working Example 12 relates to the laminated structure according to the embodiments of the present disclosure, the method for forming a laminated structure according to the second embodiment of the present disclosure, the electronic device according to the fourth embodiment of the present disclosure, and the method for manufacturing an electronic device according to the fourth embodiment of the present disclosure. FIG. 10D illustrates a schematic partial end view of the laminated structure and the electronic device according to Working Example 12. In Working Example 12, unlike Working Example 11, the gate insulating layer is a monolayer structure. Further, the gate electrode is formed in a groove provided in the base. Apart from these points, since the composition and structure of the electronic device of Working Example 12 are the same as the composition and structure of the electronic device of Working Example 11, a detailed description thereof will be omitted. It is noted that the first organic material and the second organic material in Working Example 11 are the same as in Working Example 11.

The method for forming the laminated structure and the method for manufacturing the electronic device of Working Example 12 will be described below with reference to FIGS. 10A, 10B, 10C, and 10D, which are schematic partial end views of the base and the like.

Step-1200

First, the gate electrode 44 is formed in the groove portion 43 in the base 10 in the same manner as in "Step-1000" of Working Example 10 (refer to FIG. 10A).

Step-1210

Next, using a double-nozzle slit coater, a first organic material solution layer 401', in which a first organic material formed of an insulating material is dissolved in a first solvent, and a second organic material solution layer 402', in which a second organic material that is different from the first organic material and that is formed of an organic semiconductor

material is dissolved in a second solvent, are formed on a support (specifically, the base 10 and the gate electrode 44) in the same manner as in "Step 1110" of Working Example 11, and then the first organic material solution layer 401' and the second organic material solution layer 402' are dried to obtain a laminated structure of a first layer 401 formed of the first organic material and a second layer 402 formed of the second organic material. It is noted that, similar to Working Example 11, the gate insulating layer 45 is configured by the first layer 401, and the channel formation region 47 and the channel formation region extension portion 48 are configured by the second layer 402.

In Working Example 12 too, when the first organic material solution layer 401' and the second organic material solution layer 402' are formed, the first organic material and the second organic material mix at the interface between the first organic material solution layer 401' and the second organic material solution layer 402' (refer to FIG. 10B). However, at regions away from the interface, there is no mixing of the first organic material and the second organic material, so that when the first organic material solution layer 401' and the second organic material solution layer 402' are dried, the first layer 401 (the gate insulating layer 45) and the second layer 402 (the channel formation region 47 and channel formation region extension portion 48) separate (refer to FIG. 10C). Namely, when the first organic material solution layer 301' and the second organic material solution layer 302' are dried, the first layer 301 (the second gate insulating layer 45B) and the second layer 302 (the channel formation region 47 and channel formation region extension portion 48) spontaneously and naturally separate from each other. It is noted that the region where the first organic material and the second organic material mix is indicated by reference numeral 403.

Step-1220

Then, a bottom-gate/top-contact type semiconductor device (a FET, specifically, a TFT) can be obtained (refer to FIG. 10D) by performing the same step as in "Step-940" of Working Example 9.

In Working Example 12, when the first organic material solution layer 401' and the second organic material solution layer 402' are formed, it causes mixing of the first organic material and the second organic material solution layer 402' at the interface between the first organic material solution layer 401' and the second organic material solution layer 402'. However, at regions away from the interface, there is no mixing of the first organic material and the second organic material, so that when the first organic material solution layer 401' and the second organic material solution layer 402' are dried, the first layer 401 and the second layer 402 separate. Consequently, a high level of smoothness could be obtained at the interface between the first organic material layer (the first layer 401) and the second organic material layer (the second layer 402), and a high level of precision in film thickness and reliable separation could be obtained for these layers. Further, the first organic material layer (the first layer 401) did not become contaminated before the second organic material layer (the second layer 402) was formed. In addition, as a result, an electronic device having little unevenness in its properties and excellent performance could be manufactured. Still further, for the laminated structure or the electronic device of Working Example 12, since a relationship is defined among the value for the Gibbs free energy of the mixed system of the first organic material layer and the second organic material layer, the value for the Gibbs free energy of the first organic material, and the value for the Gibbs free energy of the second organic material, a separated state can be obtained reliably, spontaneously, and naturally when forming these layers. Moreover, the interface between these layers has a high level of smoothness, and a high level of precision in film thickness could be obtained for these layers.

Working Example 13

Working Example 13 relates to the laminated structure according to the embodiments of the present disclosure, the method for forming a laminated structure according to the first embodiment of the present disclosure, the electronic device according to the fourth embodiment of the present disclosure, and the method for manufacturing an electronic device according to the fifth embodiment of the present disclosure. FIG. 11E illustrates a schematic partial end view of the laminated structure and the electronic device according to Working Example 13.

For the electronic device of Working Example 13 or the below-described Working Example 14, the channel formation region 47 and the channel formation region extension portion 48 are configured by a first layer 501, which is formed of a first organic material formed of an organic semiconductor material. On the other hand, the gate insulating layer 45 is configured by a second layer 502, which is formed of a second organic material formed of an insulating material. In Working Example 13, the same organic semiconductor material, insulating material, and solvents as the organic semiconductor material, the insulating material, and solvents described in Working Example 9 are used.

Namely, the electronic device of Working Example 13 or the below-described Working Example 14 is, specifically, a top-gate/bottom-contact type semiconductor device having a three-terminal structure, in which

a pair of source/drain electrodes formed in the base 10 are configured by first electrode and second electrodes 46,

the channel formation region 47 formed on the base 10 between the pair of source/drain electrodes and the channel formation region extension portion 48 formed on the source/drain electrodes are configured by the active layer 50,

the gate insulating layer formed on the channel formation region 47 and the channel formation region extension portion 48 is configured by the insulating layer 45, and
the gate electrode formed on the gate insulating layer 45 disposed facing the channel formation region 47 is configured by the control electrode 44.

The method for forming the laminated structure and the method for manufacturing the electronic device of Working Example 13 will be described below with reference to FIGS. 11A, 11B, 11C, 11D, and 11E, which are schematic partial end views of the base and the like.

Step-1300

First, the source/drain electrodes 46 are formed in the groove portion 43 formed in the base 10 in essentially the same manner as in "Step-1000" of Working Example 10 (refer to FIG. 11A).

Step-1310

Next, a first layer 501 (the channel formation region 47 and channel formation region extension portion 48) formed of a first organic material formed of an organic semiconductor material is formed on a support (specifically, the base 10 and the source/drain electrodes 46). More specifically, the first layer 501 (the channel formation region 47 and channel formation region extension portion 48) can be obtained by depositing a first organic material solution layer formed of a peri-xanthenoxanthene derivative in toluene on the base 10 and the source/drain electrodes 46 by a slit coater method, and then drying at 150°C (FIG. 11B).

Step-1320

Then, a second organic material solution layer 502', in which a second organic material that is different from the first organic material and that is formed of an insulating material is dissolved in a solvent, is formed on the first layer 501 (the channel formation region 47 and channel formation region extension portion 48) (refer to FIG. 11C), and dried to form a second layer 502 (the gate insulating layer 45) formed of the second organic material. Specifically, the second layer 502 (the gate insulating layer 45) formed of a polyolefin resin and having a thickness of 20 nm can be formed by, in the same manner as in "Step-920" of Working Example 9, depositing a solution of a polyolefin resin in xylene on the first layer 501 by a slit coater method, and then drying. In this way, the structure illustrated in FIG. 11D can be obtained. The rate at which the first layer 501 is dissolved in the solvent when the second organic material solution layer 502' is formed on the first layer 501 is, specifically, about 10 nm/min.

Here, when the second organic material solution layer 502' is formed on the first layer 501, the surface of the first layer 501 (the channel formation region 47 and channel formation region extension portion 48) is dissolved by the solvent (specifically, xylene) included in the second organic material solution layer 502'. This causes mixing (refer to FIG. 11C) of the first organic material and the second organic material at the interface between the first layer 501 and the second organic material solution layer 502'. However, at regions away from the interface, there is no mixing of the first organic material and the second organic material, so that when the second organic material solution layer 502' is dried, the first layer 501 (the channel formation region 47 and channel formation region extension portion 48) and the second layer 502 (the gate insulating layer 45) separate (refer to FIG. 11D). Namely, when the second organic material solution layer 502' is dried, the first layer 501 (the channel formation region 47 and channel formation region extension portion 48) and the second layer 502 (the gate insulating layer 45) spontaneously and naturally separate from each other. It is noted that the region where the first organic material and the second organic material mix is indicated by reference numeral 503.

Step-1330

Then, a contact-gate/bottom-contact type semiconductor device (a FET, specifically, a TFT) can be obtained (refer to FIG. 11E) by performing the same step as in "Step-940" of Working Example 9.

In Working Example 13, when the second organic material solution layer 102' is formed on the first layer 501, the surface of the first layer 501 is dissolved by the solvent included in the second organic material solution layer 502'. This causes mixing of the first organic material and the second organic material at the interface between the first layer 501 and the second organic material solution layer 502'. However, at regions away from the interface, there is no mixing of the first organic material and the second organic material, so that when the second organic material solution layer 502' is dried, the first layer 501 and the second layer 502 separate. Consequently, a high level of smoothness could be obtained at the interface between the first layer 501 and the second organic material layer (the second layer 502), and a high level of precision in film thickness and reliable separation could be obtained for these layers. Further, the first

layer 501 did not become contaminated before the second organic material layer (the second layer 502) was formed. In addition, as a result, an electronic device having little unevenness in its properties and excellent performance could be manufactured. Still further, for the laminated structure or the electronic device of Working Example 13, since a relationship is defined among the value for the Gibbs free energy of the mixed system of the first organic material layer and the second organic material layer, the value for the Gibbs free energy of the first organic material, and the value for the Gibbs free energy of the second organic material, a separated state can be obtained reliably, spontaneously, and naturally when forming these layers. Moreover, the interface between these layers has a high level of smoothness, and a high level of precision in film thickness could be obtained for these layers.

Working Example 14

Working Example 14 relates to the laminated structure according to the embodiments of the present disclosure, the method for forming a laminated structure according to the second embodiment of the present disclosure, the electronic device according to the fourth embodiment of the present disclosure, and the method for manufacturing an electronic device according to the sixth embodiment of the present disclosure. FIG. 11D illustrates a schematic partial end view of the laminated structure and the electronic device according to Working Example 14. Since the composition and structure of the electronic device of Working Example 14 are the same as the composition and structure of the electronic device of Working Example 13, a detailed description thereof will be omitted. It is noted that the first organic material, the second organic material, and the first and second solvents in Working Example 14 are the same as the first organic material, the second organic material, and the first and second solvents in Working Example 11.

The method for forming the laminated structure and the method for manufacturing the electronic device of Working Example 14 will be described below with reference to FIGS. 12A, 12B, 12C, and 12D, which are schematic partial end views of the base and the like.

Step-1400

First, the source/drain electrodes 46 are formed in the groove portion 43 formed in the base 10 in the same manner as in "Step-1300" of Working Example 13 (refer to FIG. 12A).

Step-1410

Next, using a double-nozzle slit coater, a first organic material solution layer 601', in which a first organic material formed of an organic semiconductor material is dissolved in a first solvent, and a second organic material solution layer 602', in which a second organic material that is different from the first organic material and that is formed of an insulating material is dissolved in a second solvent, are formed on a support (specifically, the base 10 and the source/drain electrodes 46), and then the first organic material solution layer 601' and the second organic material solution layer 602' are dried to obtain a laminated structure of a first layer 601 including the first organic material and a second layer 602 including the second organic material. It is noted that the channel formation region 47 and the channel formation region extension portion 48 are configured by the first layer 601, and the gate insulating layer 45 is configured by the second layer 602.

In Working Example 14, when the first organic material solution layer 601' and the second organic material solution layer 602' are formed, the first organic material and the second organic material mix at the interface between the first organic material solution layer 601' and the second organic material solution layer 602' (refer to FIG. 12B). However, at regions away from the interface, there is no mixing of the first organic material and the second organic material, so that when the first organic material solution layer 601' and the second organic material solution layer 602' are dried, the first layer 601 (the channel formation region 47 and channel formation region extension portion 48) and the second layer 602 (the gate insulating layer 45) separate (refer to FIG. 12C). Namely, when the first organic material solution layer 601' and the second organic material solution layer 602' are dried, the first layer 601 (the channel formation region 47 and channel formation region extension portion 48) and the second layer 602 (the gate insulating layer 45) spontaneously and naturally separate from each other. It is noted that the region where the first organic material and the second organic material mix is indicated by reference numeral 603.

Step-1420

Then, a top-gate/bottom-contact type semiconductor device (a FET, specifically, a TFT) can be obtained (refer to FIG. 12D) by performing the same step as in "Step-940" of Working Example 9.

In Working Example 14, when the first organic material solution layer 601' and the second organic material solution layer 602' are formed, it causes mixing of the first organic material and the second organic material solution

layer 602' at the interface between the first organic material solution layer 601' and the second organic material solution layer 602'. However, at regions away from the interface, there is no mixing of the first organic material and the second organic material, so that when the first organic material solution layer 601' and the second organic material solution layer 602' are dried, the first layer 601 and the second layer 602 separate. Consequently, a high level of smoothness could be obtained at the interface between the first organic material layer (the first layer 601) and the second organic material layer (the second layer 602), and a high level of precision in film thickness and reliable separation could be obtained for these layers. Further, the first organic material layer (the first layer 601) did not become contaminated before the second organic material layer (the second layer 602) was formed. In addition, as a result, an electronic device having little unevenness in its properties and excellent performance could be manufactured. Still further, for the laminated structure or the electronic device of Working Example 14, since a relationship is defined among the value for the Gibbs free energy of the mixed system of the first organic material layer and the second organic material layer, the value for the Gibbs free energy of the first organic material, and the value for the Gibbs free energy of the second organic material, a separated state can be obtained reliably, spontaneously, and naturally when forming these layers. Moreover, the interface between these layers has a high level of smoothness, and a high level of precision in film thickness could be obtained for these layers.

Working Example 15

In Working Examples 9 to 14, the electronic device was a three-terminal type electronic device. However, in Working Example 15, the electronic device is a two-terminal type electronic device. An example of an electronic device having a two-terminal structure includes a photoelectric conversion element in which current flows between the first electrode and the second electrode by irradiation of light on the active layer. If a photoelectric conversion element is configured by an electronic device, specifically, a solar cell or an image sensor can be configured by the photoelectric conversion element. It is noted that the photoelectric conversion element can also be configured from an electronic device having a three-terminal structure. In this case, a voltage may or may not be applied to the control electrode. If a voltage is applied, the current that is flowing can be modulated based on the application of the voltage to the control electrode.

Specifically, as illustrated in the schematic partial end views of FIGS. 13A and 13B, the two-terminal type electronic device of Working Example 15 includes

a first electrode 61 and a second electrode 62, and

an active layer 60 formed between the first electrode 61 and the second electrode 62.

It is noted that the active layer 60 includes a second organic material formed of an organic semiconductor material. A charge injection layer 63, which is formed of a first organic material formed of an insulating material, is formed between the base 10 and the active layer 60 (refer to FIG. 13A). Alternatively, the active layer 60 is formed of a first organic material formed of an organic semiconductor material, and the charge injection layer 63, which is formed of a second organic material formed of an insulating material, is formed on the active layer 60 (refer to FIG. 13B). In addition, power is generated by the irradiation of light on the active layer 60. Namely, the electronic device of Working Example 15 functions as a photoelectric conversion element or a solar cell. Alternatively, the electronic device of Working Example 15 functions as a light emitting element in which the active layer 60 emits light due to the application of a voltage to the first electrode 61 and the second electrode 62. When configuring a solar cell, for example, from the two-terminal type electronic device of Working Example 15, examples of the active layer 60 may include P3HT, and examples of the charge injection layer 63 may include PEDOT/PSS.

Excluding the above points, basically, the composition and structure of the electronic device of Working Example 15 may be essentially the same as the composition and structure of the electronic device described in Working Examples 9 to 14 apart from the point that a control electrode is not provided and the point that the electrode structure includes a first electrode and a second electrode. Accordingly, a detailed description thereof will be omitted. Specifically, the electronic device of Working Example 15 can be obtained by executing the same steps as "Step-920" to "Step-940" of Working Example 9, or by executing the same steps as "Step-1010" to "Step-1030" of Working Example 10, or by executing the same steps as "Step-1110" to "Step-1120" of Working Example 11, or by executing the same steps as "Step-1210" to "Step-1220" of Working Example 12, or by executing the same steps as "Step-1300" to "Step-1320" of Working Example 13, or by executing the same steps as "Step-1400" to "Step-1410" of Working Example 14.

In the above, although embodiments of the present disclosure were described based on preferred working examples, the present disclosure is not limited to these working examples. The specific structure of the dioxaanthanthrene compound is not especially limited. Further, the compositions, structures, formation conditions, and manufacturing conditions of the structure of the electronic device and the laminated structure, the method for forming a laminated structure, and the method for manufacturing an electronic device are examples that can be appropriately changed. The electronic device according to the first to fourth embodiments of the present disclosure can be, for example, when applied or used in various image display devices or various electronic devices, used as a monolithic integrated circuit in which multiple

electronic devices have been integrated on a base, a support, or a support member, or each electronic device may be individually separated and used as a discrete component.

In the dioxaanthanthrene compound represented in structural formula (2) to structural formula (9), for example, even for a dioxaanthanthrene compound in which "X" is oxygen (O), "Y" is sulfur (S), "$A_1$" and "$A_2$" are a hydrogen (H) atom, and "R" is a para-isobutylphenyl group, similar to the dioxaanthanthrene compound of Working Example 1, the dioxaanthanthrene compounds are stable in air, and can be easily isolated. Further, by manufacturing an electronic device from such dioxaanthanthrene compounds, the same electronic device as in Working Examples 4 to 8 can be manufactured.

Additionally, the present technology may also be configured as below.

(1) «Dioxaanthanthrene compound: first embodiment»

A dioxaanthanthrene compound represented by any one of structural formulae selected from the group consisting of the following structural formula (1) to structural formula (9),

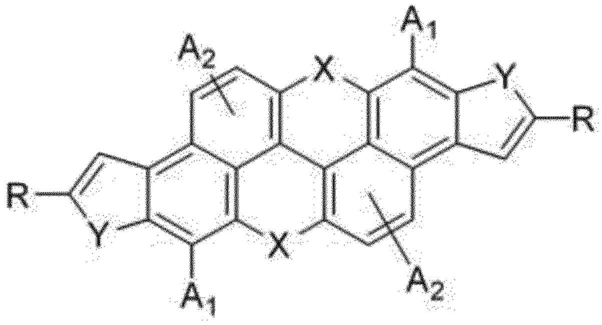

(1)

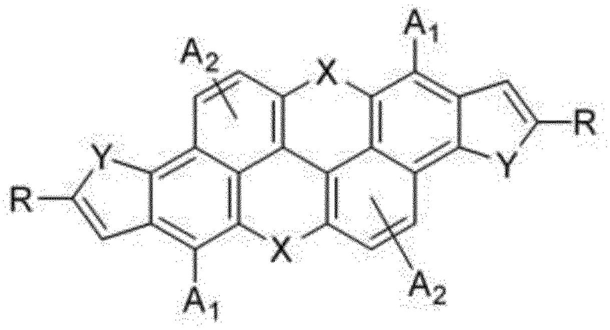

(2)

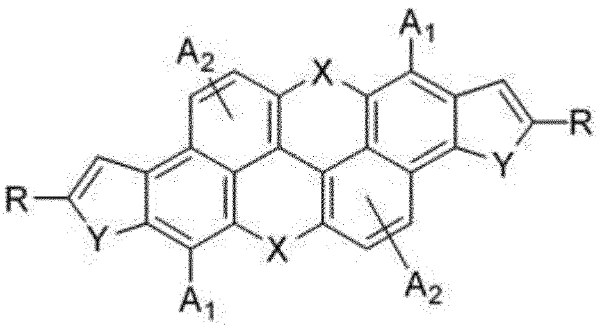

(3)

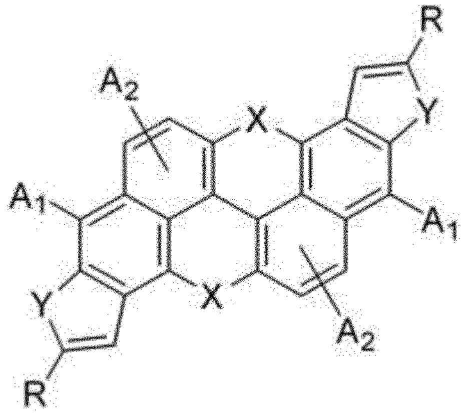

(4)

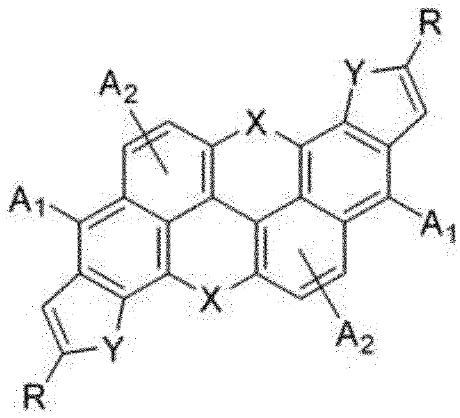

(5)

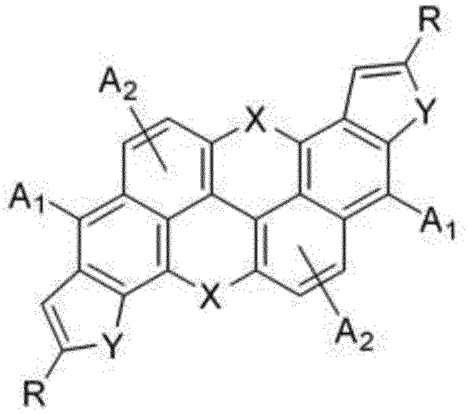

(6)

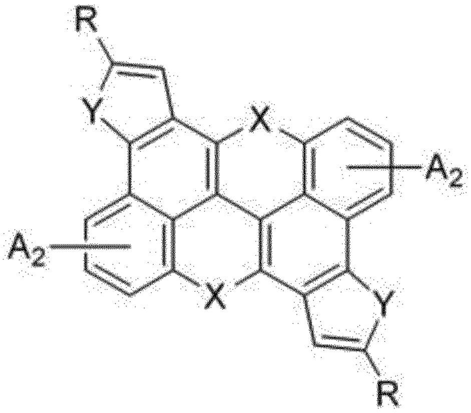

(7)

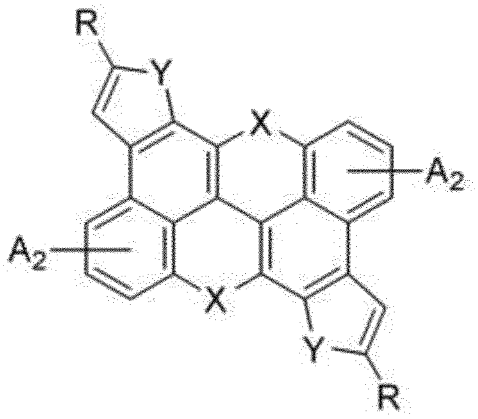

(8)

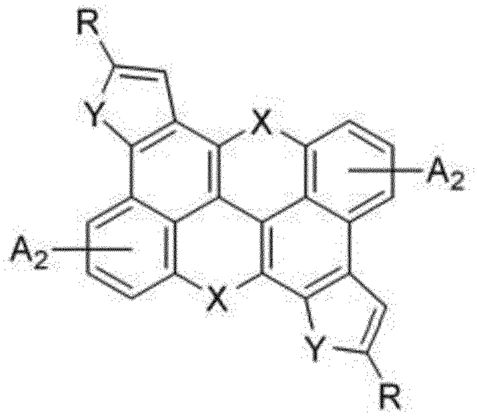

(9)

wherein X represents one atom selected from the group consisting of oxygen, sulfur, selenium and tellurium, wherein Y represents one atom selected from the group consisting of oxygen, sulfur, selenium and tellurium, and wherein R, $A_1$, and $A_2$ each represent a hydrogen atom or a substituent selected from the group consisting of an alkyl group, a cycloalkyl group, an alkenyl group, an alkynyl group, an aryl group, an arylalkyl group, an aromatic heterocycle, a heterocyclic group, an alkoxy group, a cycloalkoxy group, an aryloxy group, an alkylthio group, a cycloalkylthio group, an arylthio group, an alkoxycarbonyl group, an aryloxycarbonyl group, a sulfamoyl group, an acyl group, an acyloxy group, an amide group, a carbamoyl group, a ureido group, a sulfinyl group, an alkylsulfonyl group, an arylsulfonyl group, an amino group, a halogen atom, a fluorinated hydrocarbon group, a cyano group, a nitro group, a hydroxy group, a mercapto group, and a silyl group.

(2) The dioxaanthanthrene compound according to (1), wherein R, $A_1$, and $A_2$ each represent a hydrogen atom or a substituent selected from the group consisting of an alkyl group, an alkenyl group, an aryl group, an arylalkyl group, an aromatic heterocycle, and a halogen atom.

(3) The dioxaanthanthrene compound according to (1) or (2), wherein X represents oxygen.

(4) The dioxaanthanthrene compound according to any one of (1) to (3), wherein Y represents sulfur.
(5) The dioxaanthanthrene compound according to any one of (1) to (4), wherein $A_1$ and $A_2$ are a hydrogen atom.
(6) «Electronic device: first embodiment»
An electronic device including at least:

a first electrode;
a second electrode disposed separated from the first electrode; and
an active layer formed of an organic semiconductor material provided from the first electrode to the second electrode,
wherein the organic semiconductor material is formed of the dioxaanthanthrene compound according to any one of (1) to (5).

(7) «Dioxaanthanthrene compound: second embodiment»
A dioxaanthanthrene compound represented by the following structural formula (11),

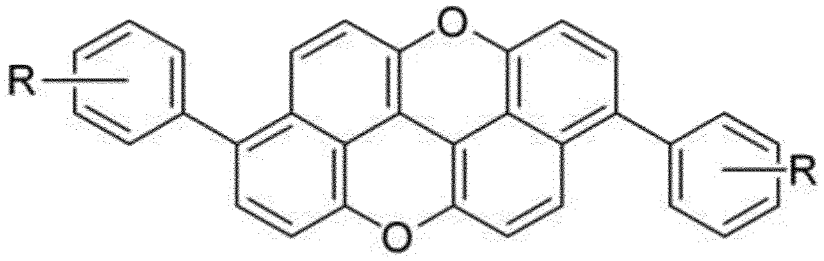

(11)

wherein R represents an alkyl group having a branch with four or more carbon atoms.
(8) «Electronic device: second embodiment»
An electronic device including at least:

a first electrode;
a second electrode disposed separated from the first electrode; and
an active layer formed of an organic semiconductor material provided from the first electrode to the second electrode,
wherein the organic semiconductor material includes the dioxaanthanthrene compound represented by the following structural formula (11), and

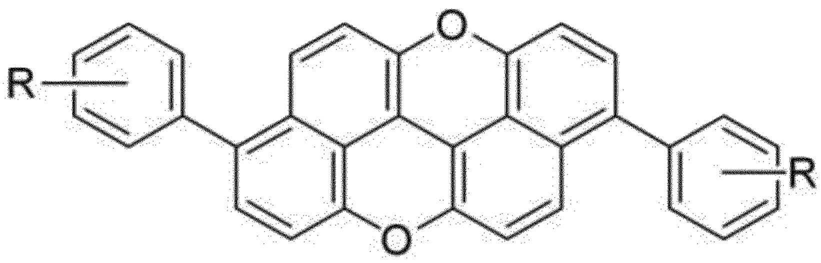

(11)

wherein R represents an alkyl group having a branch with four or more carbon atoms.

(9) The electronic device according to (8), wherein the active layer is formed by coating a dioxaanthanthrene compound on a base and drying.
(10) The electronic device according to (8) or (9), which is a display element, a display device, a solar cell, or a sensor.
(11) «Dioxaanthanthrene compound: third embodiment»
A dioxaanthanthrene compound represented by the following structural formula (21-1), or structural formula (21-2), or structural formula (21-3),

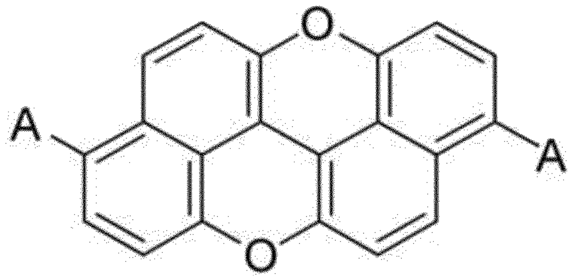

(21−1)

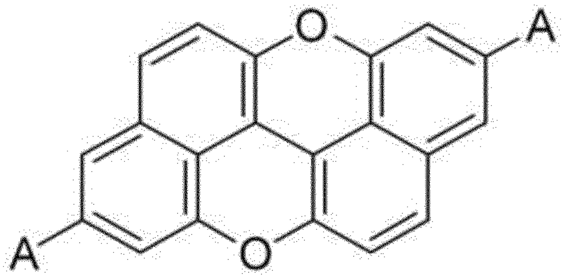

(21−2)

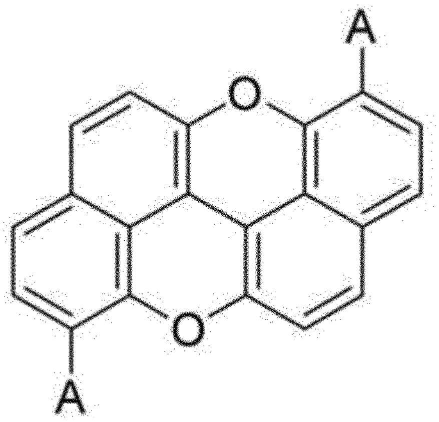

(21−3)

wherein substituent A is represented by the following structural formula (22-1) or structural formula (22-2), and wherein $X_1$, $X_2$, $X_3$, $Y_1$, $Y_2$, $Y_3$, $Y_4$, $Y_5$, $Y_6$, $Y_7$, and $Y_8$ each represent a hydrogen atom or a substituent selected from the group consisting of an alkyl group, a cycloalkyl group, an alkenyl group, an alkynyl group, an aryl group, an arylalkyl group, an aromatic heterocycle, a heterocyclic group, an alkoxy group, a cycloalkoxy group, an aryloxy group, an alkylthio group, a cycloalkylthio group, an arylthio group, an alkoxycarbonyl group, an aryloxycarbonyl group, a sulfamoyl group, an acyl group, an acyloxy group, an amide group, a carbamoyl group, a ureido group, a sulfinyl group, an alkylsulfonyl group, an arylsulfonyl group, an amino group, a halogen atom, a fluorinated hydrocarbon group, a cyano group, a nitro group, a hydroxy group, a mercapto group, and a silyl group.

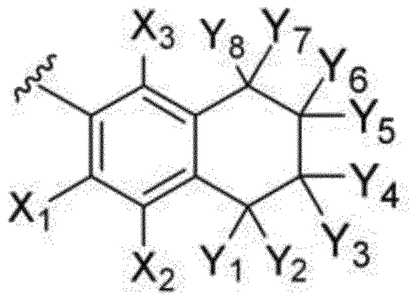

(22−1)

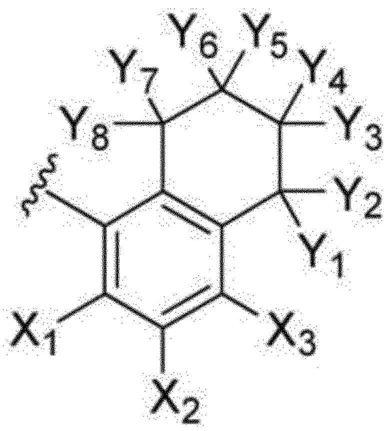

(22−2)

(12) «Dioxaanthanthrene compound: fourth embodiment»
A dioxaanthanthrene compound represented by the following structural formula (23-1) or structural formula (23-2).

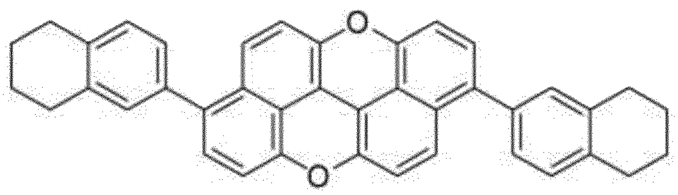

(23−1)

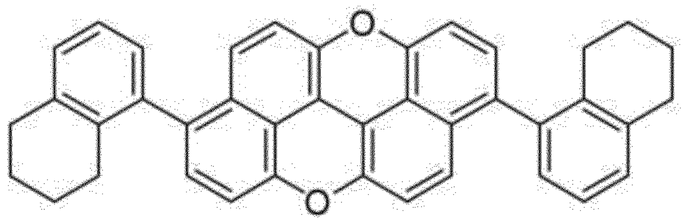

(23−2)

(13) «Electronic device: third embodiment»
An electronic device including at least:

a first electrode;
a second electrode disposed separated from the first electrode; and an active layer formed of an organic semiconductor material provided from the first electrode to the second electrode,
wherein the organic semiconductor material includes the dioxaanthanthrene compound according to (11) or (12).

(14) The electronic device according to (13), which is a display element, a display device, a solar cell, or a sensor.
(15) «Method for forming laminated structure: first embodiment»
A method for forming a laminated structure, the method including the steps, in the sequence set forth, of:

forming a first layer formed of a first organic material on a support; and
forming a second layer formed of a second organic material that is different from the first organic material by forming on the first layer a second organic material solution layer in which the second organic material is dissolved in a solvent, and then drying the second organic material solution layer,
wherein when the second organic material solution layer has been formed on the first layer, the first organic material and the second organic material mix at an interface between the first layer and the second organic material solution layer due to a surface of the first layer being dissolved by the solvent included in the second organic material solution layer, and
wherein when the second organic material solution layer has dried, the first layer and the second layer separate.

(16) The method for forming a laminated structure according to (15), wherein a rate at which the first layer is dissolved

in the solvent when the second organic material solution layer has been formed on the first layer is more than 0 nm/min to 50 nm/min or less.

(17) «Method for forming laminated structure: second embodiment»

A method for forming a laminated structure, for obtaining a laminated structure of a first layer formed of a first organic material and a second layer formed of a second organic material that is different from the first organic material, the method including forming on a support a first organic material solution layer in which the first organic material is dissolved in a first solvent and a second organic material solution layer in which the second organic material is dissolved in a second solvent, and then drying the first organic material solution layer and the second organic material solution layer,

wherein when the first organic material solution layer and the second organic material solution layer have been formed on the support, the first organic material and the second organic material mix at an interface between the first organic material solution layer and the second organic material solution layer, and

wherein when first organic material solution layer and the second organic material solution layer have dried, the first layer and the second layer separate.

(18) The method for forming a laminated structure according to any one of (15) to (17), wherein a combination of the first organic material and the second organic material is configured by a combination of materials so that a change in Gibbs free energy before and after mixing the first organic material and the second organic material is positive.

(19) The method for forming a laminated structure according to any one of (15) to (18), wherein the first organic material and the second organic material are formed of a non-curable material.

(20) «Method for manufacturing electronic device: first embodiment»

A method for manufacturing an electronic device, the method including at least the steps, in the sequence set forth, of:

(A) forming on a base a control electrode and a first insulating layer covering the control electrode;
(B) forming on the first insulating layer a first layer formed of a first organic material; and
(C) forming a second layer formed of a second organic material that is different from the first organic material by forming on the first layer a second organic material solution layer in which the second organic material is dissolved in a solvent, and then drying the second organic material solution layer,

wherein the first organic material is formed of an insulating material and the second organic material is formed of an organic semiconductor material,

wherein a second insulating layer is configured by the first layer,

wherein an active layer is configured by the second layer,

wherein when the second organic material solution layer has been formed on the first layer, the first organic material and the second organic material mix at an interface between the first layer and the second organic material solution layer due to a surface of the first layer being dissolved by the solvent included in the second organic material solution layer, and

wherein when the second organic material solution layer has dried, the first layer and the second layer separate.

(21) «Method for manufacturing electronic device: second embodiment»

A method for manufacturing an electronic device, the method including at least the steps, in the sequence set forth, of:

(A) forming a control electrode in a groove portion formed in a base;
(B) forming on the base and the control electrode a first layer formed of a first organic material; and
(C) forming a second layer formed of a second organic material that is different from the first organic material by forming on the first layer a second organic material solution layer in which the second organic material is dissolved in a solvent, and then drying the second organic material solution layer,

wherein the first organic material is formed of an insulating material and the second organic material is formed of an organic semiconductor material,

wherein an insulating layer is configured by the first layer,

wherein an active layer is configured by the second layer, wherein when the second organic material solution layer has been formed on the first layer, the first organic material and the second organic material mix at an interface between the first layer and the second organic material solution layer due to a surface of the first layer being dissolved by the solvent included in the second organic material solution layer, and

wherein when the second organic material solution layer has dried, the first layer and the second layer separate.

(22) «Method for manufacturing electronic device: third embodiment»

A method for manufacturing an electronic device, the method including at least the steps, in the sequence set forth, of:

(A) forming on a base a control electrode and a first insulating layer covering the control electrode; and

(B) obtaining a laminated structure of a first layer formed of a first organic material and a second layer formed of a second organic material that is different from the first organic material by forming on the first insulating layer a first organic material solution layer in which the first organic material is dissolved in a first solvent and a second organic material solution layer in which the second organic material is dissolved in a second solvent, and then drying the first organic material solution layer and the second organic material solution layer,

wherein the first organic material is formed of an insulating material and the second organic material is formed of an organic semiconductor material,
wherein a second insulating layer is configured by the first layer,
wherein an active layer is configured by the second layer,
wherein when the first organic material solution layer and the second organic material solution layer have been formed, the first organic material and the second organic material mix at an interface between the first organic material solution layer and the second organic material solution layer, and
wherein when the first organic material solution layer and the second organic material solution layer have dried, the first layer and the second layer separate.
(23) «Method for manufacturing electronic device: fourth embodiment»
A method for manufacturing an electronic device, the method including at least the steps, in the sequence set forth, of:

(A) forming a control electrode in a groove portion formed in a base; and

(B) obtaining a laminated structure of a first layer formed of a first organic material and a second layer formed of a second organic material that is different from the first organic material by forming on the base and the control electrode a first organic material solution layer in which the first organic material is dissolved in a first solvent and a second organic material solution layer in which the second organic material is dissolved in a second solvent, and then drying the first organic material solution layer and the second organic material solution layer,

wherein the first organic material is formed of an insulating material and the second organic material is formed of an organic semiconductor material,
wherein an insulating layer is configured by the first layer,
wherein an active layer is configured by the second layer,
wherein when the first organic material solution layer and the second organic material solution layer have been formed, the first organic material and the second organic material mix at an interface between the first organic material solution layer and the second organic material solution layer, and
wherein when the first organic material solution layer and the second organic material solution layer have dried, the first layer and the second layer separate.
(24) The method for manufacturing an electronic device according to any one of (20) to (23), further including forming a first electrode and a second electrode on the second layer after the second layer has been formed.
(25) <<Method for manufacturing electronic device: fifth embodiment>>
A method for manufacturing an electronic device, the method including at least the steps, in the sequence set forth, of:

(A) forming a first electrode and a second electrode in a groove portion formed in a base;

(B) forming on the base, the first electrode, and the second electrode a first layer formed of a first organic material; and

(C) forming a second layer formed of a second organic material that is different from the first organic material by forming on the first layer a second organic material solution layer in which the second organic material is dissolved in a solvent, and then drying the second organic material solution layer,

wherein the first organic material is formed of an organic semiconductor material and the second organic material is formed of an insulating material,
wherein an active layer is configured by the first layer,
wherein an insulating layer is configured by the second layer,
wherein when the second organic material solution layer has been formed on the first layer, the first organic material and the second organic material mix at an interface between the first layer and the second organic material solution layer due to a surface of the first layer being dissolved by the solvent included in the second organic material solution layer, and
wherein when the second organic material solution layer has dried, the first layer and the second layer separate.
(26) «Method for manufacturing electronic device: sixth embodiment»

A method for manufacturing an electronic device, the method including at least the steps, in the sequence set forth, of:

(A) forming a first electrode and a second electrode in a groove portion formed in a base; and
(B) obtaining a laminated structure of a first layer formed of a first organic material and a second layer formed of a second organic material that is different from the first organic material by forming on the base, the first electrode, and the second electrode a first organic material solution layer in which the first organic material is dissolved in a first solvent and a second organic material solution layer in which the second organic material is dissolved in a second solvent, and then drying the first organic material solution layer and the second organic material solution layer,

wherein the first organic material is formed of an organic semiconductor material and the second organic material is formed of an insulating material,
wherein an active layer is configured by the first layer,
wherein an insulating layer is configured by the second layer,
wherein when the first organic material solution layer and the second organic material solution layer have been formed, the first organic material and the second organic material mix at an interface between the first organic material solution layer and the second organic material solution layer, and
wherein when the first organic material solution layer and the second organic material solution layer have dried, the first layer and the second layer separate.
(27) The method for manufacturing an electronic device according to (25) or (26), further including forming a control electrode on the second layer after the second layer has been formed.
(28) The method for manufacturing an electronic device according to (20) or (25), wherein a rate at which the first layer is dissolved in the solvent when the second organic material solution layer has been formed on the first layer is more than 0 nm/min to 50 nm/min or less.
(29) The method for manufacturing an electronic device according to any one of (20) to (28), wherein a combination of the first organic material and the second organic material is configured by a combination of materials so that a change in Gibbs free energy before and after mixing the first organic material and the second organic material is positive.
(30) The method for manufacturing an electronic device according to any one of (20) to (29), wherein the first organic material and the second organic material are formed of a non-curable material.
(31) <<Laminated structure>>
A laminated structure including a first layer formed of a first organic material and a second layer formed of a second organic material that is different from the first organic material,
wherein a combination of the first organic material and the second organic material is configured by a combination of materials so that a value obtained by subtracting a Gibbs free energy $G_1$ of the first organic material and a Gibbs free energy $G_2$ of the second organic material from a Gibbs free energy $G_0$ of a mixed system of the first organic material and the second organic material is positive.
(32) The laminated structure according to (31), wherein the first organic material and the second organic material do not mix at an interface between the first layer and the second layer, and the first layer and the second layer are separated.
(33) The laminated structure according to (32),
wherein, by forming on the first layer a second organic material solution layer in which the second organic material is dissolved in a solvent, the first organic material and the second organic material mix at an interface between the first layer and the second organic material solution layer due to a surface of the first layer being dissolved by the solvent included in the second organic material solution layer, and
wherein when the second organic material solution layer has dried, the first layer and the second layer separate.
(34) The laminated structure according to (33), wherein a rate at which the first layer is dissolved in the solvent when the second organic material solution layer has been formed on the first layer is more than 0 nm/min to 50 nm/min or less.
(35) The laminated structure according to (32), wherein by forming a first organic material solution layer in which a first organic material is dissolved in a first solvent and a second organic material solution layer in which a second organic material different from the first organic material is dissolved in a second solvent, and then drying the first organic material solution layer and the second organic material solution layer, the first organic material and the second organic material mix at an interface between the first organic material solution layer and the second organic material solution layer, and when first organic material solution layer and the second organic material solution layer have dried, the first layer and the second layer separate.
(36) The laminated structure according to any one of (31) to (35), wherein the first organic material and the second organic material are formed of a non-curable material.

(37) «Electronic device»
An electronic device including an electrode structure, an insulating layer, and an active layer,
wherein the insulating layer is formed of a first organic material configured from an insulating material,
wherein the active layer is formed of a second organic material configured from an organic semiconductor material, and
wherein a combination of the first organic material and the second organic material is configured by a combination of materials so that a value obtained by subtracting a Gibbs free energy $G_1$ of the first organic material and a Gibbs free energy $G_2$ of the second organic material from a Gibbs free energy $G_0$ of a mixed system of the first organic material and the second organic material is positive.
(38) The electronic device according to (37), wherein the first organic material and the second organic material do not mix at an interface between the insulating layer and the active layer, and the insulating layer and the active layer are separated.
(39) The electronic device according to (38),
wherein, by forming on the first layer (the layer constituting the insulating layer or the active layer) a second organic material solution layer in which the second organic material (the material constituting the active layer or the insulating layer) is dissolved in a solvent, the first organic material and the second organic material mix at an interface between the first layer and a second organic material solution layer due to a surface of the first layer being dissolved by the solvent included in the second organic material solution layer, and
wherein when the second organic material solution layer has dried, the first layer (the layer constituting the insulating layer or the active layer) and the second layer (the layer constituting the active layer or the insulating layer) separate.
(40) The electronic device according to (39), wherein a rate at which the first layer is dissolved in the solvent when the second organic material solution layer has been formed on the first layer is more than 0 nm/min to 50 nm/min or less.
(41) The electronic device according to (38), wherein by forming a first organic material solution layer in which a first organic material is dissolved in a first solvent and a second organic material solution layer in which a second organic material different from the first organic material is dissolved in a second solvent, and then drying the first organic material solution layer and the second organic material solution layer, the first organic material and the second organic material mix at an interface between the first organic material solution layer and the second organic material solution layer, and when first organic material solution layer and the second organic material solution layer have dried, the first layer (the layer constituting the insulating layer or the active layer) and the second layer (the layer constituting the active layer or the insulating layer) separate.
(42) The electronic device according to any one of (37) to (41), wherein the first organic material and the second organic material are formed of a non-curable material.

Reference Signs List

10 base
11 glass substrate
12 insulating film
14 gate electrode (control electrode)
15 gate insulating layer (insulating layer)
16 source/drain electrodes (first electrode and second electrode)
17 channel formation region
18 channel formation region channel extension portion
20, 33 active layer
31 first electrode
32 second electrode
43 groove portion
44 control electrode (gate electrode)
45 insulating layer (gate insulating layer)
45A first insulating layer (first gate insulating layer)
45B second insulating layer (second gate insulating layer)
46 first electrode and second electrode (source/drain electrodes)
47 channel formation region
48 channel formation region channel extension portion
50 active layer

60 active layer
61 first electrode
62 second electrode
63 charge injection layer
101, 201, 301, 401, 501, 601, 701 first layer
102, 202, 302, 402, 502, 602, 702 second layer
301', 401', 601' first organic material solution layer
102', 202', 302', 402', 502', 602' second organic material solution layer
103, 203, 303, 403, 503, 603 region where the first organic material and the second organic material mix

## Claims

1. A dioxaanthanthrene compound represented by any one of structural formulae selected from the group consisting of the following structural formula (1) to structural formula (9),

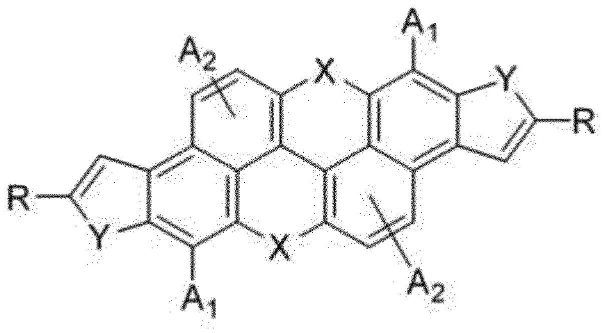

(1)

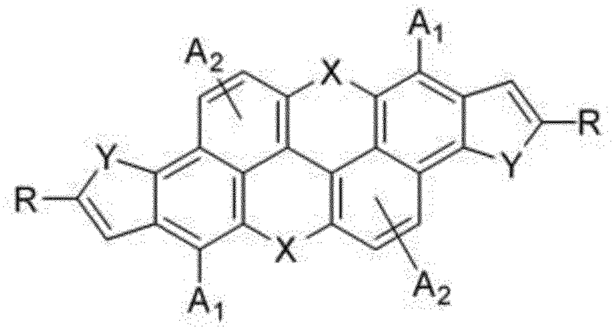

(2)

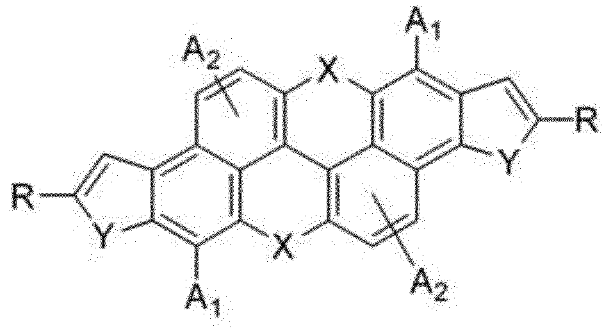

(3)

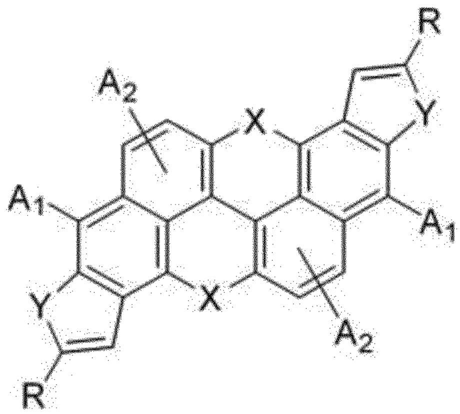

(4)

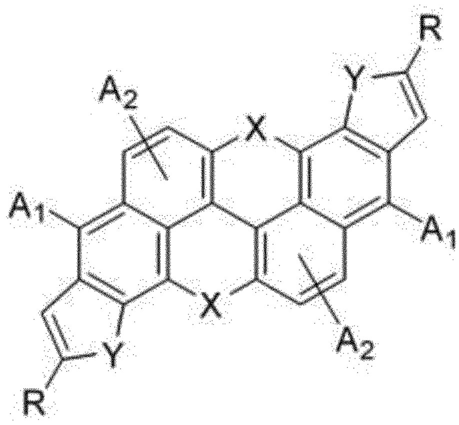

(5)

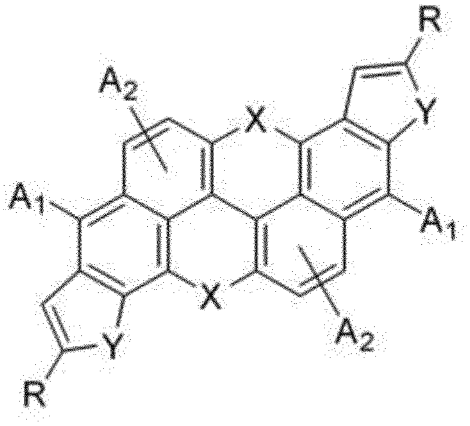

(6)

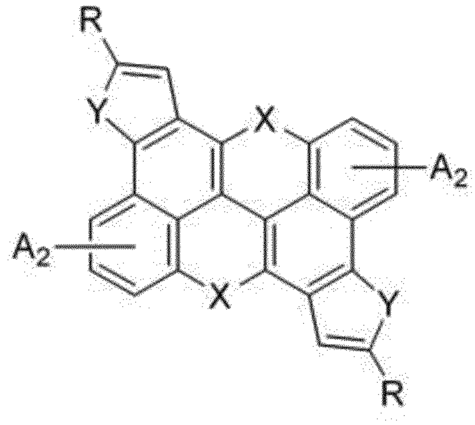

(7)

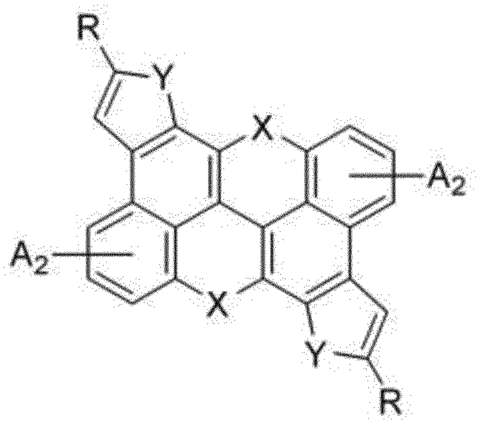

(8)

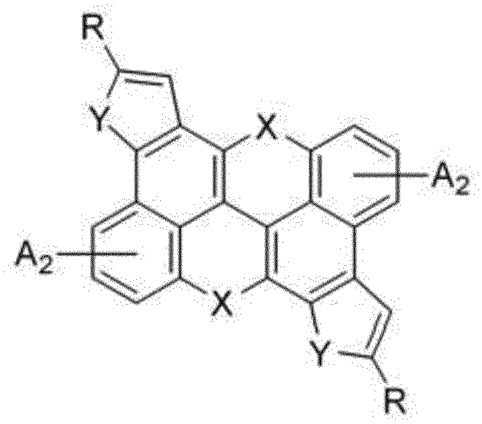

(9)

wherein X represents one atom selected from the group consisting of oxygen, sulfur, selenium and tellurium, wherein Y represents one atom selected from the group consisting of oxygen, sulfur, selenium and tellurium, and wherein R, $A_1$, and $A_2$ each represent a hydrogen atom or a substituent selected from the group consisting of an alkyl group, a cycloalkyl group, an alkenyl group, an alkynyl group, an aryl group, an arylalkyl group, an aromatic heterocycle, a heterocyclic group, an alkoxy group, a cycloalkoxy group, an aryloxy group, an alkylthio group, a cycloalkylthio group, an arylthio group, an alkoxycarbonyl group, an aryloxycarbonyl group, a sulfamoyl group, an acyl group, an acyloxy group, an amide group, a carbamoyl group, a ureido group, a sulfinyl group, an alkylsulfonyl group, an arylsulfonyl group, an amino group, a halogen atom, a fluorinated hydrocarbon group, a cyano group, a nitro group, a hydroxy group, a mercapto group, and a silyl group.

**2.** The dioxaanthanthrene compound according to claim 1, wherein R, $A_1$, and $A_2$ each represent a hydrogen atom or a substituent selected from the group consisting of an alkyl group, an alkenyl group, an aryl group, an arylalkyl group, an aromatic heterocycle, and a halogen atom.

**3.** The dioxaanthanthrene compound according to claim 1, wherein X represents oxygen.

**4.** The dioxaanthanthrene compound according to claim 1, wherein Y represents sulfur.

**5.** An electronic device comprising at least:

a first electrode;
a second electrode disposed separated from the first electrode; and
an active layer formed of an organic semiconductor material provided from the first electrode to the second electrode,
wherein the organic semiconductor material is formed of the dioxaanthanthrene compound according to any one of claims 1 to 4.

**6.** A dioxaanthanthrene compound represented by the following structural formula (11),

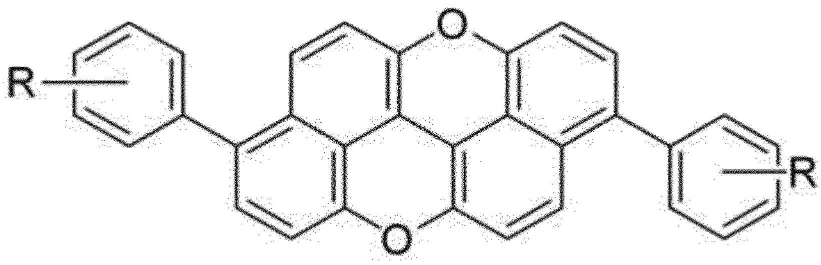

(11)

wherein R represents an alkyl group having a branch with four or more carbon atoms.

**7.** An electronic device comprising at least:

a first electrode;
a second electrode disposed separated from the first electrode; and
an active layer formed of an organic semiconductor material provided from the first electrode to the second electrode,
wherein the organic semiconductor material includes the dioxaanthanthrene compound represented by the following structural formula (11), and

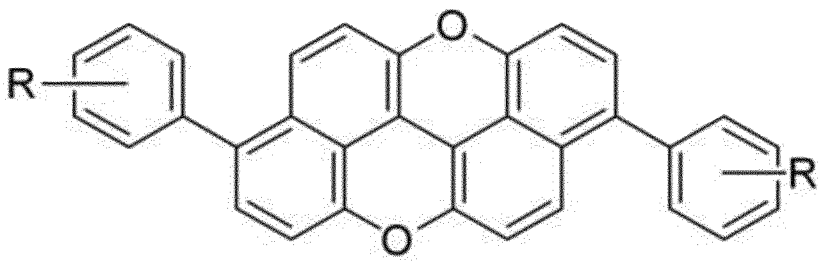

(11)

wherein R represents an alkyl group having a branch with four or more carbon atoms.

**8.** A dioxaanthanthrene compound represented by the following structural formula (21-1), or structural formula (21-2), or structural formula (21-3),

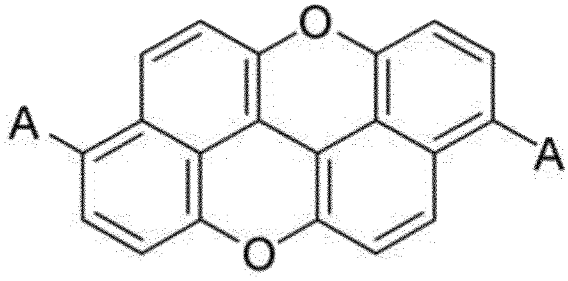

(21-1)

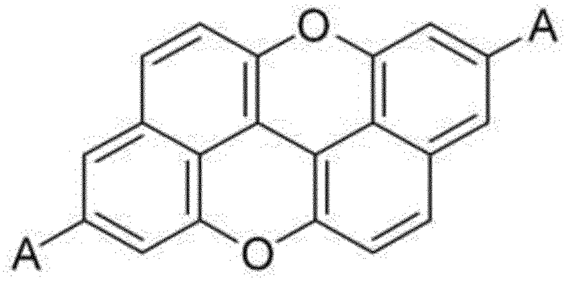

(21-2)

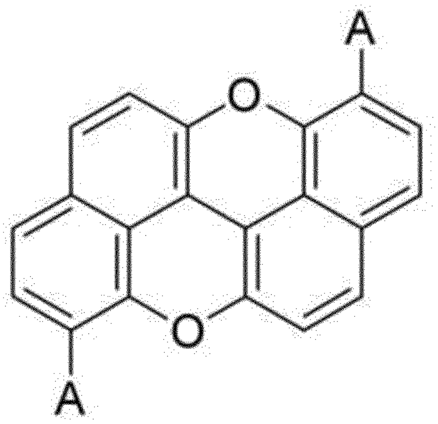

(21-3)

wherein substituent A is represented by the following structural formula (22-1) or structural formula (22-2), and wherein $X_1$, $X_2$, $X_3$, $Y_1$, $Y_2$, $Y_3$, $Y_4$, $Y_5$, $Y_6$, $Y_7$, and $Y_8$ each represent a hydrogen atom or a substituent selected from the group consisting of an alkyl group, a cycloalkyl group, an alkenyl group, an alkynyl group, an aryl group, an arylalkyl group, an aromatic heterocycle, a heterocyclic group, an alkoxy group, a cycloalkoxy group, an aryloxy group, an alkylthio group, a cycloalkylthio group, an arylthio group, an alkoxycarbonyl group, an aryloxycarbonyl group, a sulfamoyl group, an acyl group, an acyloxy group, an amide group, a carbamoyl group, a ureido group, a sulfinyl group, an alkylsulfonyl group, an arylsulfonyl group, an amino group, a halogen atom, a fluorinated hydrocarbon group, a cyano group, a nitro group, a hydroxy group, a mercapto group, and a silyl group.

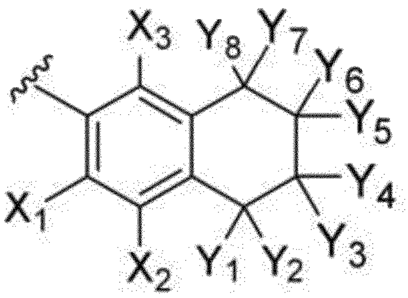

(22-1)

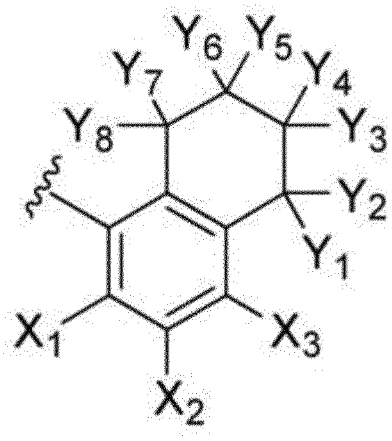

(22-2)

9. A dioxaanthanthrene compound represented by the following structural formula (23-1) or structural formula (23-2).

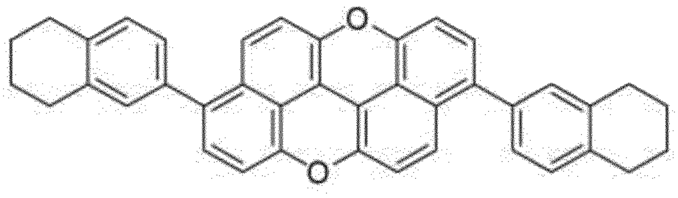

(23-1)

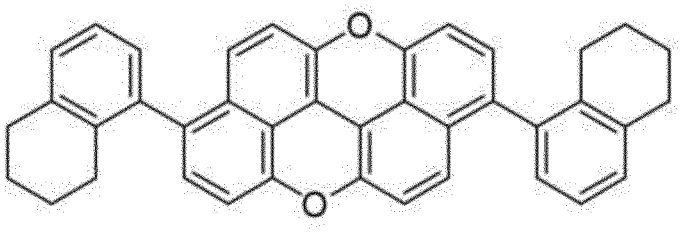

(23-2)

10. An electronic device comprising at least:

a first electrode;
a second electrode disposed separated from the first electrode; and
an active layer formed of an organic semiconductor material provided from the first electrode to the second electrode,
wherein the organic semiconductor material includes the dioxaanthanthrene compound according to claim 8 or 9.

11. A method for forming a laminated structure, the method comprising the steps, in the sequence set forth, of:

forming a first layer formed of a first organic material on a support; and
forming a second layer formed of a second organic material that is different from the first organic material by forming on the first layer a second organic material solution layer in which the second organic material is dissolved in a solvent, and then drying the second organic material solution layer,
wherein when the second organic material solution layer has been formed on the first layer, the first organic material and the second organic material mix at an interface between the first layer and the second organic material solution layer due to a surface of the first layer being dissolved by the solvent included in the second organic material solution layer, and
wherein when the second organic material solution layer has dried, the first layer and the second layer separate.

12. A method for forming a laminated structure, for obtaining a laminated structure of a first layer formed of a first organic material and a second layer formed of a second organic material that is different from the first organic material, the method comprising forming on a support a first organic material solution layer in which the first organic material is

dissolved in a first solvent and a second organic material solution layer in which the second organic material is dissolved in a second solvent, and then drying the first organic material solution layer and the second organic material solution layer,
wherein when the first organic material solution layer and the second organic material solution layer have been formed on the support, the first organic material and the second organic material mix at an interface between the first organic material solution layer and the second organic material solution layer, and
wherein when first organic material solution layer and the second organic material solution layer have dried, the first layer and the second layer separate.

**13.** A method for manufacturing an electronic device, the method comprising at least the steps, in the sequence set forth, of:

(A) forming on a base a control electrode and a first insulating layer covering the control electrode;
(B) forming on the first insulating layer a first layer formed of a first organic material; and
(C) forming a second layer formed of a second organic material that is different from the first organic material by forming on the first layer a second organic material solution layer in which the second organic material is dissolved in a solvent, and then drying the second organic material solution layer,

wherein the first organic material is formed of an insulating material and the second organic material is formed of an organic semiconductor material,
wherein a second insulating layer is configured by the first layer,
wherein an active layer is configured by the second layer,
wherein when the second organic material solution layer has been formed on the first layer, the first organic material and the second organic material mix at an interface between the first layer and the second organic material solution layer due to a surface of the first layer being dissolved by the solvent included in the second organic material solution layer, and
wherein when the second organic material solution layer has dried, the first layer and the second layer separate.

**14.** A method for manufacturing an electronic device, the method comprising at least the steps, in the sequence set forth, of:

(A) forming a control electrode in a groove portion formed in a base;
(B) forming on the base and the control electrode a first layer formed of a first organic material; and
(C) forming a second layer formed of a second organic material that is different from the first organic material by forming on the first layer a second organic material solution layer in which the second organic material is dissolved in a solvent, and then drying the second organic material solution layer,

wherein the first organic material is formed of an insulating material and the second organic material is formed of an organic semiconductor material,
wherein an insulating layer is configured by the first layer,
wherein an active layer is configured by the second layer,
wherein when the second organic material solution layer has been formed on the first layer, the first organic material and the second organic material mix at an interface between the first layer and the second organic material solution layer due to a surface of the first layer being dissolved by the solvent included in the second organic material solution layer, and
wherein when the second organic material solution layer has dried, the first layer and the second layer separate.

**15.** A method for manufacturing an electronic device, the method comprising at least the steps, in the sequence set forth, of:

(A) forming on a base a control electrode and a first insulating layer covering the control electrode; and
(B) obtaining a laminated structure of a first layer formed of a first organic material and a second layer formed of a second organic material that is different from the first organic material by forming on the first insulating layer a first organic material solution layer in which the first organic material is dissolved in a first solvent and a second organic material solution layer in which the second organic material is dissolved in a second solvent, and then drying the first organic material solution layer and the second organic material solution layer,

wherein the first organic material is formed of an insulating material and the second organic material is formed of

an organic semiconductor material,
wherein a second insulating layer is configured by the first layer,
wherein an active layer is configured by the second layer,
wherein when the first organic material solution layer and the second organic material solution layer have been formed, the first organic material and the second organic material mix at an interface between the first organic material solution layer and the second organic material solution layer, and
wherein when the first organic material solution layer and the second organic material solution layer have dried, the first layer and the second layer separate.

**16.** A method for manufacturing an electronic device, the method comprising at least the steps, in the sequence set forth, of:

(A) forming a control electrode in a groove portion formed in a base; and
(B) obtaining a laminated structure of a first layer formed of a first organic material and a second layer formed of a second organic material that is different from the first organic material by forming on the base and the control electrode a first organic material solution layer in which the first organic material is dissolved in a first solvent and a second organic material solution layer in which the second organic material is dissolved in a second solvent, and then drying the first organic material solution layer and the second organic material solution layer,

wherein the first organic material is formed of an insulating material and the second organic material is formed of an organic semiconductor material,
wherein an insulating layer is configured by the first layer,
wherein an active layer is configured by the second layer,
wherein when the first organic material solution layer and the second organic material solution layer have been formed, the first organic material and the second organic material mix at an interface between the first organic material solution layer and the second organic material solution layer, and
wherein when the first organic material solution layer and the second organic material solution layer have dried, the first layer and the second layer separate.

**17.** A method for manufacturing an electronic device, the method comprising at least the steps, in the sequence set forth, of:

(A) forming a first electrode and a second electrode in a groove portion formed in a base;
(B) forming on the base, the first electrode, and the second electrode a first layer formed of a first organic material; and
(C) forming a second layer formed of a second organic material that is different from the first organic material by forming on the first layer a second organic material solution layer in which the second organic material is dissolved in a solvent, and then drying the second organic material solution layer,

wherein the first organic material is formed of an organic semiconductor material and the second organic material is formed of an insulating material,
wherein an active layer is configured by the first layer,
wherein an insulating layer is configured by the second layer,
wherein when the second organic material solution layer has been formed on the first layer, the first organic material and the second organic material mix at an interface between the first layer and the second organic material solution layer due to a surface of the first layer being dissolved by the solvent included in the second organic material solution layer, and
wherein when the second organic material solution layer has dried, the first layer and the second layer separate.

**18.** A method for manufacturing an electronic device, the method comprising at least the steps, in the sequence set forth, of:

(A) forming a first electrode and a second electrode in a groove portion formed in a base; and
(B) obtaining a laminated structure of a first layer formed of a first organic material and a second layer formed of a second organic material that is different from the first organic material by forming on the base, the first electrode, and the second electrode a first organic material solution layer in which the first organic material is dissolved in a first solvent and a second organic material solution layer in which the second organic material is

dissolved in a second solvent, and then drying the first organic material solution layer and the second organic material solution layer,

wherein the first organic material is formed of an organic semiconductor material and the second organic material is formed of an insulating material,
wherein an active layer is configured by the first layer,
wherein an insulating layer is configured by the second layer,
wherein when the first organic material solution layer and the second organic material solution layer have been formed, the first organic material and the second organic material mix at an interface between the first organic material solution layer and the second organic material solution layer, and
wherein when the first organic material solution layer and the second organic material solution layer have dried, the first layer and the second layer separate.

**19.** A laminated structure comprising a first layer formed of a first organic material and a second layer formed of a second organic material that is different from the first organic material,
wherein a combination of the first organic material and the second organic material is configured by a combination of materials so that a value obtained by subtracting a Gibbs free energy $G_1$ of the first organic material and a Gibbs free energy $G_2$ of the second organic material from a Gibbs free energy $G_0$ of a mixed system of the first organic material and the second organic material is positive.

**20.** An electronic device comprising an electrode structure, an insulating layer, and an active layer,
wherein the insulating layer is formed of a first organic material configured from an insulating material,
wherein the active layer is formed of a second organic material configured from an organic semiconductor material, and
wherein a combination of the first organic material and the second organic material is configured by a combination of materials so that a value obtained by subtracting a Gibbs free energy $G_1$ of the first organic material and a Gibbs free energy $G_2$ of the second organic material from a Gibbs free energy $G_0$ of a mixed system of the first organic material and the second organic material is positive.

# FIG. 1

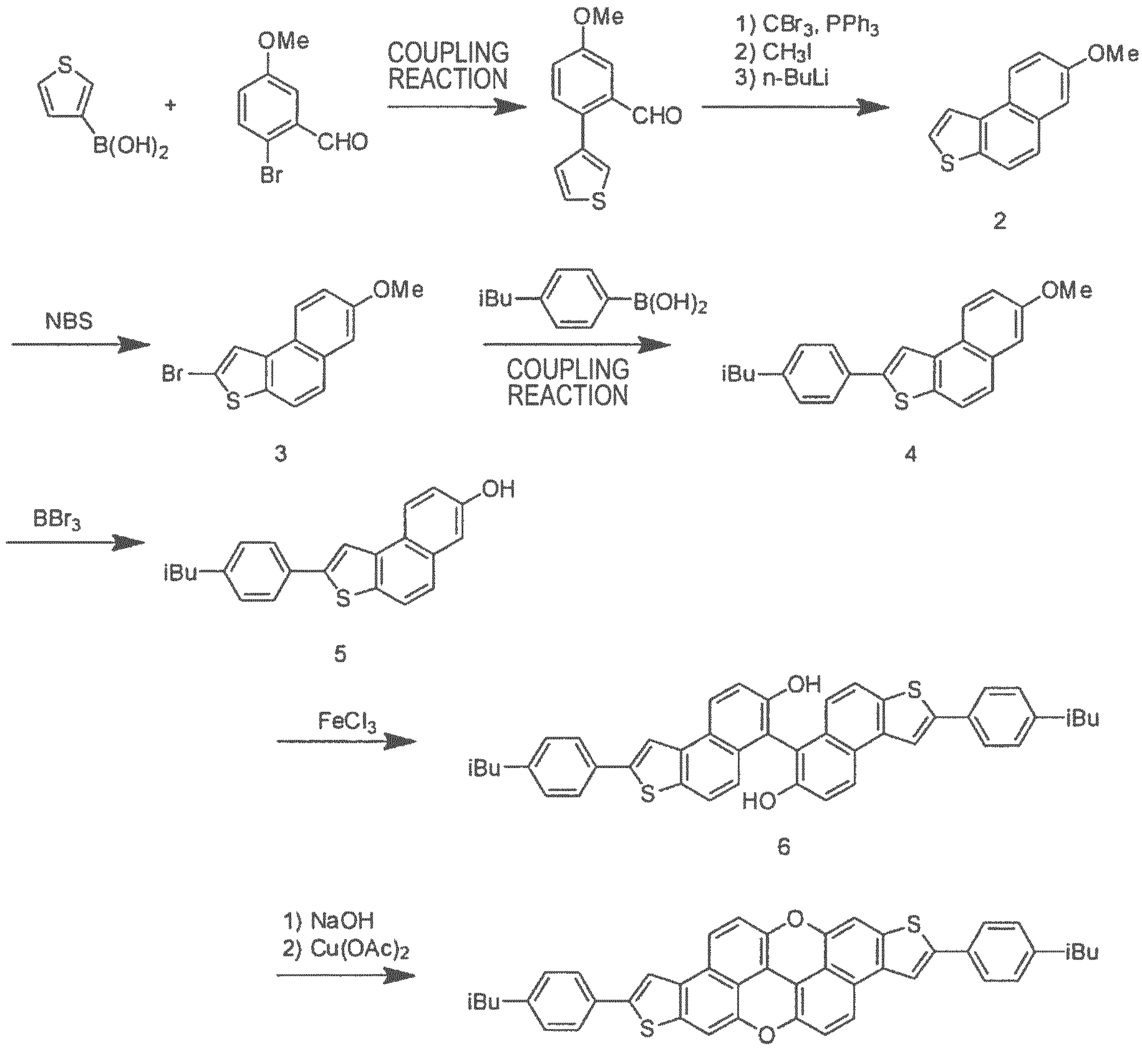
COUPLING REACTION
1) CBr3, PPh3
2) CH3I
3) n-BuLi
NBS
COUPLING REACTION
BBr3
FeCl3
1) NaOH
2) Cu(OAc)2

2

3

4

5

6

1

# FIG. 2

A
STEP-420

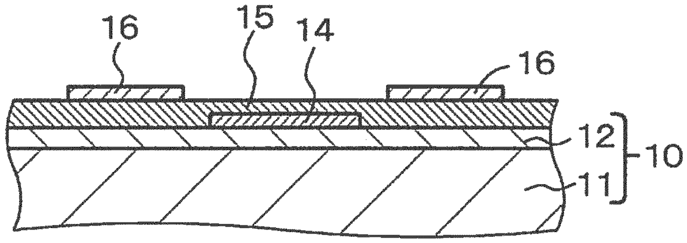


B
STEP-430

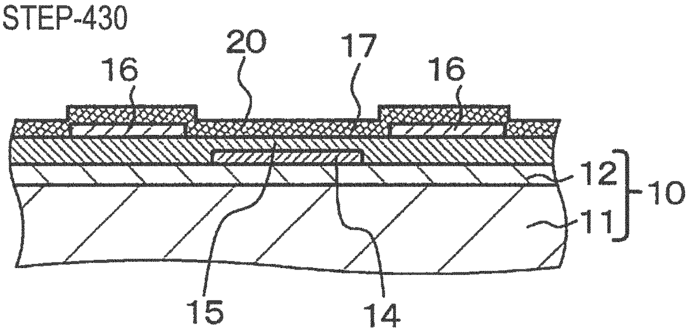


# FIG. 3

A
STEP-510

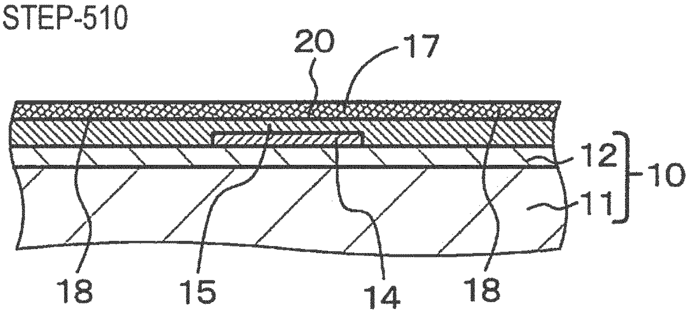


B
STEP-520

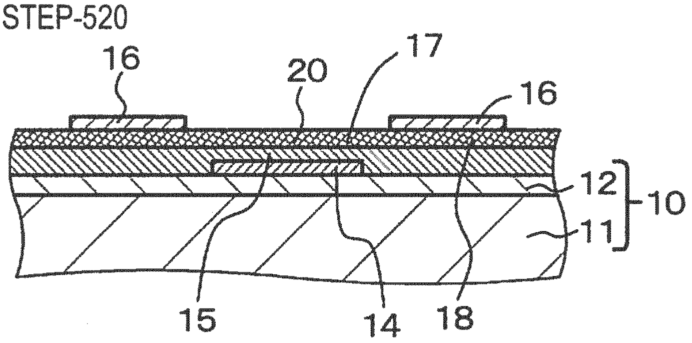

FIG. 4

A

STEP-600

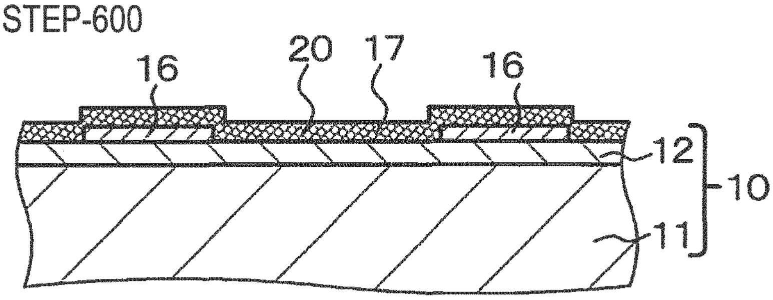


B

STEP-610

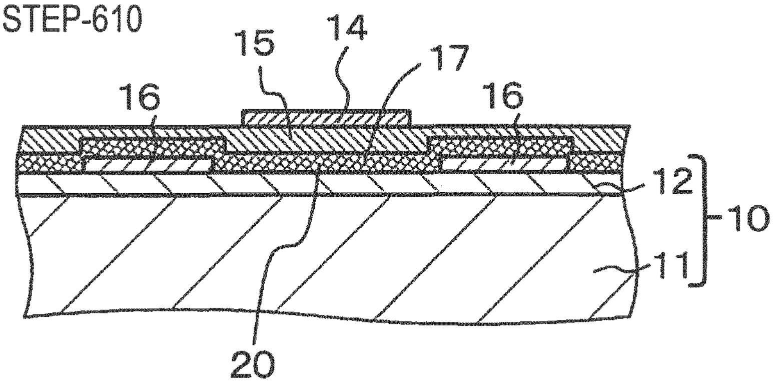

FIG. 5

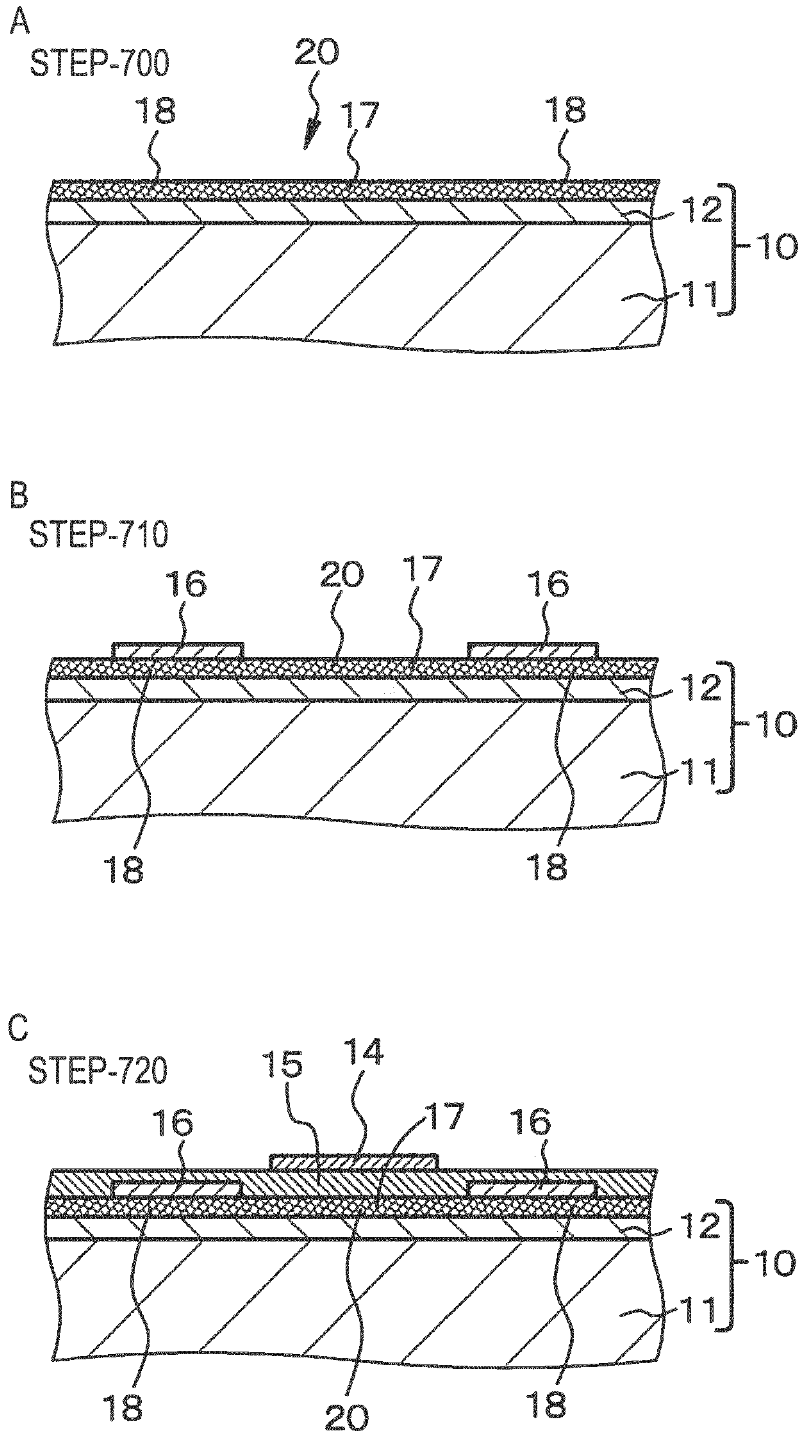
A
STEP-700
20
18
17
18
12
10
11
B
STEP-710
16
20
17
16
12
10
11
18
18
C
STEP-720
15
14
17
16
16
12
10
11
18
20
18

# FIG. 6

A

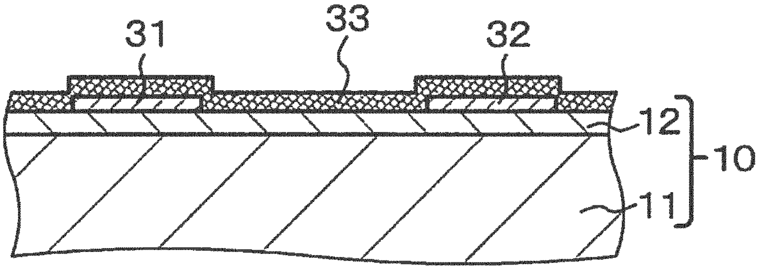


B

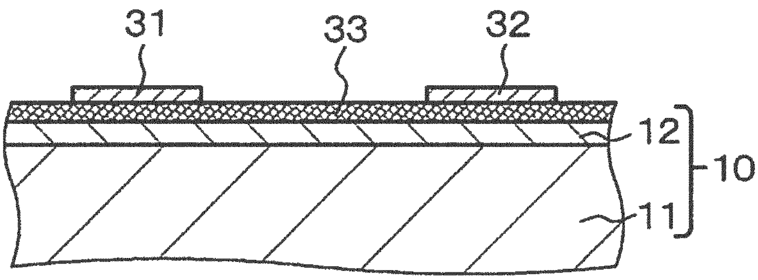

# FIG. 7

A STEP-910

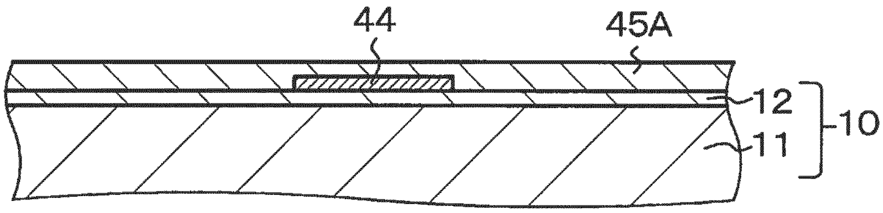


B STEP-920

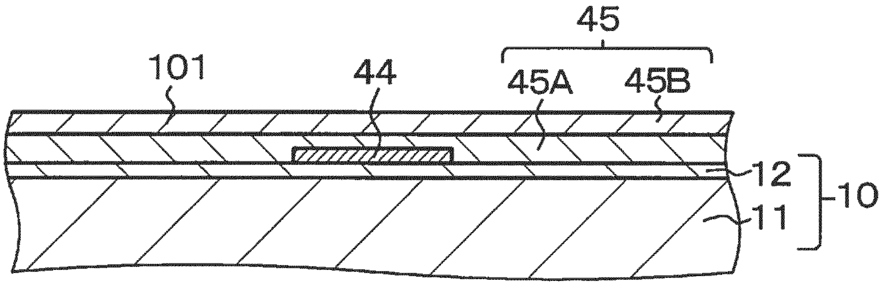


C STEP-930

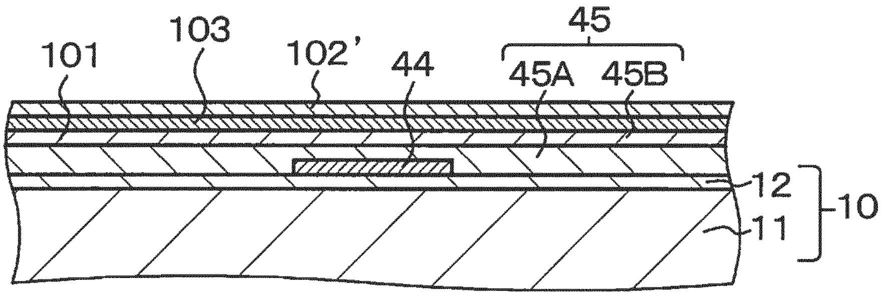


D STEP-930 (CONTINUED)

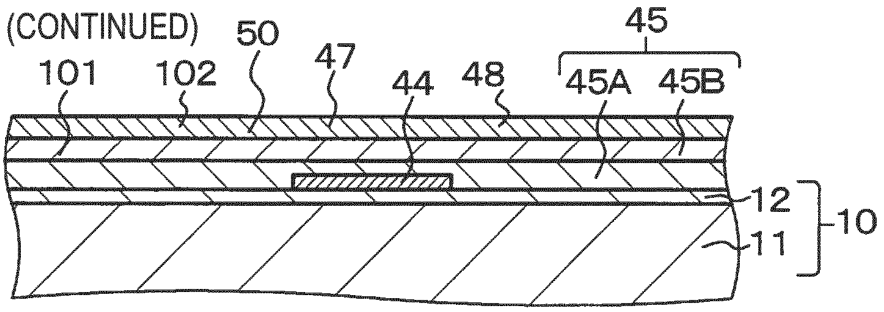


E STEP-940

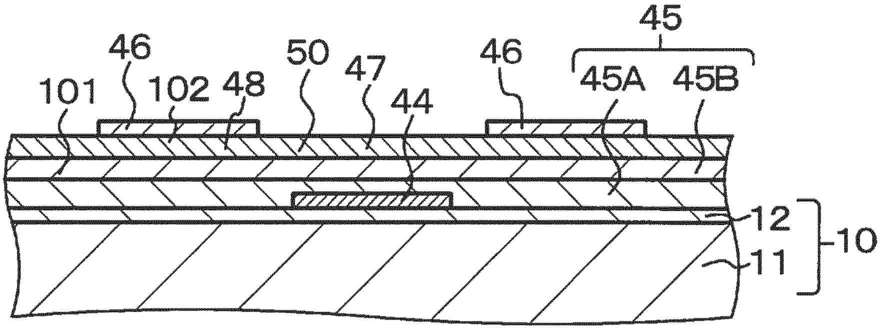

# FIG. 8

A STEP-1000

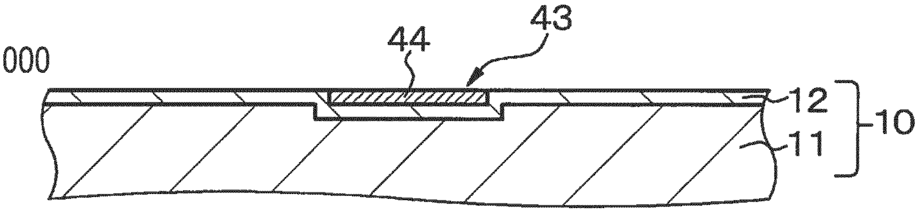


B STEP-1010

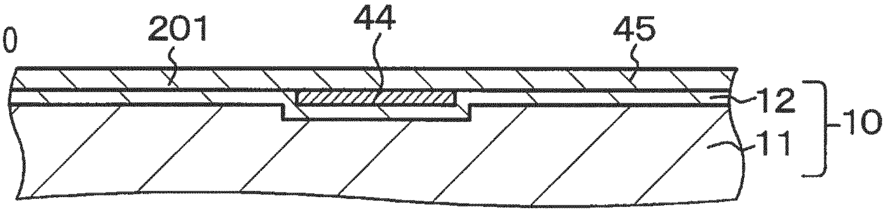


C STEP-1020

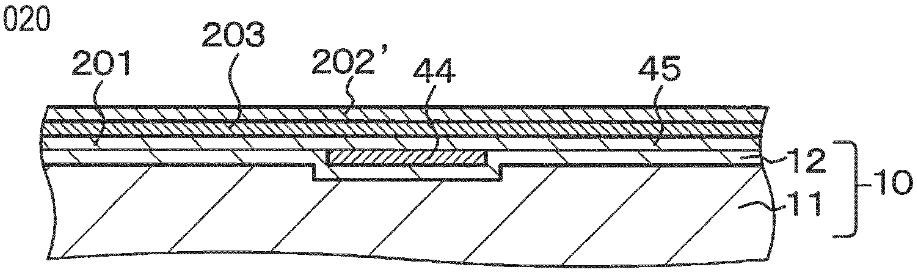


D STEP-1020 (CONTINUED)

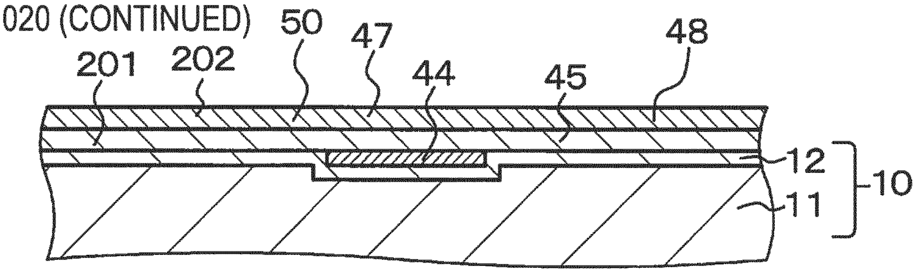


E STEP-1030

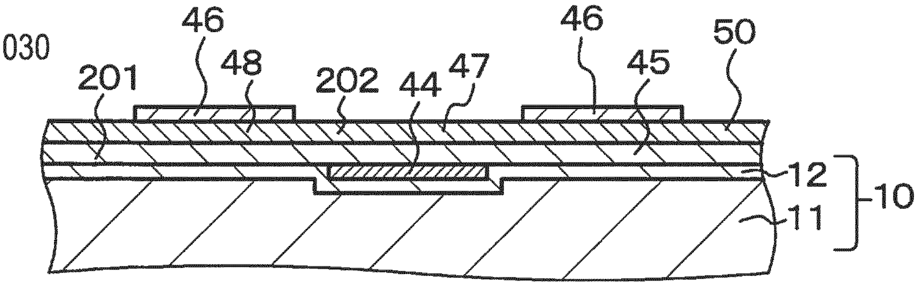

FIG. 9

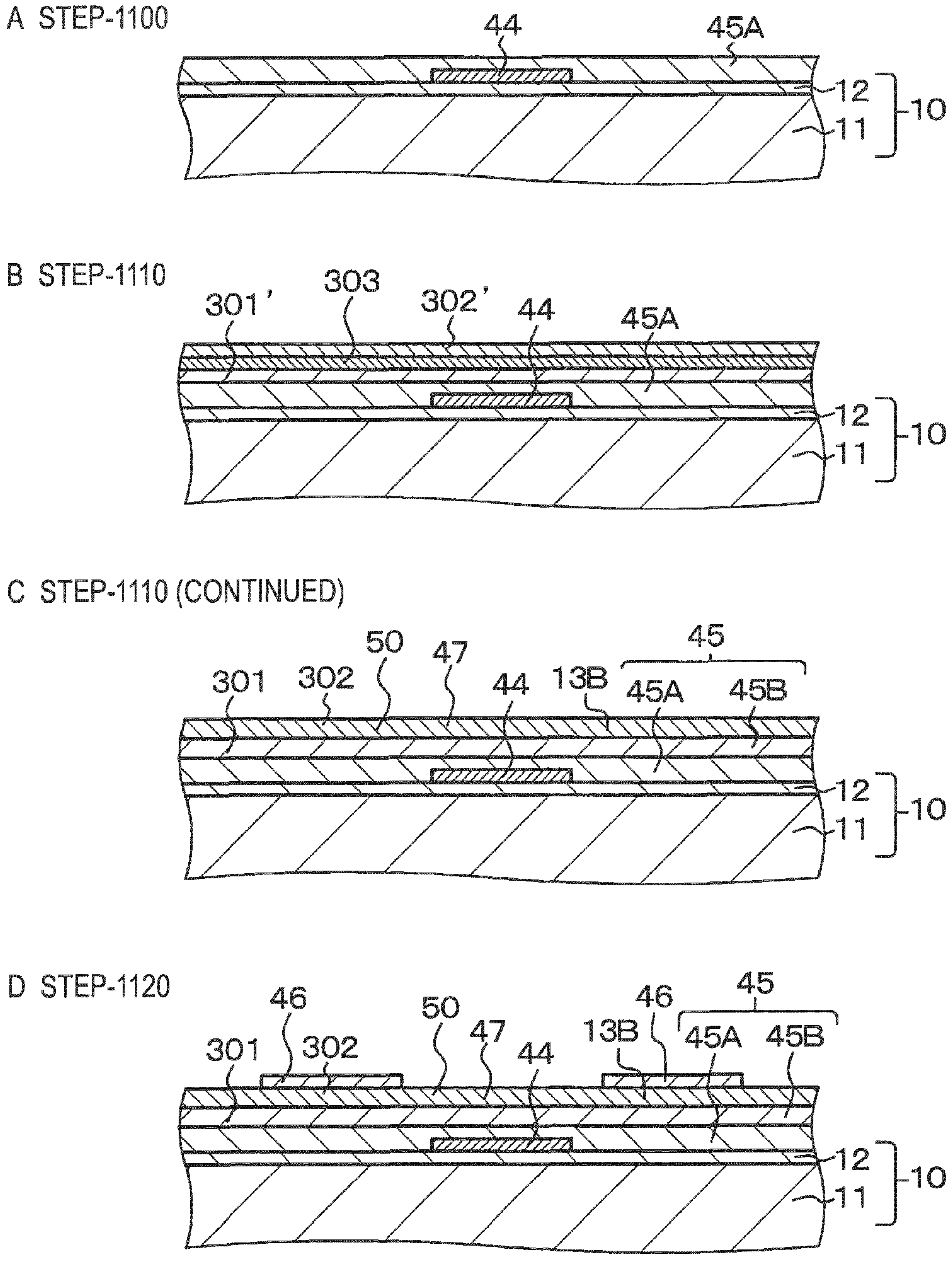
A STEP-1100
44
45A
12
10
11
B STEP-1110
301'
303
302'
44
45A
12
10
11
C STEP-1110 (CONTINUED)
50
47
45
301
302
44
13B
45A
45B
12
10
11
D STEP-1120
46
50
46
45
301
302
47
44
13B
45A
45B
12
10
11

# FIG. 10

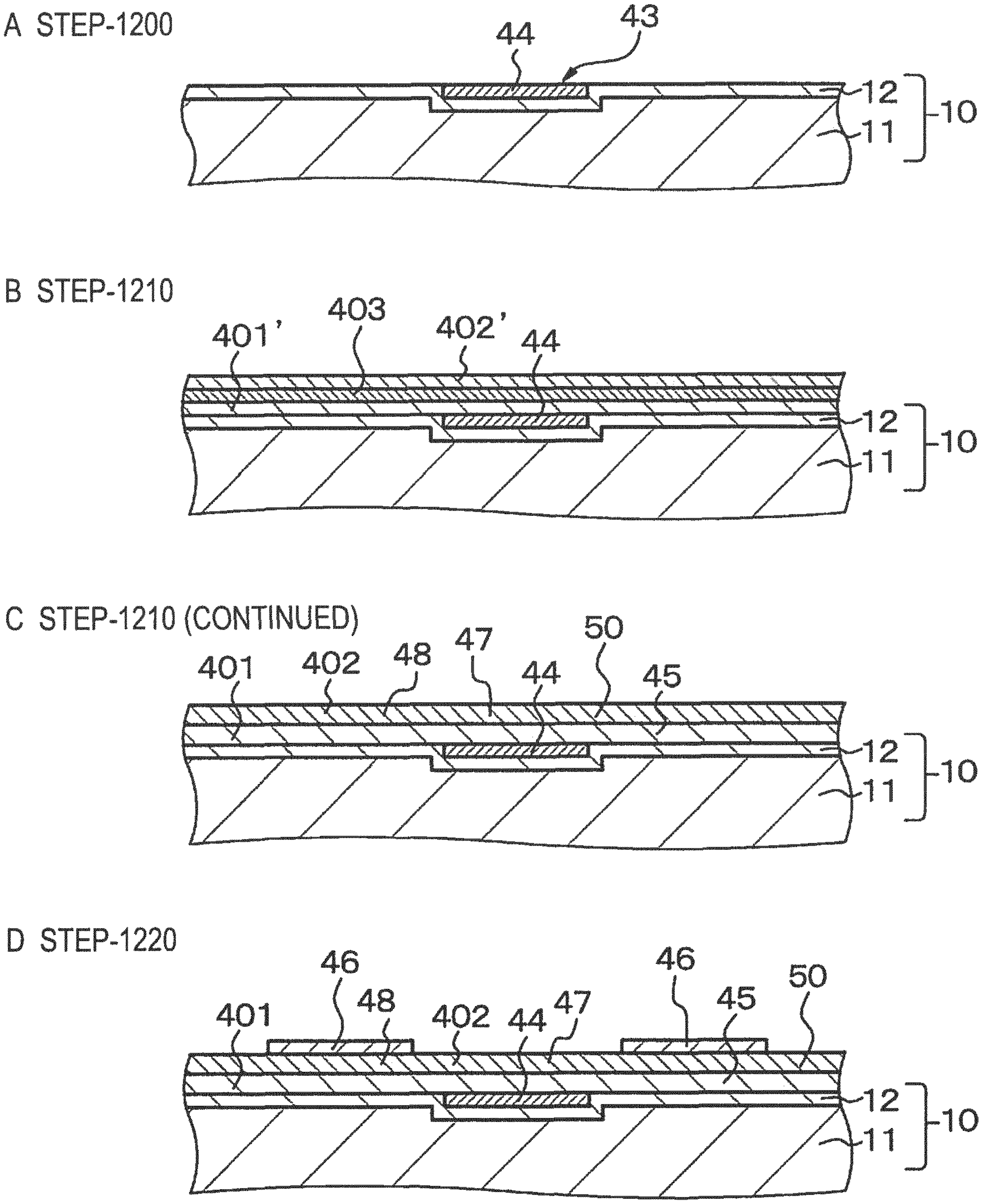
A STEP-1200
44
43
12
10
11
B STEP-1210
401'
403
402'
44
12
10
11
C STEP-1210 (CONTINUED)
401
402
48
47
44
50
45
12
10
11
D STEP-1220
46
48
401
402
44
47
46
45
50
12
10
11

# FIG. 11

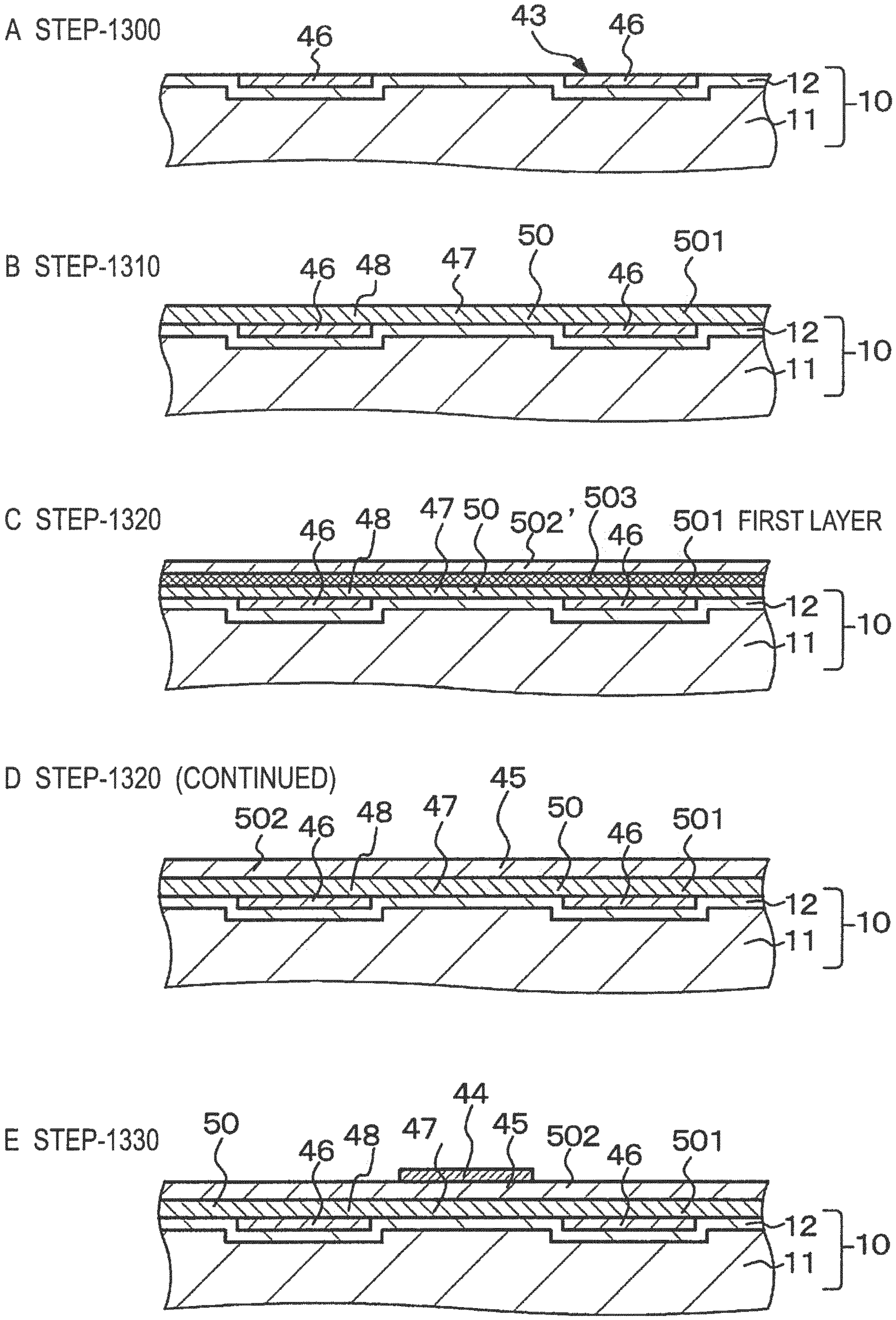
A STEP-1300
46
43
46
12
10
11
B STEP-1310
46
48
47
50
501
46
12
10
11
C STEP-1320
46
48
47
50
502'
503
46
501
FIRST LAYER
12
10
11
D STEP-1320 (CONTINUED)
502
46
48
47
45
50
46
501
12
10
11
E STEP-1330
50
46
48
47
44
45
502
46
501
12
10
11

FIG. 12

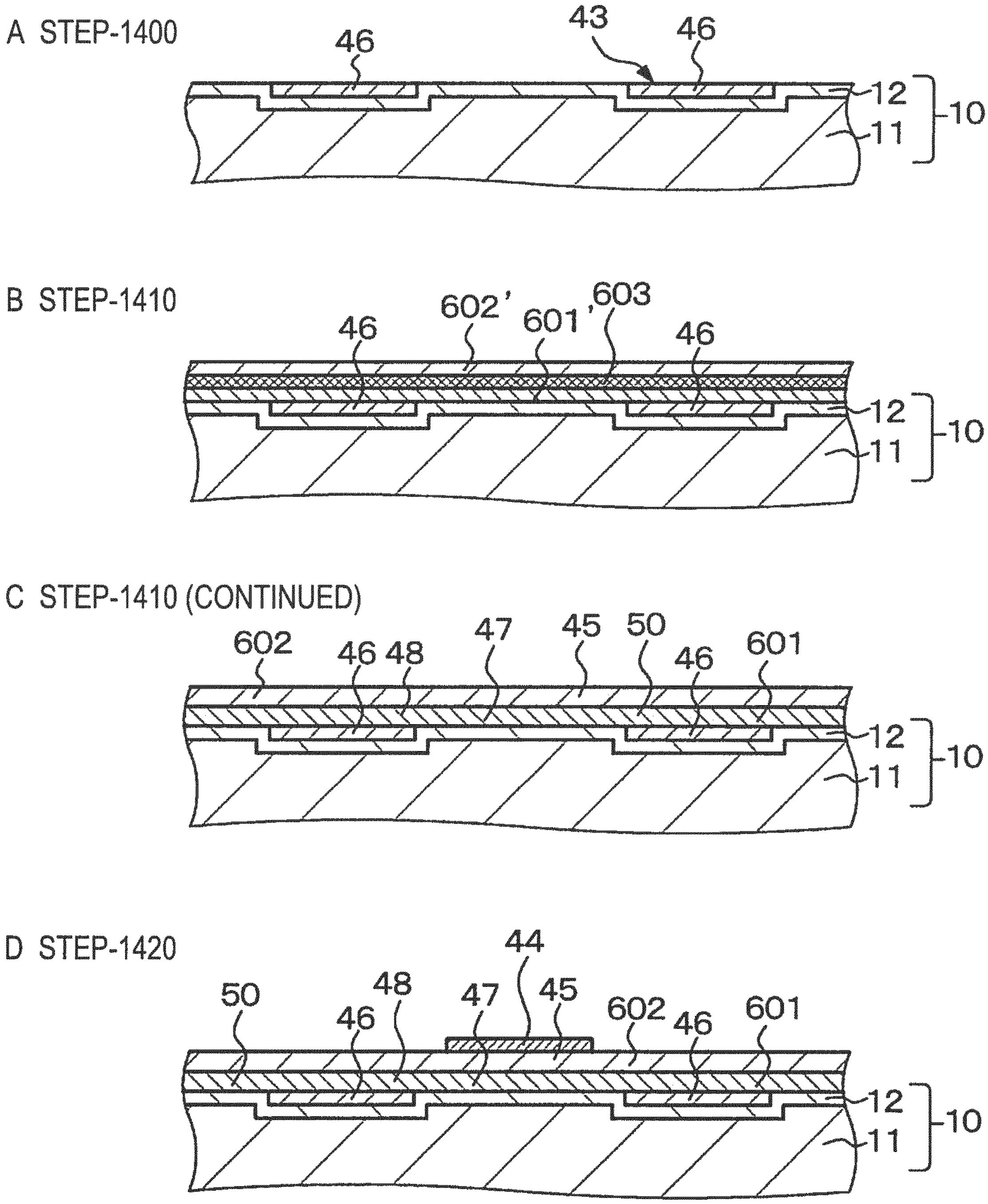
A STEP-1400
46
43
46
12
10
11
B STEP-1410
602'
601'
603
46
46
12
10
11
C STEP-1410 (CONTINUED)
602
46 48
47
45
50
46
601
12
10
11
D STEP-1420
44
50
48
47
45
46
602
46
601
12
10
11

# FIG. 13

A

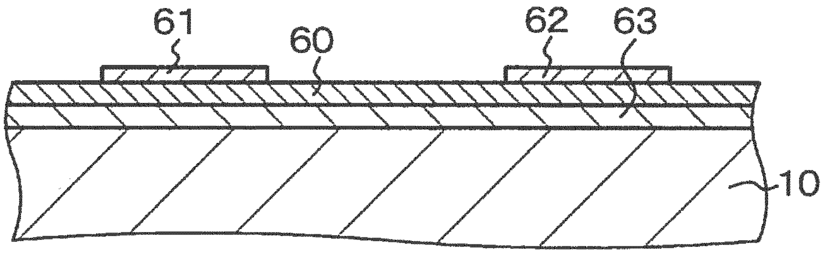


B

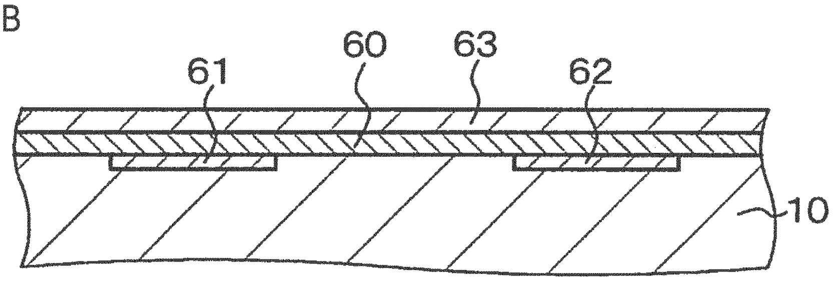

## INTERNATIONAL SEARCH REPORT

International application No. PCT/JP2012/075784

A. CLASSIFICATION OF SUBJECT MATTER

*C07D495/22*(2006.01)i, *C07D495/16*(2006.01)i, *H01L29/786*(2006.01)i, *H01L31/10*(2006.01)i, *H01L51/05*(2006.01)i, *H01L51/30*(2006.01)i, *H01L51/42*(2006.01)i

According to International Patent Classification (IPC) or to both national classification and IPC

B. FIELDS SEARCHED

Minimum documentation searched (classification system followed by classification symbols)

C07D495/22, C07D495/16, H01L29/786, H01L31/10, H01L51/05, H01L51/30, H01L51/42

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)

CA/REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | JP 2009-544743 A (MERCK PATENT GMBH), 17 December 2009 (17.12.2009), pages 35 to 38, 28 & DE 102006035035 A1 & WO 2008/011964 A1 & EP 2046915 A1 & KR 2009033493 A & CN 101490208 A & US 2010/013381 A1 & US 8114531 B2 | 1-5 |
| A | JP 2010-006794 A (SONY CORP.), 14 January 2010 (14.01.2010), compounds of the examples & US 2009/289248 A1 & CN 101591343 A | 1-5 |
| A | JP 2009-029746 A (SONY CORP.), 12 February 2009 (12.02.2009), compounds of the examples | 1-5 |

☐ Further documents are listed in the continuation of Box C. ☐ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| | |
|---|---|
| Date of the actual completion of the international search<br>05 December, 2012 (05.12.12) | Date of mailing of the international search report<br>18 December, 2012 (18.12.12) |
| Name and mailing address of the ISA/<br>Japanese Patent Office | Authorized officer |
| Facsimile No. | Telephone No. |

Form PCT/ISA/210 (second sheet) (July 2009)

## INTERNATIONAL SEARCH REPORT

International application No.
PCT/JP2012/075784

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
See extra sheet.

1. ☐ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☒ No required additional search fees were timely paid by the applicant. Consequently, this international search report is restricted to the invention first mentioned in the claims; it is covered by claims Nos.:
1-5

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

INTERNATIONAL SEARCH REPORT

International application No. PCT/JP2012/075784

Continuation of Box No.III of continuation of first sheet(2)

Invention 1: claims 1-5
Invention 2: claims 6-7
Invention 3: The part of claims 8-10 which relates to structural formula (21-1)
Invention 4: The part of claims 8 and 10 which relates to structural formula (21-2)
Invention 5: The part of claims 8 and 10 which relates to structural formula (21-3)
Invention 6: claims 11-20

Invention 1 and inventions 2-5 have a partial structure common therebetween which is a 6,12-dioxaanthanthrene skeleton. However, this skeleton is known in the light of, for example, the document (JP 2010-006794 A (SONY CORP.), 14 January 2010 (14.01.2010)) cited by the applicant. With respect to the chemical structures thereof, invention 1 and inventions 2-5 are not considered to have a novel basic skeleton common therebetween.

A partial structure common among inventions 2-5 is a 6,12-dioxaanthanthrene compound having two substituted phenyl groups. However, this partial structure is also not considered to be novel, for the same reason.

Invention 6 relates to a method for forming a layered structure, and includes no statement concerning a compound having a 6,12-dioxaanthanthrene skeleton.

Further, since there is no other common matter which is the matter common to all claims and can be considered to be a special technical feature, the number of inventions involved in the present application is six.

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2010006794 A
- JP 2009177136 A


### Non-patent literature cited in the description


- **WEI-QIAO DENG ; WILLIAM A. GODDARD III.** J. Phys. Chem. B. American Chemical Society, 2004, vol. 108, 8614-8621
- **T. OHE.** *App. Phys. Lett.,* 2008, vol. 93, 053303
- *Journal of Organic Chemistry,* 2010, vol. 75, 8241-8251
- **J. L. BARRAT ; J. P. HANSEN.** Basic Concept for Simple and Complex Liquids. Cambridge University Press, 2003
- **P.M. CHAIKIN ; T.C. LUBENSKY.** Principles of Condensed Matter Physics. Yoshioka Shoten, 2000